# FASCICULE DE BREVET EUROPEEN

(11) **EP 0 259 206 B1**
(45) Date de publication et mention de la délivrance du brevet: **09.10.1991**
(21) Numéro de dépôt: 87401808.8
(22) Date de dépôt: 04.08.1987
(51) Int. Cl.: C07D 211/90, A61K 31/44

(54) **Dérivés de la dihydro-1,4 pyridine leurs procédés de préparation et les compositions pharmaceutiques qui les contiennent**
1,4-Dihydropyridinderivate, Verfahren zu ihrer Herstellung und sie enthaltende pharmazeutische Zusammensetzungen
Derivatives of 1,4-dihydropyridine, process for their preparation and pharmaceutical compositions containing them

(30) Priorité: 04.08.1986 FR 8611260
(43) Date de publication de la demande: 09.03.1988
(73) Titulaire: ADIR ET COMPAGNIE, 92415 Courbevoie Cédex (FR)
(72) Inventeur: Péglion, Jean-Louis, F-78110 Le Vésinet (FR); Gargouil, Yves-Michel, F-75016 Paris (FR); Vilaine, Jean-Paul, F-92530 Le Plessis Robinson (FR)

(56) Documents cités:
- EP-A- 0 060 674
- EP-A- 0 089 167
- EP-A- 0 119 050
- EP-A- 0 218 068
- DE-A- 2 844 595
- J. Med. Chem. 1986, 29, p. 1696
- Progress in Pharmacology 1982, 5, p. 25

## Description

La présente invention concerne de nouveaux dérivés de la dihydro-1,4 pyridine, leurs procédés de préparation et les compositions pharmaceutiques les renfermant.

On connait certains dérivés de la dihydro-1,4 pyridine tels que la nifédipine (Brevet U.S. 3.485.847) et l'amlodipine (Publication EP 89.167) ayant des propriétés pharmacologiques intéressantes, notamment comme inhibiteurs des mouvements transmembranaires et intracellulaires du calcium. D'autres composés des dérivés d'alcoxyméthyl-2 dihydro-1,4 pyridine substitués au niveau de l'éther-oxyde par des hétérocycles aliphatiques ou aromatiques comportant un ou plusieurs atomes d'azote (Publications EP 100.189, EP 106.462, EP 107.293, EP 132.375, EP 172.029, EP 164.247, et EP 150.939) ou par des groupements aminoalkyl (Publications EP 116.769, EP 60.674, et EP 119.050) ou hydroxyalkyle (Publication EP 161.917) sont connus. La Publication EP 02.148.068 décrit des dérivés de la dihydro-1,4 pyridine substitués en position 2 par un radical hydroxyamine. Ces composés sont des inhibiteurs des mouvements transmenbra- naires et intracellulaires du calcium et sont aussi doués des propriétés beta-bloquantes.

D'autre part, des dérivés de la méthyl-2, et de l'aminométhyl-2 dihydro-1,4 pyridine sont décrits dans la publication EP 145.434. Certains composés de l'aminoalkyl-2 dihydro-1,4 pyridine sont aussi connus (Publication DE 28 44 595, Demande JP 80/47656).

Les composés de la présente invention se distinguent des autres dihydro-1,4 pyridines connues dans l'état de l'art, par leur structure et par leur activité pharmacologique. En effet, les composés de l'invention sont de puissants inhibiteurs de mouvements transmembranaires du calcium ayant une activité de très longue durée, permettant ainsi de proposer des traitements avec une seule prise quotidienne.

La présente invention a plus particulièrement pour objet les dérives de la dihydro-1,4 pyridine de formule générale 1 : 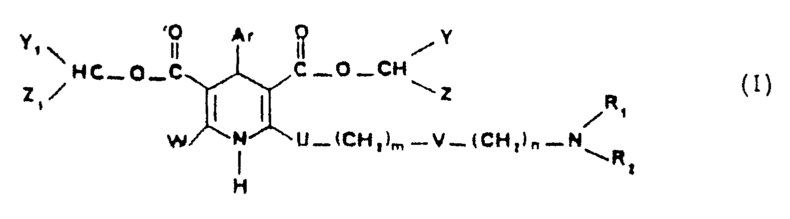 dans laquelle :
- Ar représente un radical phényle, comportant éventuellement un à cinq substituants identiques ou différents représentant chacun un atome d'halogène, un radical alkoxy renfermant de 1 à 4 atomes de carbone, un radical alkylthio renfermant de 1 à 4 atomes de carbone, un radical trihalogénométhyle, ou un radical méthylènedioxy,
- Y, Z, Y₁ et Z₁ identiques ou différents représentent chacun un atome d'hydrogène, un radical alkyle inférieur linéaire ou ramifié renfermant de 1 à 4 atomes de carbone, un radical cyclopropyle, un radical dicyclopropylméthyle, un radical dicyclopropyl-2,2 éthyle, un radical dicyclopropyl-2,2 éthylène, un radical dicyclopropyl-3,3 propyle, ou un radical dicyclopropyl-3,3 propylène-1,
- W représente un radical alkyle inférieur, linéaire ou ramifié, renfermant de 1 à 4 atomes de carbone ou un radical alcoxyméthyle inférieur renfermant de 2 à 5 atomes de carbone,
- V représente un radical méthylène ou un atome d'oxygène,
- U représente un radical méthylèneoxy ou un radical éthylèneoxy quand V représente un atome d'oxygène, ou un radical méthylène quand V représente aussi un radical méthylène,
- m et n identiques ou différents représentent un nombre entier pouvant prendre les valeurs de 1 à 4,
- Ri, et R₂ identiques ou différents représentent chacun un atome d'hydrogène, un radical alkyle inférieur linéaire ou ramifié renfermant de 1 à 4 atomes de carbone, ou un radical alkylène inférieur linéaire ou ramifié renfermant de 1 à 4 atomes de carbone, un radical trihalogénoacétyle à condition toutefois que dans ce cas V ne représente jamais un radical méthylène, un radical phénalkyle de 7 à 10 atomes de carbone éventuellement substitué sur le cycle aromatique par un ou plusieurs radicaux alkyle ou alcoxy renfermant de 1 à 4 atomes de carbone ou par un ou plusieurs radicaux hydroxy, un radical hydroxy-1 phényl-2 éthyle éventuellement substitué sur le cycle aromatique par un ou plusieurs radicaux alkyle ou alcoxy renfermant de 1 à 4 atomes de carbone ou par un ou plusieurs radicaux hydroxy, ou forment ensemble avec l'atome d'azote auquel ils sont attachés un groupement phtalimido, sous forme racémique ou d'isomères optiques, et leurs sels d'addition à un acide minéral ou organique pharmaceutiquement acceptable, ainsi que leurs sels d'ammonium quaternaire formés avec un halogénure d'alkyle ou alkylène inférieur renfermant de 1 à 4 atomes de carbone quand ils comportent une amine tertiaire.

La présente invention a également pour objet le procédé de préparation de composés de formule générale I, caractérisé en ce que :
- soit
   l'on condense un composé de formule générale II : 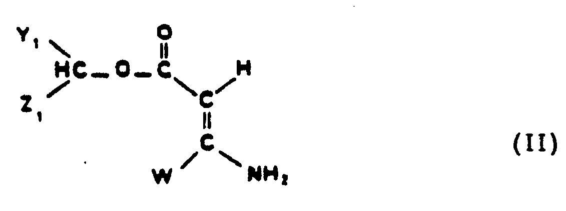 dans laquelle la définition des substituants Y₁, Z₁ et W demeure celle définie précédemment pour la formule générale I,
   avec un cetoester de formule générale III, 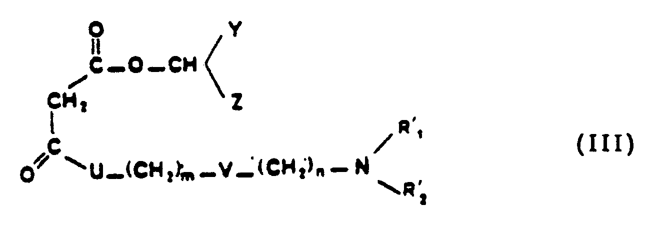 dans laquelle Y, Z, U, V, m et n ont la signification précédemment définie dans la formule I, et R'₁ et R'₂ représentent un radical méthyle et un radical benzyle, ou un atome d'hydrogène et un radical trihalogénoacétyle à condition toutefois que dans ce cas V ne représente jamais simultanément un radical méthylène, ou forment ensemble avec l'atome d'azote auquel ils sont attachés un radical phtalimido,
   et avec un aldéhyde aromatique de formule générale IV : 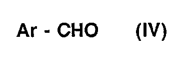 dans laquelle la définition de Ar demeure celle définie précédemment pour la formule générale I, dans un solvant organique polaire tel qu'un alcool primaire ou secondaire ou un acide organique, de petit poids moléculaire, et à une température comprise entre 40⁰ C et 100 °C, pour obtenir un composé de formule générale l', 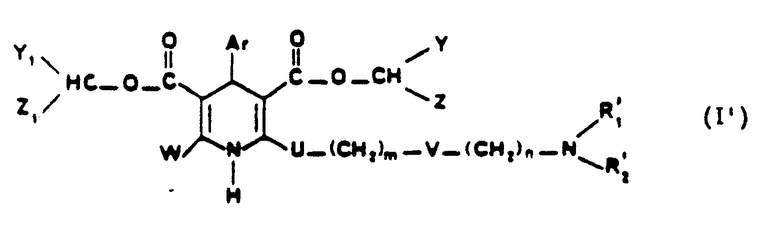 dans laquelle la définition de Ar, Y, Z, Y₁, Z₁, W, U, V, m et n demeure celle indiquée précédemment et la définition de R'₁ et R'₂ reste identique à celle donnée pour R'₁ et R'₂ de la formule générale III,
soit
   l'on condense un cetoester de formule générale V : 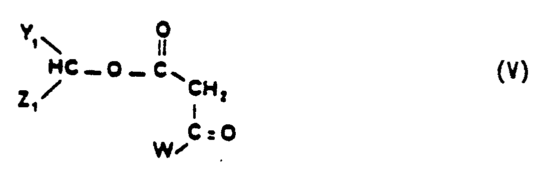 dans laquelle la définition de Y₁, Z₁ et W reste identique à celle donnée pour la formule I,
   avec un composé de formule générale VI : 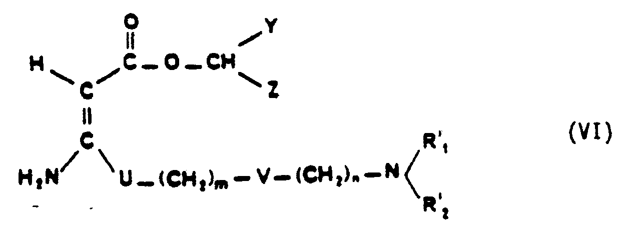 dans laquelle Y, Z, U, V, m et n ont la signification précédemment définie pour la formule I, et la définition de R'₁ et R'₂ reste identique à celle donnée pour la formule générale III,
   et avec un aldéhyde aromatique de formule générale IV,
   dans un solvant organique polaire tel qu'un alcool primaire ou secondaire ou un acide organique, de petit poids moléculaire, et à une température comprise entre 40⁰ et 100° C,
   pour obtenir un composé de formule générale l',
- soit
   l'on condense un benzylidène de formule générale VII, 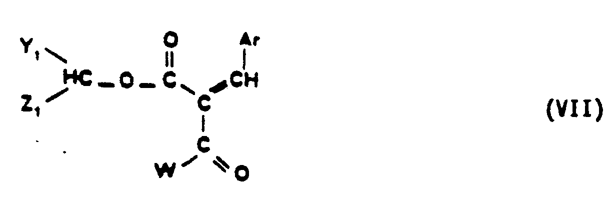 dans laquelle la définition de Y₁, Zi, W et Ar reste identique à celle donnée pour la formule I,
   avec un composé de formule générale VI, dans un solvant organique polaire tel qu'un alcool primaire ou secondaire ou un acide organique, de petit poids moléculaire et à une température comprise entre 40 ° et 100 ° C,
   pour obtenir les composés de formule générale l',
- et ensuite,
   si on le désire, on soumet les composés de formule générale l'
   dans laquelle la signification de Ar, Y, Z, Y₁, Zi, W, U, V, m et n demeure celle indiquée précédemment et R₁ et R₂ représentent un radical hydrogène et un radical trihalogénoacétyle, ou forment ensemble avec l'atome d'azote auquel ils sont attachés un radical phtalimido,
   à l'action de l'hydrazine ou d'un sel minéral basique tel que le carbonate de potassium, en présence d'eau, dans un solvant alcoolique polaire de petit poids moléculaire miscible à l'eau, et à une température comprise entre 40⁰ et 100 ° C
   pour obtenir les composés de la formule générale I,
   dans laquelle Ar, Y, Z, Y₁, Z₁ W, U, V, m et n ont la signification précédemment définie et R₁ et
   R₂ représentent un atome d'hydrogène,
- et ensuite,
   si on le désire
soit
   l'on soumet à l'action d'un aryléthylèneoxyde de formule générale VIII : 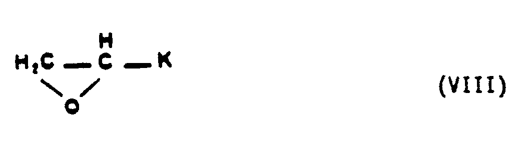 dans laquelle K représente un radical phényle, éventuellement substitué par un ou plusieurs radicaux alkyle ou alcoxy renfermant de 1 à 4 atomes de carbone ou un ou plusieurs radicaux hydroxy,
   pour obtenir un composé de formule générale I"
   dans laquelle la signification de Ar, Y, Z, Yi, Zi, W, U, V, m et n demeure identique à celle indiquée précédemment, et la signification de K reste identique à celle donnée pour la formule générale VIII, 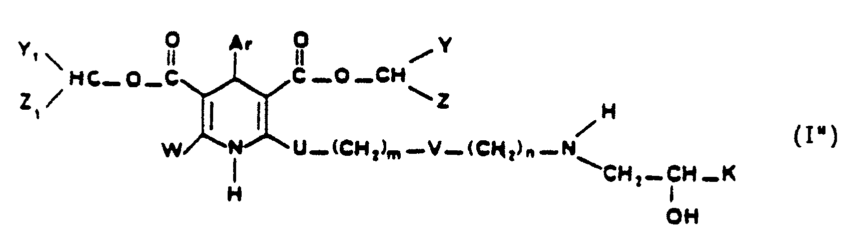 soit
   l'on soumet à l'action d'un agent alcoylant de formule générale IX : 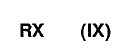 dans laquelle X représente un halogène et R représente un radical alkyle ou alkylène inférieur linéaire ou ramifié renfermant de 1 à 4 atomes de carbone,
   dans un solvant organique polaire tel que l'acétonitrile en présence d'un sel minéral basique tel que le carbonate de potassium à une temperature comprise entre 40⁰ et 100° C,
   pour obtenir les composés de la formule générale I dans laquelle Ar, Y, Z, Y₁, Zi, W, U, V, m et n ont la même signification que celle précédemment définie et R₁ et R₂ sont identiques et ont la même signification que R de la formule générale IX, ou bien
   soit d'abord à l'action du benzaldéhyde en présence d'un solvant aromatique inerte et apolaire, tel que le benzène et à une température entre 50-120 ° C, et ensuite, après élimination du solvant utilisé à l'action de borohydrure de sodium, en présence d'un alcool aliphatique polaire de petit poids moléculaire, pour obtenir un composé de formule générale 1
   dans laquelle la signification de Ar, Y, Z, Yi, Zi, W, U, V, m et n demeure identique à celle indiquée précédemment, R₁ représente un atome d'hydrogène et R₂ un radical benzyle, lequel ensuite,
   si on le désire est soumis à l'action d'un agent alcoylant de formule générale IX,
   pour obtenir un composé de la formule générale I
   dans laquelle Ar, Y, Z, Y₁, Zi, W, U, V, m et n ont la signification précédemment définie, la définition de Ri est identique à celle de R de la formule générale IX et R₂ représente un radial benzyle, sous forme de sels d'ammonium quaternaire ou d'amines tertiaires, lequel ensuite,
   si on le désire est soumis à l'action du triéthylborohydrure de lithium ou a une hydrogénation catalytique pour obtenir respectivement par les sels d'ammonium quaternaire ou les amines tertiaires, les composés de la formule générale I dans laquelle Ar, Y, Z, Y₁, Zi, W, U, V, m et n ont la même signification que celle définie précédemment, R₁ ayant la même signification que R de la formule générale IX et R₂ représente respectivement un radical benzyle ou un atome d'hydrogène,
   puis,
   si on le désire, on le soumet à l'action d'un agent alcoylant de formule générale IX,
   pour obtenir les composés de formule générale 1
   dans laquelle R₁ et R₂ identiques ou différents représentent chacun un radical alkyle inférieur, linéaire ou ramifié, renfermant de 1 à 4 atomes de carbone ou un radical alkylène linéaire ou ramifié, renfermant de 1 à 4 atomes de carbone,
   et ensuite,
   s̅i̅ o̅n̅ le désire, on transforme les composés de la formule générale I en un sel d'addition avec un acide organique ou minéral pharmaceutiquement acceptable,
   ou dans le cas où ils portent sur leur chaîne latérale en position 2 une amine tertiaire, en un sel d'ammonium quaternaire avec un alkyle ou alkylene halogéné de formule générale IX.

Les composés de formule générale Il peuvent être obtenus selon la methode décrite par CELERIER et Coll. (Synthesis,1981,p. 130-133).

Les composés de formule générale III peuvent être obtenus par traitement des composés de formule générale X : 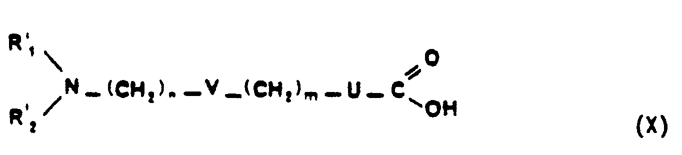 dans laquelle U, V, m et n, Ri' et R₂' ont la signification précédemment définie pour la formule III,
avec un chlorure d'acide, suivi d'une condensation avec de l'acide de Meldrum en présence de pyridine,
pour obtenir les composés de formule générale XI : 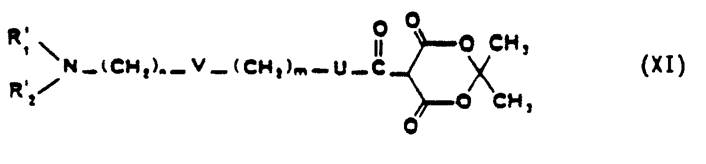 dans laquelle la définition de U, V, m, n, R'₁ et R'₂ reste identique a celle donnée précédemment. Les composés de la formule générale XI sont ensuite soumis à l'action d'un alcool de formule générale XII, 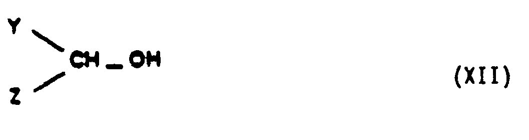 dans laquelle Y et Z ont la signification précédemment définie pour la formule générale I, à une température comprise entre 40-150 ° C, pour obtenir les composés de la formule générale III.

La préparation des composés de formule générale X est connue (Carbohydrate Research, 1981,88, p. 213-221).

Alternativement, et lorsque dans la formule générale III, U et V représentent un atome d'oxygène, on peut aussi les préparer selon la méthode décrite par TROOSTWIJK C. et KELLOGG R. (J.C.S. Chem.Comm.,1977, p.932-933).

Les composés de la formule générale V peuvent être préparés aussi par condensation d'un chlorure d'acide de formule générale XIII 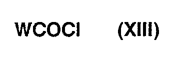 dans laquelle W a la même signification que pour la formule générale avec l'acide de Meldrum. Les produits issus de cette réaction sont ensuite soumis à l'action d'un alcool de formule générale XII pour donner les composés de formule générale V.

Les composés de formule générale VI peuvent être obtenus par l'action de l'acétate d'ammonium sur les composés de la formule générale III, dans un alcool polaire et à une température comprise entre 50 et 100° C.

Les composés de la formule générale VII peuvent être obtenus par condensation des composés de formule générale V avec un aldéhyde aromatique de formule générale IV. (Can.J.Chem.,1967,45,p. 1001).

Le procédé d'obtention des amines secondaires à partir des sels d'ammonium quaternaire après réaction avec le triéthylborohydrure de lithium est connu. (J.Org.Chem.,1975,40,n 4,p. 532). De la même manière, l'alkylation des amines primaires avec des aryléthylèneoxydes de formule générale VIII est décrite dans la littérature. (Tetra.Let.1986,27,n ° 22, p. 2451-2454).

Les isomères optiques des produits de la formule générale qui font l'objet de la présente invention peuvent être obtenus par des méthodes connues.

Parmi les acides pharmaceutiquement acceptables pour la préparation des sels d'addition aux composés de formule générale I, on peut citer les acides phosphorique, chlorhydrique, citrique, oxalique, maléïque, sulfurique, tartrique, mandélique, fumarique, méthanesulfonique etc...

Les composés selon l'invention ainsi que leurs sels d'addition ou d'ammonium quaternaire sont doués de propriétés pharmacologiques fort intéressantes et se distinguent des autres dérivés de la dihydro-1,4 pyridine déjà connus.

En effet, les essais pharmacologiques in vitro ont montré que ces composés sont de puissants inhibiteurs de la pénétration intracellulaire du calcium.

La concentration en calcium intracellulaire joue un rôle de messager pour de nombreuses fonctions biologiques : contractions, sécrétions; cette concentration dépend largement de la pénétration transmembra- naire en calcium, qui, très concentré dans les milieux extracellulaires, transite à travers les canaux sélectifs pour le calcium situés dans la membrane.

C'est ainsi que des inhibiteurs de ces canaux limitant ou supprimant la pénétration du calcium peuvent avoir des effets thérapeutiques intéressants dans nombre de pathologies comme par vasorelaxation, pour le traitement de l'hypertension artérielle et l'hypertension pulmonaire, des maladies vasculaires périphériques et coronaires. (Am.J.Card.1980,46,p.1047-1058; Burger's Medicinal Chemistry 4ème Edition, Part III,p.5456 - John Wiley and Sons inc. USA,1̅9̅81; Life Science, 1983,33,p.2571-2581). Des effets bénéfiques induits sont également constatés pour le traitement des insuffisances cardiaques.

La modération de la contraction myocardique est aussi utile dans les situations ischémiques cardiaques. (Medicine,1985,64,p.61-73). La limitation de la pénétration du calcium dans les cellules peut également jouer un rôle important pour prévenir l'accumulation calcique caractéristique du vieillissement cellulaire et liée à certaines maladies dégénératives vasculaires -athéromatoses en particulier- (Medicinal research review,1985,5,p.394-425).

La modulation calcique présente également un intérêt pour le traitement de l'épilepsie et des vertiges d'origine centrale. La limitation du calcium ionisé dans les fibres lisses des parois du tube digestif permet aussi la levée des spasmes oesophagiens et au niveau pulmonaire celui du spasme bronchique (traitement de l'asthme). Cette modération du calcium ionisé peut aussi être utile comme adjuvant dans les traitements du cancer et celui de l'hypercoagulation.

La présente description n'est d'ailleurs pas limitative, les travaux fondamentaux mettent en effet l'accent sur le rôle de premier plan joué par le calcium dans nombre de phénomènes physiologiques et physiopathologiques.

Les essais pharmacologiques chez le chien et le rat, ont prouvé in vivo, que l'activité des composés de l'invention est au moins égale à celle des autres dérivés de la dihydro-1,4 pyridine connus, mais qu'ils sont doués d'une durée d'action plus longue et confirment l'intérêt de leur emploi en thérapeutique. En effet, les différents dérivés de la dihydro-1,4 pyridine connus jusqu'à présent ont une activité de courte durée ce qui constitue un désavantage considérable en thérapeutique humaine et animale.

L'invention s'étend aussi aux compositions pharmaceutiques renfermant comme principe actif au moins un composé de formule générale I, l'un de ses isomères optiques ou l'un des sels d'addition avec un acide minéral ou organique ou d'ammonium quaternaire avec un ou plusieurs excipients, inertes, non toxiques et appropriés.

Les compositions pharmaceutiques ainsi obtenues sont présentées avantageusement sous des formes diverses telles que par exemple comprimés, dragées, gélules, glossettes ou autres préparations galéniques appropriées pour une administration sublinguale, suppositoires, solutions injectables ou buvables.

La posologie peut varier largement en fonction de l'âge, du poids du patient, de la nature et de la sévérité de l'affection ainsi que de la voie buccale ou parentérale. D'une manière générale, la posologie unitaire s'échelonnera entre 0,05 et 50 mg et la posologie journalière administrée par voie orale, utilisable en thérapeutique humaine ou animale entre 0,05 et 100 mg.

Les points de fusion indiqués sont mesurés selon la technique micro-Kôfler.

Les spectres de résonance magnétique nuclaire du proton (R.M.N.) ont été enregistrés à 60 MHz.

### EXEMPLE 1 :

### Hémifumarate de (4R,S) {[(amino-2 éthoxy)-2 éthoxy] méthyl}-2 éthoxycarbonyl-3 méthoxycarbonyl-5 méthyl-6 pentafluorophényl-4 dihydro-1,4 pyridine.

### STADE A :

### E̅s̅t̅e̅r̅ é̅th̅yl̅ique de l'acide phtalimido-10 oxo-3 dioxa-5,8 décanoïque

A une suspension de 31 g d'hydrure de sodium dans 400 ml de tétrahydrofuranne rajouter par portions 77 g de [(phtalimido)-2 éthoxyl]-2 éthanol, en maintenant la température aux environs de 25⁰ C. Agiter pendant une heure et ensuite ajouter 53,6 g de chloroacétoacétate d'éthyle en maintenant la température du mélange à -20 C. Laisser reposer une nuit à la température ambiante et ensuite hydrolyser avec 1 1 d'acide chlor₋hydrique 1N. Décanter, extraire à l'éther, réunir les phases organiques. Laver à l'eau et ensuite sécher sur sulfate de magnésium. Concentrer et purifier le résidu ainsi obtenu (110 g) par chromatographie sur colonne de silice en utilisant comme solvant d'élution un mélange de dichlorométhane et d'acétone (95:5). Après élimination du solvant d'élution, on obtient 35,7 g de l'ester éthylique de l'acide phtalimido-10 oxo-3 dioxa-5,8 décanoïque.
Rendement : 30 %
Analyse élémentaire :

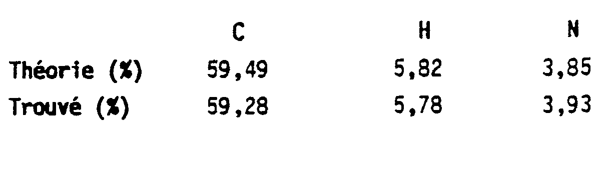

### STADE B :

### (4R,S) éthoxycarbonyl-3 méthoxycarbonyl-5 méthyl-6 pentafluorophényl-4 {[(phtalimido-2 éthoxy)-2 éthoxy] méthyl}-2 dihydro-1,4 pyridine.

Dans une solution de 105 ml d'isopropanol contentant 20 g de l'ester éthylique de l'acide phtalimido-10 oxo-3 dioxa-5,8 décanoïque précédemment obtenu et 6,30g d'amino-2 crotonate de méthyle ajouter 10,8 g de pentafluorobenzaldéhyde.

Porter à reflux pendant une nuit et ensuite évaporer sous pression réduite pour obtenir 39 g d'une huile épaisse. Purifier par chromatographie sur colonne de silice en utilisant comme solvant d'élution un mélange de cyclohexane et d'acétate d'éthyle (80:20). Après évaporation du solvant d'élution, recristalliser deux fois dans le méthanol pour obtenir 2,3 g de (4R,S) éthoxycarbonyl-3 méthoxycarbonyl-5 méthyl-6 pentafluorophényl-4 {[(phtalimido-2 éthoxy)-2 éthoxy] méthyl}-2 dihydro-1,4 pyridine.
Rendement : 6 %
Point de fusion : 143-144° C
Analyse élémentaire :

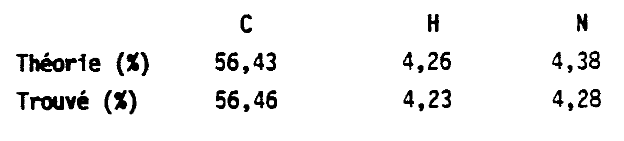

### STADE C:

Dissoudre 6,8 g du composé obtenu au stade précédent dans 68 ml d'éthanol contenant 2,3 ml d'hydrate d'hydrazine. Porter le mélange à reflux sous agitation pendant trois heures et ensuite filtrer. Recueillir le filtrat et ensuite évaporer. Dissoudre le résidu obtenu dans l'éther éthylique, filtrer et extraire par une solution d'acide sulfurique 1 N. Alcaliniser la phase aqueuse à l'aide de soude concentrée et ensuite extraire par l'éther éthylique. Evaporer à sec. Après recristallisation du résidu dans l'éther isopropylique, on obtient 2,3 g de (4R,S) {[(amino-2 éthoxy)-2 éthoxy] méthyl}-2 éthoxycarbonyl-3 méthoxycarbonyl-5 méthyl-6 pentafluorophényl-4 dihydro-1,4 pyridine.
Rendement : 35 %
Point de fusion : 72-73° C

Pour former l'hémifumarate de la (4R,S) {[(amino-2 éthoxy)-2 éthoxy] méthyl}-2 éthoxycarbonyl-3 méthoxycarbonyl-5 méthyl-6 pentafluorophényl-4 dihydro-1,4 pyridine dissoudre la quantité de la base obtenue précédemment dans une solution de 0,00226 M d'acide fumarique à 2%. Porter le mélange à reflux dans l'éthanol puis évaporer à sec et recristalliser dans l'éthanol.
Point de fusion : 146-148° C
Analyse élémentaire :

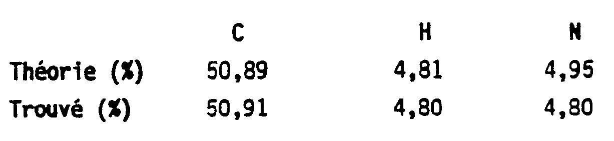

Les constantes physiques spectrales de l'hémifumarate de la (4R,S) {[(amino-2 éthoxy)-2 éthoxy] méthyl}-2 éthoxycarbonyl-3 méthoxycarbonyl-5 méthyl-6 pentafluorophényl-4 dihydro-1,4 pyridine sont indiquées dans le Tableau 1.

### EXEMPLE 2 :

(4R,S) {[(amino-2 éthoxy)-2 éthoxy] méthyl}-2 éthoxycarbonyl-3 méthoxycarbonyl-5 méthyl-6 (méthylènedioxy-2,3 phényl)-4 dihydro-1,4 pyridine.

Ce composé a été préparé selon le procédé décrit dans l'exemple 1, mais en remplaçant au stade B le pentafluorobenzaldéhyde par le méthylènedioxy-2,3 benzaldéhyde.
Rendement global : 15,5 %
Point de fusion : 98-100 C
Analyse élémentaire :

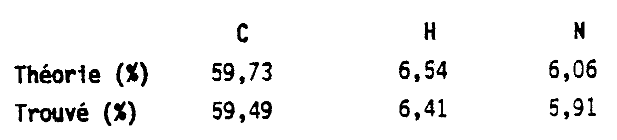

Les constantes physiques spectrales sont données dans le Tableau I.

### EXEMPLE 3 :

### Fumarate de (4R,S) {[(amino-2 éthoxy)-2 éthoxy] méthyl}-2 éthoxycarbonyl-3 méthoxycarbonyl-5 méthyl-6 (triméthoxy-2,3,4 phényl)-4 dihydro-1,4 pyridine.

Ce composé a été aussi préparé selon le procédé décrit dans l'exemple 1 mais en remplaçant au stade B le pentafluorobenzaldéhyde par le triméthoxy-2,3,4 benzaldéhyde.
Rendement global : 8,4 %
Point de fusion : 130 ° C
Analyse élémentaire :

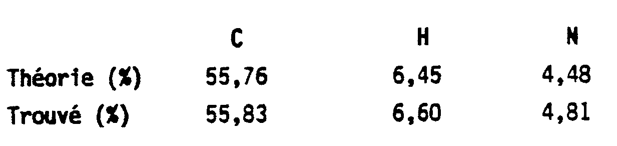

Les constantes physiques spectrales de la base correspondante sont données dans le Tableau I.

### EXEMPLE 4 :

Fumarate de (4R,S) {[(amino-2 éthoxy)-2 éthoxy] méthyl}-2 (chloro-2 phényl)-4 (dicyclopropyl-2,2 éthoxycarbonyl)-3 méthoxycarbonyl-5 méthyl-6 dihydro-1,4 pyridine.

### STADE A

Ester dicyclopropyl-2,2 éthylique de l'acide phtalimido-10 oxo-3 dioxa-5,8 décanoïque

Porter à reflux jusqu'à fin de dégagement gazeux environ 29,5 g de l'acide phtalimido-8 dioxa-3,6 octanoïque et 155 ml de chlorure de thionyle. Après refroidissement, éliminer par distillation l'excès de chlorure de thionyle et reprendre par le benzène les 31,1 g du chlorure d'acide ainsi obtenu. Evaporer le solvant et dissoudre le résidu dans 10 ml de chlorure de méthylène. Introduire cette solution goutte à goutte dans un mélange composé de 13,6 g d'acide de Meldrum, 15,3 ml de Pyridine et 75 ml de chlorure de méthylène, en maintenant la température entre 0 ° et 5 ° C. Abandonner 3 heures à la température ambiante et ensuite diluer avec 300 ml de chlorure de méthylène, laver avec 100 ml d'acide chlorhydrique 1 N puis avec une solution saturée de bicarbonate de sodium, rincer à l'eau et sécher sur sulfate de magnésium anhydre. Evaporer le solvant organique et dissoudre le résidu de l'évaporation dans 48 g de cyclopropyl-2,2 éthanol. Ajouter quelques mg d'acide malonique et porter à 145 ° C pendant environ 5 heures. Eliminer ensuite l'excès du dicyclopropyl-2,2 éthanol par distillation, reprendre le résidu par du dichlorométhane et laver avec une solution saturée de bicarbonate de sodium puis à l'eau, sécher sur sulfate de magnésium anhydre et évaporer le solvant organique.

Purifier l'huile obtenue après évaporation par chromatographie sur colonne de silice en utilisant comme solvant d'élution un mélange de dichlorométhane et d'acétate d'éthyle (95:5).

On obtient 5,7 g de l'ester dicyclopropyl-2,2 éthylique de l'acide phtalimido-10 oxo-3 dioxa-5,8 décanoïque.
Rendement : 12,5 %
Analyse élémentaire :

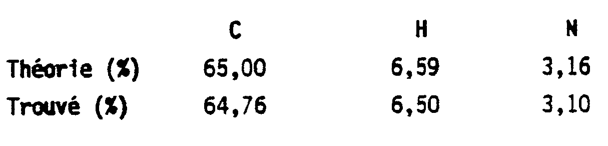

Spectre de résonance magnétique nucléaire du proton (CDCl₃) : 0,0-0,9ppm,m,11H; 3,5ppm,s,2H; 3,7ppm,s,4H; 3,7-4,Oppm,m,4H; 4,0-4,4ppm,m, 2H; 4,2ppm,s,2H; 7,6-8,2ppm,m,4H.

### STADE B :

Le fumarate de (4R,S) {[(amino-2 éthoxy)-2 éthoxy] méthyl}-2 (chloro-2 phényl)-4 (dicyclopropyl-2,2 éthoxycarbonyl)-3 méthoxycarbonyl-5 méthyl-6 dihydro-1,4 pyridine est obtenu en faisant réagir l'ester préparé au stade A avec le chloro-2 benzaldéhyde et l'amino-2 crotonate de méthyle selon le procédé décrit dans les stades B et C de l'exemple 1.
Rendement : 10 %
Point de fusion : 198-199 ° C
Spectre de masse de la base (ionisation chimique NH₃) : 533 m/_{z} (M⁺ + 1), 430 m/_{z}, 428m/_{z}, 106m/_{z}.

### EXEMPLE 5 :

Fumarate de (4R,S) {[(amino-2 éthoxy)-2 éthoxy] méthyl}-2 (chloro-2 phényl)-4 éthoxycarbonyl-3 méthoxycarbonyl-5 méthyl-6 dihydro-1,4 pyridine.

Ce composé a été préparé selon le procédé décrit dans l'exemple 1 mais en remplaçant la pentafluorobenzaldéhyde par la chloro-2 benzaldéhyde.
Rendement : 10 %
Point de fusion : 188-190° C
Analyse élémentaire :

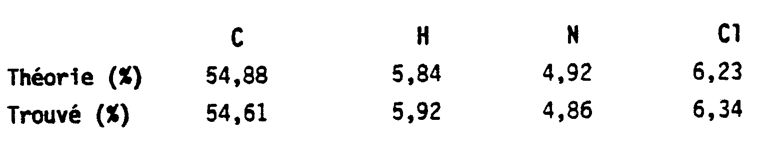

Les constantes physiques spectrales de la base correspondante sont données dans le Tableau I.

### EXEMPLE 6 :

(4R,S) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine.

Ce composé a été préparé selon le procédé décrit dans l'exemple 1 mais en remplaçant la pentafluorobenzaldéhyde par du dichloro-2,3 benzaldéhyde.
Rendement : 12 %
Point de fusion : 77-79° C
Analyse élémentaire :

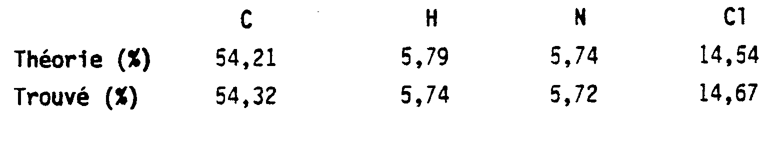

Ses constantes physiques spectrales sont données dans le Tableau I.

### EXEMPLE 7 :

### Tartrate de (4R,S) {[((N-méthyl N-benzyl amino)-2 éthoxy)-2 éthoxy] méthyl}-2 (chloro-2 phényl)-4 éthoxycarbonyl-3 méthoxycarbonyl-5 méthyl-6 dihydro-1,4 pyridine.

### STADE A

Ester éthylique de l'acide (N-méthyl N-benzyl amino)-10 oxo-3 dioxa-5,8 décanoïque.

Pour préparer ce composé, on utilise le mode opératoire décrit pour l'exemple 1 (stade A) mais on remplace le (phtalimido-2 éthoxy)-2 éthanol par le (N-méthyl N-benzyl amino-2 éthoxy) -2 éthanol. Rendement : 6,8 %
Spectre de résonance magnétique nucléaire du proton (CDCl₃):
1,3ppm,t,3H; 2,3ppm,s,3H; 2,6ppm,t2H; 3,4-3,7ppm,m,10H; 4,2ppm,s,2H; 4,2pmm,m,2H; 7,4ppm,s,5H.

### STADE B

(4R,S) {[(N-méthyl N-benzyl amino-2 éthoxy)-2 éthoxy] méthyl}-2 éthoxycarbonyl-3 (chloro-2 phényl)-4 méthoxycarbonyl-5 méthyl-6 dihydro-1,4 pyridine.

Cette base est obtenue selon la méthode décrite pour l'exemple 1 (stade B) mais en remplaçant le pentafluorobenzaldéhyde par le chloro-2 benzaldéhyde, et aussi en remplaçant l'ester éthylique de l'acide phtalimido-10 oxo-3 dioxa-5,8 décanoïque par celui obtenu au stade A ci-dessus.
Rendement : 40 %

Les constantes physiques spectrales de cette base sont indiquées dans le tableau I.

Soumise à l'action de l'acide tartrique, la base ci-dessus décrite est transformée en tartrate. Analyse élémentaire :

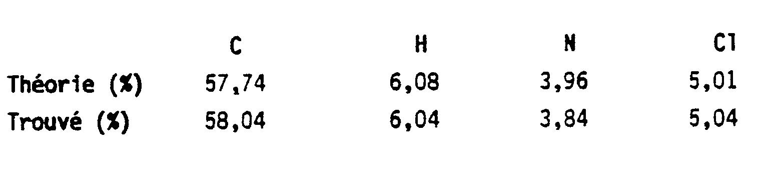

### EXEMPLE 8 :

Hémifumarate de (4R,S) (chloro-2 phényl)-4 {[((N,N-diallylamino)-2 éthoxy)-2 éthoxy] méthyl}-2 éthoxycarbonyl-3 méthoxycarbonyl-5 méthyl-6 dihydro-1,4 pyridine.

Dissoudre dans 40 ml d'acétonitrile 4,5 g de composé de l'exemple 5, et 1,92 g de bromure d'allyle en présence de 0,0086 M de carbonate de potassium sec. Chauffer à reflux pendant 16 heures et ensuite filtrer le précipité, évaporer le solvant et reprendre par du dichlorométhane ; laver à l'eau et évaporer. Chromatographier le résidu sur colonne de silice en utilisant comme solvant un mélange du dichlorométhane et d'acétate d'éthyle (50:50) pour obtenir la (4R,S) (chloro-2 phényl)-4 {[((N,N-diallylamino)-2 éthoxy)-2 éthoxy] méthyl}-2 éthoxycarbonyl-3 méthoxycarbonyl-5 méthyl-6 dihydro-1,4 pyridine pure (0,7 g). Rendement : 13 %

Les constantes physiques spectrales de ce composé sont indiquées dans le tableau I.

Les 0,7 g du composé obtenu ci-dessus sont soumis à l'action de l'acide fumarique pour obtenir après recristallisation dans l'éthanol 0,4 g d'hémifumarate correspondant.
Point de fusion : 126-128° C
Analyse élémentaire :

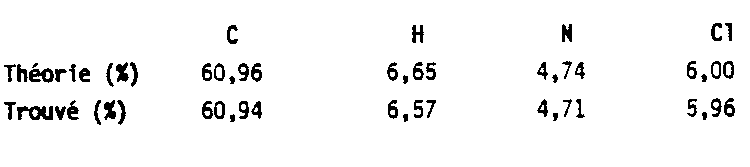

### EXEMPLE 9 :

Fumarate de (4R,S) {[(amino-2 éthoxy)-2 éthoxy] méthyl}-2 (chloro-2 phényl)-4 (dicyclopropyl-2,2 éthoxycarbonyl)-5 éthoxycarbonyl-3 méthyl-6 dihydro-1,4 pyridine.

La base correspondante est obtenue selon le procédé décrit dans l'exemple 1, mais en remplaçant la pentafluorobenzaldéhyde par le chloro-2 benzaldéhyde et l'amino-2 crotonate de méthyle par l'amino-2 crotonate de dicyclopropyl-2,2 éthyle. Rendement global : 19 %

Les constantes physiques spectrales de la (4R,S) {[(amino-2 éthoxy)-2 éthoxy] méthyl}-2 (chloro-2 phényl)-4 (dicyclopropyl-2,2 éthoxycarbonyl)-5 éthoxycarbonyl-3 méthyl-6 dihydro-1,4 pyridine sont indiquées au tableau 1.

Dissoudre 3,9 g de cette base dans une solution d'acide fumarique dans l'éthanol à 2 %. Recristalliser le précipité obtenu dans le méthanol pour obtenir 2,7 g du sel attendu.
Point de fusion : 166-168° C.
Analyse élémentaire :

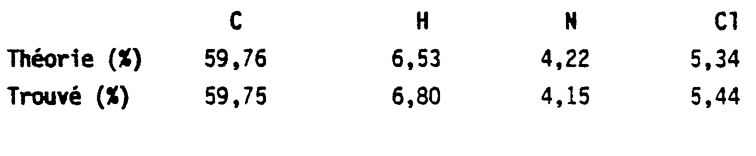

### EXEMPLE 10 :

(4R,S) {((amino-2 éthoxy)-2 éthoxy] méthyl}-2 (dichloro-2,3 phényl)-4 éthoxycarbonyl-3 méthoxycarbonyl-5 méthoxyméthyl-6 dihydro-1,4 pyridine.

### STADE A

Ester méthylique de l'acide (dichloro-2,3 benzylidène)-2 oxo-3 méthoxy-4 butanoïque.

Porter à reflux pendant une heure en éliminant l'eau formée, une solution contenant 160 ml de benzène anhydre, 5 g de dichloro-2,3 benzaldéhyde, 4,2 g d'ester méthylique de l'acide méthoxy-4 oxo-3 butanoïque 16 gouttes de pyridine et 22 gouttes d'acide hexanoïque. Laver ensuite par une solution saturée de bicarbonate de sodium puis par une solution d'acide chlorhydrique 0,1 N. Reprendre la phase organique, évaporer à sec pour obtenir 8,3 g d'une huile.
Rendement : 96,5 %
Spectre de résonance magnétique nucléaire du proton (CDCl₃)
3,3-3,4ppm,d,3H; 3,7-3,9ppm,d,3H; 4,0-4,3ppm,d,2H; 7,0-7,7ppm,m,3H; 8ppm, d,1 H.

### STADE B

### (̅4̅R̅,̅S̅)̅ {[phtalimido-2 éthoxy)-2 éthoxy] méthyl}-2 (dichloro-2,3 phényl)-4 éthoxycarbonyl-3 méthoxycarbonyl-5 méthoxyméthyl-6 dihydro-1,4 pyridine.

Porter à reflux pendant 20 minutes 8,2 g de l'ester du stade A de l'exemple 1, 1,7 g d'acétate d'ammonium et 10 ml d'éthanol. Ajouter ensuite dans le milieu réactionnel une solution composée de 8,2 g d'ester méthylique obtenu au stade A précédent et 5 ml d'éthanol. Porter le milieu réactionnel à ébullition pendant deux heures et demie. Après évaporation reprendre par le chlorure de méthylène, laver au bicarbonate puis à l'eau. Evaporer à sec et purifier l'huile ainsi obtenue sur colonne de silice en utilisant comme éluant un mélange de cyclohexane et d'acétate d'éthyle (70 : 30). Après évaporation du solvant d'élution, on obtient 2,7 g du produit attendu.
Rendement : 19 %
Point de fusion : 138-140 C.

### STADE C

Appliquer le procédé décrit au stade C de l'exemple 1 au composé obtenu ci-dessus pour obtenir après deux recristallisations dans l'éther isopropylique la (4R,S) {[(amino-2 éthoxy)-2 éthoxy] méthyl}-2 (dichloro-2,3 phényl)-4 éthoxycarbonyl-3 méthoxycarbonyl-5 méthoxyméthyl-6 dihydro-1,4 pyridine.
Rendement : 35 %
Point de fusion : 78-83 C

Les constantes physiques spectrales sont données dans le Tableau I. Analyse élémentaire :

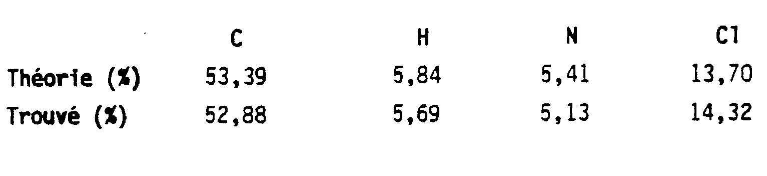

### EXEMPLE 11 :

Iodure de N-benzyl [(4R,S) éthoxycarbonyl-3 (dichloro-2,3 phényl)-4 méthoxycarbonyl-5 méthyl-6 dihydro-1,4 pyridyle-2]-7 N,N'-diméthyl, dioxa-3,6 heptanammonium.

### STADE A

(4R,S) {[((N-benzyl amino)-2 éthoxy)-2 éthoxy] méthyl}-2 (dichloro-2,3 phényl)-4 éthoxycarbonyl-3 méthoxycarbonyl-5 méthyl-6 dihydro-1,4 pyridine.

Porter à reflux un mélange composé de 3,7 g du composé de l'exemple 6, 0,8 g de benzaldéhyde et 10 ml de benzène anhydre, en éliminant l'eau avec un appareil approprié (extracteur d'eau par élimination azéotropique). Evaporer le mélange organique à sec; reprendre par l'éthanol anhydre et ajouter par portions 0,3 g de borohydrure de sodium en refroidissant de façon intermittente par l'intermédiaire d'un bain d'eau froide. Abandonner 10 minutes sous agitation, hydrolyser sur de la glace, extraire à l'éther éthylique, puis à l'acétate d'éthyle pour obtenir 3,2 g de (4R,S) {[((N-benzyl amino)-2 éthoxy)-2 éthoxy] méthyl}-2 (dichloro-2,3 phényl)-4 éthoxycarbonyl-3 méthoxycarbonyl-5 méthyl-6 dihydro-1,4 pyridine.

### STADE B

Reprendre 3,9 g du produit précédent par 9 ml de dichlorométhane en présence de 1,1 ml d'hydroxyde de sodium à 40 % et 1,3 ml d'iodure de méthyle. Abandonner une nuit sous agitation. Filtrer les cristaux obtenus et ensuite les laver à l'eau glacée pour obtenir l'iodure de N-benzyl [(4R,S) éthoxycarbonyl-3 (dichloro-2,3 phényl)-4 méthoxycarbonyl-5 méthyl-6 dihydro-1,4 pyridyle-2]-7 N,N'-diméthyl dioxa-3,6 heptanammonium.
Rendement : 56,8 %
Analyse élémentaire :

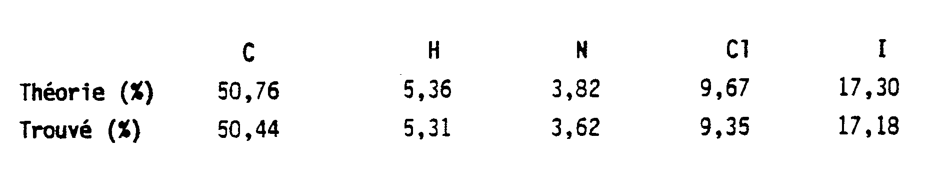

Les constantes physiques spectrales de ce composé sont indiquées dans le tableau I.

### EXEMPLE 12 :

Fumarate de (4R,S) (amino-7 heptyl)-2 (chloro-2 phényl)-4 éthoxycarbonyl-3 méthoxycarbonyl-5 méthyl-6 dihydro-1,4 pyridine.

### STADE A

Ester éthylique de l'acide phtalimido-10 oxo-3 décanoïque

Ajouter 10 g de chlorure d'oxalyle dans une solution formée de 10,5 g de l'acide phtalimido-8 octanoïque, de 40 ml de benzène anhydre et de 0,2 ml de pyridine. Porter ensuite 15 minutes à reflux. Evaporer le solvant, reprendre 2 fois par le benzène et séparer après filtration un composé huileux. Ajouter 3,0 g de chlorure d'acide ainsi obtenu dans un mélange préalablement refroidi à 0° C, contenant 1,3 g d'acide de Meldrum, 1,45 g de pyridine et 8 ml de dichlorométhane en maintenant la température à 0°C. Abandonner ensuite une nuit à température ambiante, reprendre par le dichlorométhane, laver par 10 ml d'acide chlorhydrique 1 N, sécher sur sulfate de magnésium anhydre et ensuite évaporer à sec. Reprendre le résidu pr 2,3 ml d'éthanol et porter à reflux jusqu'à fin de dégagement gazeux. Après évaporation du solvant, on obtient 2,5 g de cristaux de l'ester éthylique de l'acide phtalimido-10 oxo-3 décanoïque. Rendement : 77 %

Analyse élémentaire :

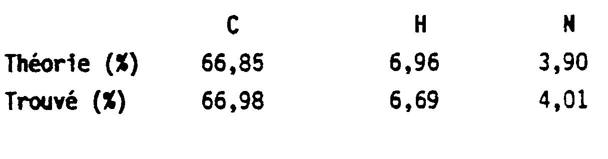

### STADE B

(4R,S) (chloro-2 phényl)-4 éthoxycarbonyl-3 méthoxycarbonyl-5 méthyl-6 (phtalimido-7 heptyl)-2 dihydro-1,4 pyridine.

Ce composé a été préparé à partir de l'ester obtenu au stade précédent en utilisant le procédé de préparation décrit dans l'exemple 1 stade B mais en remplaçant le pentafluorobenzaldéhyde par le chloro-2 benzaldéhyde.

### STADE C

La (4R,S) (amino-7 heptyl)-2 (chloro-2 phényl)-4 éthoxycarbonyl-3 méthoxycarbonyl-5 méthyl-6 dihydro-1,4 pyridine a été obtenue selon le procédé décrit dans l'exemple 1 stade C.

Les constantes physiques spectrales sont indiquées dans le tableau I.

Dissoudre ensuite ce composé dans une solution à 2 % d'acide fumarique. Evaporer à sec, dissoudre le résidu dans l'acétonitrile et évaporer à nouveau. Recristalliser le fumarate obtenu dans l'isopropanol. Rendement : 28 %
Point de fusion : 135° C
Analyse élémentaire :

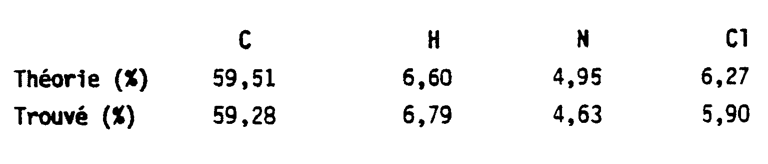

### EXEMPLE 13 :

Fumarate de (4R,S) (amino-7 heptyl)-2 (chloro-2 phényl)-4 (dicyclopropyl-2,2 éthoxycarbonyl)-3 méthoxycarbonyl-5 méthyl-6 dihydro-1,4 pyridine.

Ce composé est obtenu selon la méthode décrite pour l'exemple 12 mais en remplaçant l'ester éthylique de l'acide phtalimido-10 oxo-3 décanoïque par l'ester dicyclopropyl-2,2 éthylique de l'acide phtalimido-10 oxo-3 décanoïque.
Rendement : 12 %
Point de fusion : 105-108 C

Les constantes physiques spectrales de la (4R,S) (amino-7 heptyl)-2 (dicyclopropyl-2,2 éthoxycarbonyl)-3 (chloro-2 phényl)-4 méthoxycarbonyl-5 méthyl-6 dihydro-1,4 pyridine sont indiquées au tablaeu I.

Pour obtenir le fumarate correspondant salifier par l'acide fumarique en solution à 2 % dans l'éthanol et ensuite cristalliser dans l'acétonitrile.
Point de fusion : 137-140° C
Analyse élémentaire :

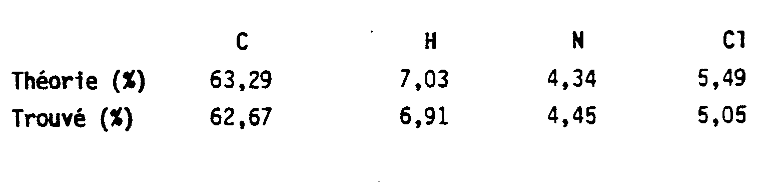

### EXEMPLE 14 :

Citrate de (4R,S) (amino-5 pentyl)-2 (chloro-2 phényl)-4 éthoxycarbonyl-3 méthoxycarbonyl-5 méthyl-6 dihydro-1,4 pyridine.

### STADE A

Ester éthylique de l'acide phtalimido-8 oxo-3 octanoïque

Cet ester est synthétisé selon le procédé décrit dans le stade A de l'exemple 4, mais en remplaçant l'acide phtalimido-8 dioxy-3,6 octanoïque par l'acide phtalimido-6 hexanoïque, et le dicyclopropyl-2,2 éthanol par l'éthanol.

### STADE B et C

La (4R,S) (amino-5 pentyl)-2 (chloro-2 phényl)-4 éthoxycarbonyl-3 méthoxycarbonyl-5 méthyl-6 dihydro-1,4 pyridine est obtenue selon les procédés décrits dans les stades B et C de l'exemple 1 mais en remplaçant au stade B le pentafluorobenzaldéhyde par le chloro-2 benzaldéhyde.

Rendement : 65 %

Ses constantes physiques spectrales sont indiquées dans le Tableau I.

Pour obtenir le citrate, la base obtenue est lyophilisée en présence d'une solution aqueuse d'acide citrique 0,1 N.

Analyse élémentaire :

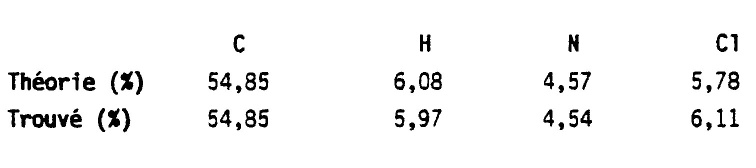

### EXEMPLE 15 :

### (4R,S) {[(amino-2 éthoxy)-2 éthoxy] méthyl}-2 diéthoxycarbonyl-3,5 méthyl-6 pentafluorophényl-4 dihydro-1,4 pyridine

Ce composé a été préparé selon le procédé décrit dans l'exemple 1 mais en utilisant au Stade B l'amino-2 crotonate d'éthyle au lieu de son homologue méthylé.
Rendement global : 9%
Point de fusion : 69-70 C
Analyse élémentaire :

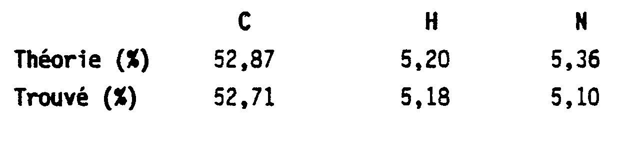

Les constantes physiques spectrales de ce composé sont indiquées dans le Tableau I.

### EXEMPLE 16 :

Oxalate de (4R,S) diéthoxycarbonyl-3,5 méthyl-6 pentafluorophényl-4 {[((N,propylamino)-2 éthoxy)-2 éthoxy] méthyl}-2 dihydro-1,4 pyridine

### STADE A

(4R,S) {[((N-benzyl amino)-2 éthoxy)-2 éthoxy] méthyl}-2 diéthoxycarbonyl-3,5 méthyl-6 pentafluorophényl-4 dihydro-1,4 pyridine.

Ce composé a été préparé selon le procédé décrit dans l'exemple 11, Stade A, mais en utilisant au lieu de la (4R,S) {[amino-2 éthoxy)-2 éthoxy] méthyl}-2 (dichloro-2,3 phényl)-4 éthoxycarbonyl-3 méthoxy carbonyl-5 méthyl-6 dihydro-1,4 pyridine le composé de l'exemple 15.

### STADE B

(4R,S) {[((N-benzyl N-allyl amino)-2 éthoxy)-2 éthoxy] méthyl}-2 diéthoxycarbonyl-3,5 méthyl-6 pentafluorophényl-4 dihydro-1,4 pyridine

Un mélange contenant 1,6 g du composé obtenu au stade précédent, 0,31 g de bromure d'allyle et 0,18 g de carbonate de potassium dans 20 ml d'acétonitrile est porté à reflux sous agitation pendant une nuit. Après évaporation du solvant, le résidu est repris à l'éther, épuisé à l'acide chlorhydrique 0,1 N basifié à froid et ensuite extrait à l'acétate d'éthyle pour obtenir après évaporation une huile correspondant à la structure attendue.

### STADE C

0,9 g du composé précédemment obtenu, 0,125 g d'acide oxalique, 0,3 g d'hydroxyde de palladium en solution dans 50 ml de méthanol, sont soumis à une hydrogénation catalytique à température ambiante et sous pression atmosphérique. Après filtration du catalyseur, on obtient 0,5 g du sel attendu.
Rendement : 54%
Point de fusion : 151-153° C

Les constantes physiques spectrales de ce sel sont indiquées dans le Tableau I.

### EXEMPLE 17 :

### Fumarate de (4R,S) {[(amino-2 éthoxy)-2 éthoxy] méthyl}-2 éthoxycarbonyl-3 méthoxycarbonyl-5 méthyl-6 (trifluorométhyl-2 phényl)-4 dihydro-1,4 pyridine

Ce composé a été préparé selon le procédé décrit dans l'exemple 1, Stades B et Stade C, mais en utilisant au Stade B la trifluorométhyl-2 benzaldéhyde au lieu de la pentafluorobenzaldéhyde.
Rendement : 9%
Point de fusion : 176-178° C
Analyse élémentaire :

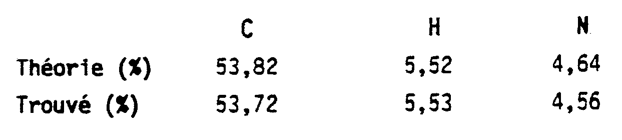

Les constantes physiques spectrales de la base correspondante sont indiquées dans le Tableau I.

### EXEMPLE 18 :

Tartrate de (4R,S) {[(amino-2 éthoxy)-2 éthoxy] méthyl}-2 (chloro-3 phényl)-4 éthoxycarbonyl-3 méthoxycarbonyl-5 méthyl-6 dihydro-1,4 pyridine

Ce composé a été préparé selon le procédé de l'exemple 1, Stades B et C mais en remplaçant la pentafluorobenzaldéhyde par la chloro-3 benzaldéhyde (Stade B) et pour la salification (Stade C) l'acide fumarique par l'acide tartrique.
Rendement : 9,5%
Point de fusion : 118-124° C

Les constantes physiques spectrales de ce sel sont indiquées dans le Tableau I.

### EXEMPLE 19 :

Tartrate de (4R,S) {[(amino-2 éthoxy)-2 éthoxy] méthyl}-2 diméthoxycarbonyl-3,5 méthyl-6 pentafluorophényl-4 dihydropyridine.

Ce composé a été préparé selon le procédé décrit dans l'exemple 1, (Stades B et C) mais en utilisant au Stade B l'ester méthylique de l'acide phtalimido-10 oxo-3 dioxa-5,6 décanoïque au lieu de l'ester éthylique et pour la salification, l'acide tartrique au lieu d'acide fumarique.
Rendement : 15%
Point de fusion : 187-189 ° C
Analyse élémentaire :

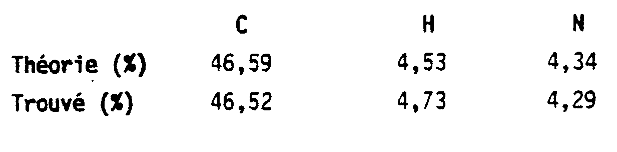

Les constantes physiques spectrales de la (4R,S) {[(amino-2 éthoxy)-2 éthoxy] méthyl}-2 diméthoxycarbonyl-3,5 méthyl-6 pentafluoro-phényl-4dihydropyridine sont indiquées dans le Tableau I.

### EXEMPLE 20 :

Tartrate de (4R,S) {[(amino-2 éthoxy)-2 éthoxy] méthyl}-2 éthoxycarbonyl-5 méthoxycarbonyl-3 méthyl-6 pentafluorophényl-4 dihdyropyridine.

Ce composé aussi a été préparé selon le procédé décrit dans l'exemple 1 mais en utilisant au stade B le crotonate d'éthyle au lieu du crotonate de méthyle.
Rendement : 6%
Point de fusion : 190-192° C
Analyse élémentaire

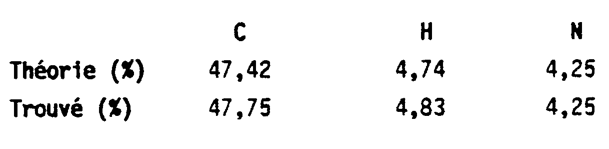

Les constantes physiques spectrales de la base correspondante sont indiquées dans le Tableau I.

### EXEMPLE 21 :

(4R,S) {[((N-trifluoroacétylamino)-2 éthoxy)-2 éthoxy] méthyl}-2 éthoxycarbonyl-3 méthoxycarbonyl-5 méthyl-6 pentafluorophényl-4 dihydro-1,4 pyridine

Pour obtenir ce composé, on soumet le composé de l'exemple 1 à l'action de la triéthylamine et de l'ester éthylique de l'acide trifluoroacétique dans le méthanol, à température ambiante pendant environ 48 heures. Après Après filtration du précipité formé, laver avec du méthanol glacé (14° C), et on obtient le produit attendu pur.
Point de fusion : 148-150° C
Analyse élémentaire :

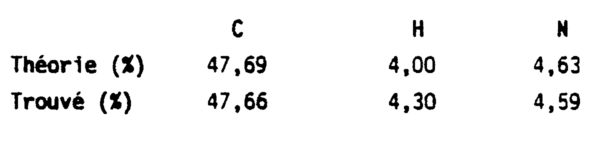

Les constantes physiques spectrales de cette base sont indiquées dans le Tableau I.

### EXEMPLE 22 :

### Fumarate de (4R,S) {[(amino-2 éthoxy)-2 éthoxy] méthyl}-2 éthoxycarbonyl-3 méthoxycarbonyl-5 (méthoxy-2 méthylthio-3 phényl)-4 dihydro-1,4 pyridine

Ce composé a été obtenu avec le procédé de synthèse décrit dans l'exemple 1 (Stades B et C) mais en utiliant au lieu de la pentafluorobenzaldéhyde la méthoxy-2 méthylthio-3 benzaldéhyde.

La préparation de cette dernière substance est connue (Bull.Soc.Chim. of Japan (1978),51,(8),p. 2435-2436).
Rendement : 13,5%
Point de fusion : 1200 C
Analyse élémentaire :

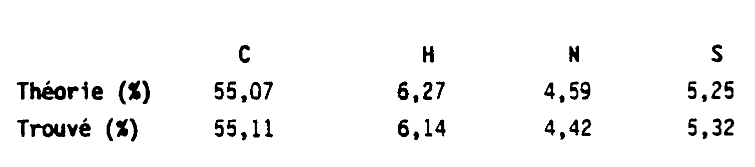

Les constantes physiques spectrales de ce sel sont indiquées dans le Tableau I.

### EXEMPLE 23 :

Tartrate de (4R,S) {[(amino-2 éthoxy)-2 éthoxy] méthyl}-2 (nitro-4 benzyloxycarbonyl)-3 méthoxy-5 méthyl-6 pentafluorophényl-4 dihydro-1,4 pyridine

### STADE A

Ester nitro-4 benzylique de l'acide phtalimido-10 oxo-3 dioxa-5,8 décanoïque

Laisser pendant une nuit sous azote, 35 g d'acide phtalimido-8 dioxa-3,6 octanoïque avec 18 g de carbonyldiimidazole dans 750 ml d'un mélange de diméthylformamide et d'acétonitrile (3:1). Ajouter ensuite 34 g (nitro-4 benzyloxycarbonyl)-2 acétate de magnésium et laisser en contact pendant 18 heures. Après évaporation du solvant réactionnel, reprendre le résidu au dichlorométhane, et laver à l'eau. Le résidu est ensuite purifié sur colonne de gel de silice en utilisant comme éluant un mélange de dichlorométhane et d'acétone (95:5) pour donner le composé attendu. Rendement : 32,8%

Spectre de résonance magnétique nucléaire du proton (CDCl₃) :
3,6 ppm,s,6H; 3,7-4 ppm,m,4H; 4,2 ppm,s,2H; 5,3 ppm,s,2H; 7,8 ppm,m,4H; 7,6 ppm,d,2H; 8,3 ppm,d,2H.

### STADE B

La {[(amino-2 éthoxy)-2 éthoxy] méthyl}-2 (nitro-4 benzyloxycarbonyl)-3 méthoxy-5 méthyl-6 pentafluorophényl-4 dihydro-pyridine a été préparée selon le procédé décrit dans l'exemple 1 Stades B et C en condensant le composé obtenu au stade précédent avec le pentafluorobenzaldéhyde. Ses constantes physiques spectrales sont indiquées dans le Tableau I.
Rendement : 50%
Analyse élémentaire :

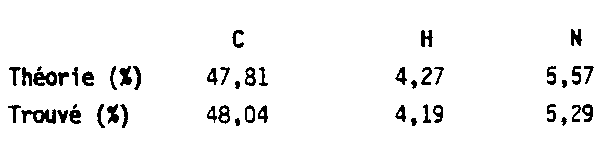

Le sel correspondant a été obtenu après addition d'une quantité adéquate d'acide tartrique en solution dans l'éthanol. Point de fusion : 152-154° C

### EXEMPLE 24 :

Tartrate de (4R,S) {[(amino-3 propoxy)-3 propoxy] méthyl}-2 éthoxycarbonyl-3 méthoxycarbonyl-5 pentafluorophényl-4 dihydro-1,4 pyridine

### STADE A :

Ester éthylique de l'acide phtalimido-12 oxo-3 dioxa-5,9 dodécanoïque

Ce composé a été préparé selon le procédé décrit dans le stade A de l'exemple 1 mais en utilisant le (phtalimido-3 propoxy)-3 propanol

Spectre de résonance magnétique nucléaire du proton (CDCl₃) : 1,3 ppm,t,3H; 1,6-2,2 ppm,m,4H; 3,3-4,0 ppm,m,10H; 4,1 ppm,s,2H; 4,2 ppm,q,2H; 7,7-,3 ppm,m,4H.

### STADE B :

La {[(amino-3 propoxy)-3 propoxy] méthyl}-2 éthoxycarbonyl-3 méthoxycarbonyl-5 pentafluorophényl-4 dihydro-1,4 pyridine a été préparée selon le procédé décrit dans l'exemple 1, Stades B et C, en utilisant le composé obtenu ci-dessus.

Ses constantes physiques spectrales sont indiquées dans le Tableau I.

Le tartrate correspondant a été formé après addition d'une quantité adéquate d'acide tartrique. Rendement : 11 %
Point de fusion : 104° C
Analyse élémentaire :

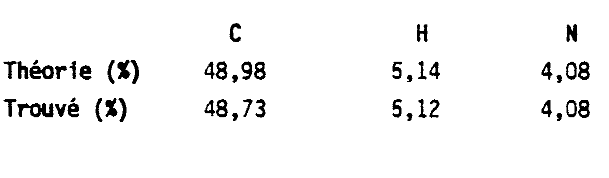

### EXEMPLE 25 :

Fumarate de (4R,S) {[(amino-2 éthoxy)-2 éthoxy] méthyl}-2 (dichloro-2,3 phényl)-4 isopropoxycarbonyl-3 méthoxycarbonyl-5 méthyl-6 dihydro-1,4 pyridine

### STADE A

Ester isopropylique de l'acide phtalimido-10 oxo-3 dioxa-5,8 décanoïque

Ce composé a été préparé selon le procédé décrit dans l'exemple 4 stade A, mais en utilisant l'alcool isopropylique au lieu du dicyclopropyl-2,2 éthanol.
Rendement : 67%
Spectre de résonance magnétique nucléaire du proton (CDCl₃) : 1,3 ppm,d,6H; 3,5 ppm,s,2H; 3,7 ppm,s,4H;
3,7-4,2 ppm,m,4H; 4,2 ppm,s,2H; 4,8-5,4 ppm,m,1 H; 7,6-8,2 ppm,m,4H.

### STADE B

La (4R,S) {[(amino-2 éthoxy)-2 éthoxy] méthyl}-2 (dichloro-2,3 phényl)-4 isopropoxycarbonyl-3 méthoxycarbonyl-5 méthyl-6 dihydro-1,4 pyridine a été préparée à partir du composé obtenu au stade précédent et la dichloro-2,3 benzaldéhyde selon le procédé décrit dans l'exemple 1 Stades B et C. Ses constantes physiques spectrales sont indiquées dans le Tableau I. Rendement : 17,5%

Cette base a été ensuite salifiée par l'acide fumarique pour former le sel correspondant. Point de fusion : 106° C (décomposition) Analyse élémentaire :

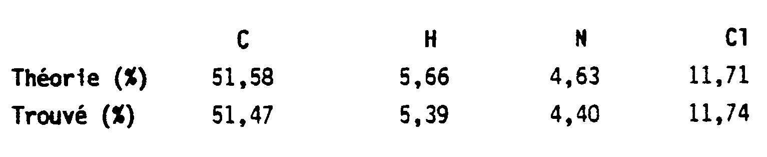

### EXEMPLE 26 :

Tartrate de (4R,S) {[(amino-2 éthoxy)-2 éthoxy] méthyl}-2 isopropoxycarbonyl-3 méthoxycarbonyl-5 méthyl-6 pentafluorophényl-4 dihydro-1,4 pyridine

La base correspondante a été préparée selon le procédé décrit dans l'exemple précedent mais en utilisant le pentafluorobenzaldéhyde au lieu du dichloro-2,3 benzaldéhyde.
Rendement : 9%
Point de fusion : 56,9° C

Le tartrate de (4R,S) {[(amino-2 éthoxy)-2 éthoxy] méthyl}-2 isopropoxycarbonyl-3 méthoxycarbonyl-5 méthyl-6 pentafluorophényl-4 dihydro-1,4 pyridine a été formé après addition d'une quantité adéquate de l'acide (d,l) tartrique en solution dans l'éthanol.
Point de fusion : 130° C
Analyse élémentaire :

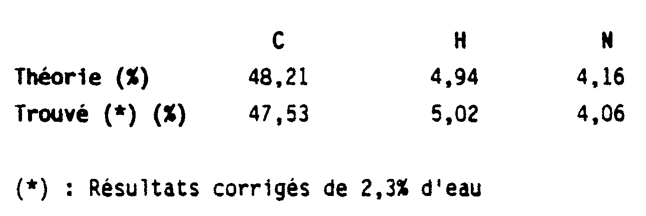

Les constantes physiques spectrales de la base sont indiquées dans le Tableau I.

### EXEMPLE 27:

Fumarate de (4R,S) {[(amino-3 propoxy)-3 propoxy] méthyl}-2 (dichloro-2,3 phényl)-4 éthoxycarbonyl-3 méthoxycarbonyl-5 méthyl-6 dihydro-1,4 pyridine

Ce composé a été préparé selon le procédé décrit dans l'exemple 24 mais en utilisant le dichloro-2,3 benzaldéhyde au lieu du pentafluorobenzaldéhyde et pour la salification l'acide fumarique à 2% en solution dans l'éthanol au lieu de l'acide tartrique. Les constantes physiques spectrales de la base sont indiquées dans le Tableau I.
Rendement : 25%
Point de fusion : 131-133° C
Analyse élémentaire :

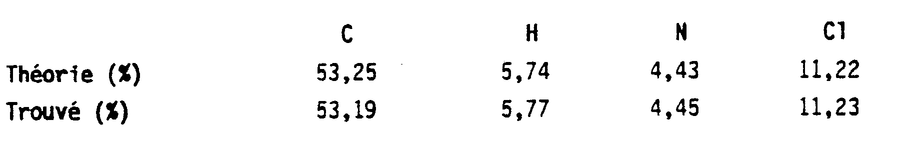

### EXEMPLE 28 :

Fumarate de (4R,S) {[(amino-2 éthoxy)-2 éthoxy] méthyl}-2 (dichloro-2,3 phényl)-4 isobutyloxycarbonyl-3 méthoxycarbonyl-5 méthyl-6 dihydro-1,4 pyridine

### STADE A :

Ester isobutylique de l'acide phtalimido-10 oxo-3 dioxa-5,8 décanoïque

Ce composé a été préparé selon le procédé décrit dans l'exemple 4, Stade A, mais en utilisant l'alcool isobutylique au lieu du dicyclopropyl-2,2, éthanol.
Rendement : 55%
Spectre de résonance magnétique nucléaire du proton (CDCl₃) : 0,9 ppm,d,6H; 1,5-2,4 ppm,m,1H; 3,6 ppm,s,2H; 3,5-4,2 ppm,m,8H; 4 ppm,d,2H; 4,2 ppm,s,2H; 7,5-8 ppm,m,4H

### STADE B

Le fumarate de (4R,S) {[(amino-2 éthoxy)-2 éthoxy] méthyl}-2 (dichloro-2,3 phényl)-4 isobutyloxycarbonyl-3 méthoxycarbonyl-5 méthyl-6 dihydro-1,4 pyridine a été préparé à partir du composé obtenu au stade précédent et selon le procédé décrit dans l'exemple 25 stade B. Ses constantes physiques spectrales sont indiquées dans le Tableau I.
Rendement : 17%
Point de fusion : 130° C
Analyse élémentaire :

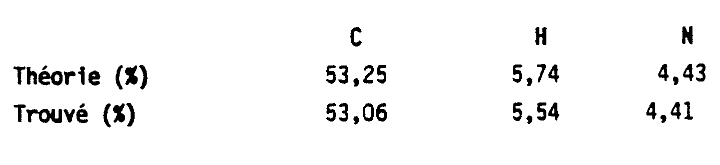

### EXEMPLE 29 :

Tartrate de (4R,S) {[(amino-2 éthoxy)-2 éthoxy] méthyl}-2 (dichloro-2,3 phényl)-4 [-(dicyclopropyl-4,4 butène-2E) oxycarbonyl]-3 méthoxycarbonyl-5 méthyl-6 dihydro-1,4 pyridine

### STADE A

Ester éthylique de l'acide dicyclopropyl-4,4 butène-3E oique

Un mélange de 0,383 moles de dicyclopropyl-2,2 acétaldéhyde et de 0,574 moles de (carboéthoxy méthylène) triphénylphosphorane dans 800 mi de toluène sont portés à reflux pendant 24 heures. Après élimination du précipité formé, le solvant est évaporé et l'huile résiduelle est dissoute dans 1575 ml de diméthylformamide. Après addition de 1575 ml d'acide sulfurique 3N, le milieu réactionnel est extrait par 3 litres d'hexane. La phase organique est ensuite séchée et évaporée à sec pour obtenir l'ester attendu.
Rendement : 79%
Spectre de résonance magnétique nucléaire du proton (CDCl₃) : 0,0-1,4 ppm,m,11H; 1,5 ppm,t,3H; 6 ppm,d,1H; 7,1 ppm,d,1H.

### STADE B

Dicyclopropyl-4,4 butène-3E ol-1

Refroidir à 0° C 656 ml une solution d'isobutyl hydruroaluminium 1,5M dans le toluène et ajouter lentement 68,5 g de l'ester obtenu au stade précédent. Laisser reposer une nuit à température ambiante et hydrolyser d'abord avec 500 ml d'un mélange de toluène et de méthanol (1:1) et ensuite avec 1 1 d'acide chlorhydrique 1 N. Après distillation, on obtient l'alcool attendu.
Rendement : 75%
Spectre de résonance magnétique nucléaire du proton (CDCl₃) : 0-1,5 ppm, m,11 H; 1,5-2 ppm,m,1H; 4,0 - 4,3 ppm,m,2H; 5,6-5,9 ppm,m,15H

### STADE C

Ester dicyclopropyl-4,4 butène-2E ique de l'acide phtalimido-10 oxo-3 dioxa-5,8 décanoïque

Ce composé a été préparé selon le procédé décrit dans l'exemple 4 stade A en utilisant l'alcool obtenu au stade précédent.
Rendement : 50%

### STADE D

La (4R,S) {[(amino-2 éthoxy)-2 éthoxy] méthyl}-2 (dichloro-2,3 phényl)-4 [(dicyclopropyl-4,4 butène-2E) oxycarbonyl]-3 méthoxycarbonyl-5 méthyl-6 dihydro-1,4 pyridine a été obtenue en utilisant l'ester préparé au stade précédent et selon le procédé décrit dans l'exemple 1, Stades B et C.

Les constantes physiques spectrales de cette base sont indiquées dans le Tableau I.

Après salification avec l'acide (d,l) tartrique, on obtient le tartrate correspondant sous forme solide.
Rendement : 18%
Point de fusion : 146° C
Analyse élémentaire :

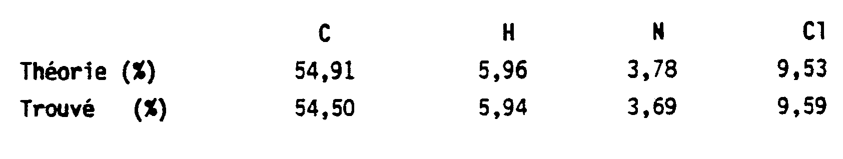

### EXEMPLE 30 :

Fumarate de (4R,S) {[(amino-3 propoxy)-3 propoxy] méthyl}-2 éthoxycarbonyl-3 méthoxycarbonyl-5 méthyl-6 (méthylthio-2 trifluorométhyl-3 phényl)-4 dihydro-1,4 pyridine.

### STADE A :

Chloro-2 trifluorométhyl-3 benzaldéhyde

Refroidir à -65° C, 0,6 mole de chloro-2 trifluorométhylbenzène en solution dans 1 I de tétrahydrofuranne et ajouter 0,58 mole de butyllithium en solution dans l'hexane. Laisser à la même température pendant 2 heures et ajouter ensuite goutte à goutte un mélange contenant 44 ml de diméthylformamide et 200 ml de tétrahydrofuranne. Laisser le milieu réactionnel revenir à température ambiante, ajouter 600 ml d'eau, extraire ensuite à l'éther éthylique et évaporer à sec. Distiller pour purifier.
Rendement : 40%
Spectre de résonance magnétique nucléaire du proton (CDCl₃) : 7,5-7,9 ppm,m,2H; 8,0-8,4 ppm,m,2H.

### STADE B :

Méthylthio-2 trifluorométhyl-3 benzaldéhyde

On ajoute à une suspension contenant 0,45 mole de méthylsulfure de sodium dans 190 ml de diméthylformamide 0,45 mole d'aldéhyde obtenu au stade précédent en solution dans 250 ml de diméthylformamide. Chauffer le milieu à 55° C, laisser revenir à température ambiante, hydrolyser à l'aide de 500 ml d'eau, extraire à l'éther, sécher et distiller pour obtenir l'aldéhyde attendu.
Rendement : 30%
Spectre de résonance magnétique nucléaire du proton (CDCl₃) : 2,4 ppm, s,3H; 7,4-8,3 ppm,m,3H; 10,9 ppm,s,1H.

### STADE C :

La (4R,S) {[(amino-3 propoxy)-3 propoxy] méthyl}-2 éthoxycarbonyl-3 méthoxycarbonyl-5 méthyl-6 (méthylthio-2 trifluorométhyl-3 phényl)-4 dihydro-1,4 pyridine a été préparée selon le procédé décrit dans l'exemple 1, Stades B et C en utilisant l'aldéhyde décrit ci-dessus. Le sel correspondant a été obtenu en utilisant une quantité adéquate d'acide fumarique. Les constantes physiques de ce composé sont décrites dans le Tableau I.
Rendement : 15%
Point de fusion : 158° C
Analyse élémentaire :

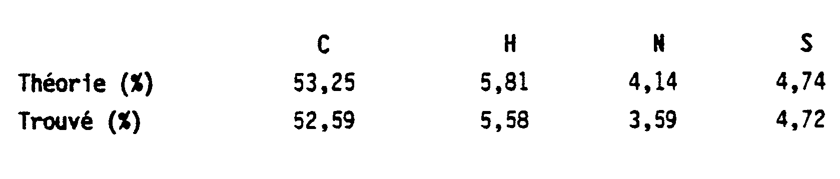

### EXEMPLE 31 :

Fumarate de (4R,S) {[(amino-2 éthoxy)-2 éthoxy] méthyl}-2 éthoxycarbonyl-3 méthoxycarbonyl-5 méthyl-6 (trichloro-2,3,5 phényl)-4 dihydro-1,4 pyridine.

Ce composé a été obtenu selon le procédé décrit dans l'exemple 1, stades B et C, en utilisant le trichloro-2,3,5 benzaldéhyde et la quantité adéquate d'acide fumarique.
Rendement : 21 %
Point de fusion : 114° C
Analyse élémentaire :

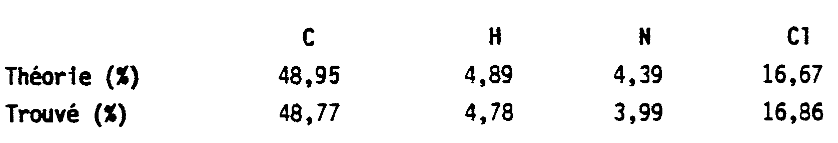

Les constantes physiques spectrales du composé sont données dans le Tableau I.

### EXEMPLE 32 :

Fumarate de (4R,S) {[(amino-2 éthoxy)-2 éthoxy] méthyl}-2 éthoxycarbonyl-3 méthoxycarbonyl-5 méthyl-6 (trichloro-2,3,6 phényl)-4 dihydro-1,4 pyridine.

Ce composé a été préparé selon le procédé décrit dans l'exemple 31 mais en utilisant le trichloro-2,3,6 benzaldéhyde au lieu du trichloro-2,3,6 benzaldéhyde.
Point de fusion : 118° C
Analyse élémentaire :

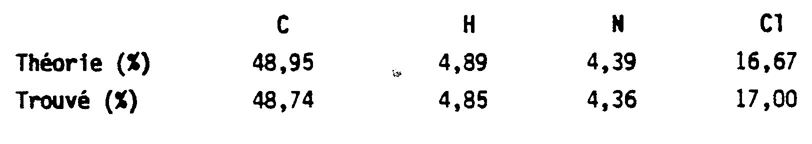

Les constantes physiques spectrales de la base correspondante sont décrites dans le Tableau I.

### EXEMPLE 33 :

Fumarate de (4R,S) {[(amino-2 éthoxy)-2 éthoxy] méthyl}-2 (dichloro-2,3 phényl)-4 (dicyclopropyl-4,4 butyloxycarbonyl)-3 méthoxycarbonyl-5 méthyl-6 dihydro-1,4 pyridine.

### STADE A :

Ester éthylique de l'acide dicyclopropyl-4,4 butanoïque

0,298 mole de l'ester obtenu au Stade A de l'exemple 29 sont dissouts dans 500 ml d'éthanol et ensuite ils sont soumis à une hydrogénation catalytique à température ambiante et en présence de palladium sur charbon à 5%.
Rendement : 94,5%
Spectre de résonance magnétique nucléaire du proton (CDCl₃) : 1,0 ppm, m,11 H; 1,3 ppm,t,3H; 1,85 ppm,q,2H; 2,5 ppm,t,2H; 4,2 ppm,q,2H.

### STADE B :

Dicyclopropyl-4,4 butanol

Soumettre 0,1 mole de l'ester obtenu au stade précédent dans 400 ml d'éther éthylique à l'action de 0,1 mole d'hydrure de lithium et d'aluminium. Hydrolyser et ensuite distiller pour obtenir l'alcool attendu. Rendement : 93%

### STADE C :

Ester dicyclopropyl-4,4 butylique de l'acide phtalimido-10 oxo-3 dioxa-5,8 décanoïque

Ce composé a été préparé selon le procédé décrit dans l'exemple 4, stade A, mais en utilisant l'alcool obtenu ci-dessus au lieu du dicyclopropyl-2,2 éthanol.
Rendement : 48%
Spectre de résonance magnétique nucléaire du proton (CDCl₃) : 0,0-0,7 ppmp,m,10H; 1,0-2,0 ppm,m, 5H; 3,5-4,3 ppm,t,+s+m,14H; 7,6-8,1 ppm,m,4H.

### STADE D :

Le fumarate de (4R,S) {[(amino-2 éthoxy)-2 éthoxy] méthyl}-2 (dichloro-2,3 phényl)-4 (dicyclopropyl-4,4 butyloxycarbonyl)-3 méthoxycarbonyl-5 méthyl-6 dihydro-1,4 pyridine a été préparé à partir de l'ester décrit au stade C ci-dessus et selon le procédé décrit dans les stades B et C de l'exemple 1.
Rendement : 20%
Point de fusion : 175° C
Analyse élémentaire :

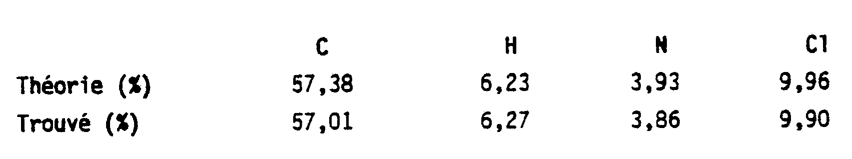

Les constantes physiques spectrales de la base correspondante sont indiquées dans le Tableau I.

### EXEMPLE 34 :

Fumarate de (4R,S) éthoxycarbonyl-3 méthoxycarbonyl-5 méthyl-6 pentafluorophényl-4 {[((N-propyl amino)-2 éthoxy)-2 éthoxy] méthyl}-2 dihydro-1,4 pyridine

### STADE A

(4R,S) éthoxycarbonyl-3 méthoxycarbonyl-5 méthyl-6 pentafluorophényl-4 {[((N-allyl N-benzyl amino)-2 éthoxy)-2 éthoxy] méthyl}-2 dihydro-1,4 pyridine

La (4R,S) éthoxycarbonyl-3 méthoxycarbonyl-5 méthyl-6 pentafluorophényl-4 {[((N-benzyl amino)-2 éthoxy)-2 éthoxy] méthyl}-2 dihydro-1,4 pyridine a été obtenue sous forme d'huile en faisant réagir le composé de l'exemple 1 avec du benzaldéhyde selon le procédé décrit dans l'exemple 11. 0,027 moles de ce composé sont ensuite portés à reflux pendant une nuit sous azote avec 0,027 moles de bromure d'allyle et 0,0135 moles de carbonate de potassium dans 200 ml d'acétonitrile. Après filtration, le solvant réactionnel est évaporé, le résidue est repris à l'eau et l'éther éthylique et ensuite la phase éthérée est épuisée à l'acide chlorhydrique 1N. La phase acqueuse est basifiée et extraite à l'éther. Les phases organiques sont séchées et évaporées à sec pour obtenir le composé attendu.
Rendement : 57%

Spectre de résonance magnétique nucléaire du proton (CDCl₃) : 1,0-1,3 ppm,t,3H; 2,3 ppm,s,3H; 2,5-2,8 ppm,t,2H; 3,0-3,2 ppm,d,2H; 3,4-3,7 ppm, m +s,8H + 3H; 3,8-4,2 ppm,q,2H; 4,6 ppm,s,2H; 4,9-6,1 ppm,m + s + m,1 H + 1 H + 1 H; 7,3 ppm,m,5H; 7,6 ppm,1H échangeable.

### STADE B

Un mélange contenant 0,014 moles du composé obtenu au stade précédent et 0,014 moles d'acide oxalique dans 500 ml de méthanol, est soumis à une hydrogénation catalytique en présence de 3 g d'hydroxyde de palladium à température ambiante et sous pression atmosphérique. Après évaporation et basification, on obtient la (4R,S) éthoxycarbonyl-3 méthoxycarbonyl-5 méthyl-6 pentafluorophényl-4 {[((N-propyl amino)-2 éthoxy)-2 éthoxy] méthyl}-2 dihydro-1,4 pyridine que l'on transforme en son fumarate. Rendement : 59%
Point de fusion : 124° C
Analyse élémentaire :

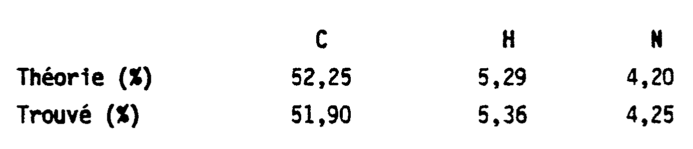

Les constantes physiques spectrales de la base sont données dans le Tableau I.

### EXEMPLE 35 :

Tartrate de (4R,S) {[(amino-2 éthoxy-2 éthoxy] méthyl}-2 (chloro-2 trifluorométhyl-3 phényl)-4 isopropoxycarbonyl-3 méthoxycarbonyl-5 méthyl-6 dihydro-1,4 pyridine.

Ce composé sous forme de base a été préparé selon le procédé décrit dans l'exemple 1 Stades B et C en utilisant le chloro-2 trifluorométhyl-3 benzaldéhyde décrit dans l'exemple 30 Stade A, et l'ester isopropylique de l'acide phtalimido-10 oxo-3 dioxa-5,8 décanoïque. Ses constantes physiques spectrales sont indiquées dans le Tableau I.

Après salification, on obtient le tartrate correspondant.
Rendement : 16%
Point de fusion : 135° C
Analyse élémentaire :

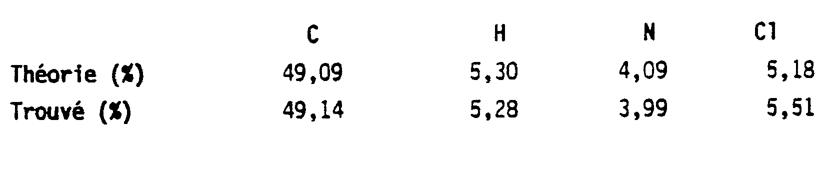

### EXEMPLE 36 :

Fumarate (4R,S) {[(amino-2 éthoxy)-2 éthoxy méthyl}-2 (chloro-2 trifluorométhyl-3 phényl)-4 (dicyclopropyl-4,4 butyloxycarbonyl)-3 méthoxycarbonyl-5 méthyl-6 dihydro-1,4 pyridine.

Ce composé a été préparé selon le procédé décrit dans l'exemple 1 stades B et C en utilisant la chloro-2 trifluorométhyl-3 benzaldéhyde et l'ester dicyclopropyl-4,4 butylique de l'acide phtalimido-10 oxo-3 dioxa-5,8 décanoïque décrit dans l'exemple 33.
Rendement : 10%
Point de fusion : 122° C
Analyse élémentaire :

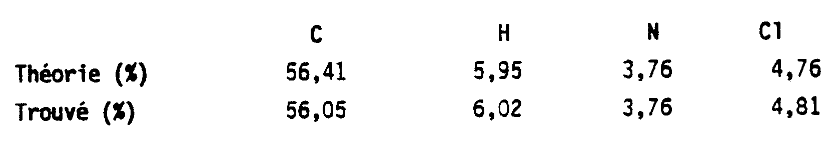

Les constantes physiques spectrales de la base correspondante sont données dans le Tableau I.

### EXEMPLE 37 :

Fumarate de (4R,S) {[(amino-2 éthoxy)-2 éthoxy] méthyl}-2 (chloro-2 trifluorométhyl-3 phényl)-4 [(dicyclopropyl-4,4 butène-2E) oxycarbonyl]-3 méthoxycarbonyl-5 méthyl-6 dihydro-1,4 pyridine.

Ce composé a été préparé selon le procédé décrit dans l'exemple 1, stades B et C, et en utilisant le chloro-2 trifluorométhyl-3 benzaldéhyde et l'ester dicyclopropyl-4,4 butène-2E ique de l'acide phtalimido-10 oxo-3 dioxa-5,8 décanoïque décrit dans l'exemple 29.
Rendement : 13%
Point de fusion : 122° C
Analyse élémentaire :

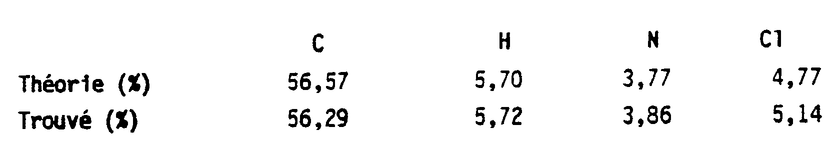

Les constantes physiques spectrales de la base correspondante sont indiquées dans le Tableau I.

### EXEMPLE 38 :

Fumarate de (4R,S) {[(amino-2 éthoxy)-2 éthoxy] méthyl}-2 (chloro-2 trifluorométhyl-3 phényl)-4 isobutyloxycarbonyl-3 méthoxycarbonyl-5 méthyl-6 dihydro-1,4 pyridine.

Ce composé a été préparé aussi selon le procédé décrit dans l'exemple 1 stades B et C en utilisant le chloro-2 trifluorométhyl-3 benzaldéhyde et l'ester isobutylique de l'acide phtalimido-10 oxo-3 dioxa-5,8 décanoïque décrit dans l'exemple 28.
Rendement : 17%
Point de fusion : 118° C
Analyse élémentaire :

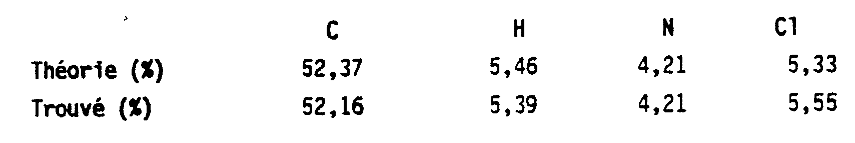

Les constantes physiques spectrales de la base correspondante sont indiquées dans le Tableau I.

### EXEMPLE 39 :

Fumarate de (4R,S) {[(amino-2 éthoxy)-2 éthoxy] méthyl}-2 (dichloro-2,3 phényl)-4 [(méthyl-2 propényl-2) oxycarbonyl]-3 méthoxycarbonyl-5 méthyl-6 dihydro-1,4 pyridine.

### STADE A :

Ester méthyl-2 propène-2 ique de l'acide phtalimido-10 oxo-3 dioxa-5,8 décanoïque

Ce composé a été obtenu selon le procédé décrit dans l'exemple 25 stade A, mais en utilisant le méthyl-2 propène-2 ol au lieu de l'alcool isopropylique.
Rendement : 80%
Spectre de résonance magnétique nucléaire du proton (CDCl₃) : 1,75 ppm,s,3H; 3,55 ppm,s,2H; 3,65 ppm,s,4H; 3,5-4 ppm,m,4H; 4,1 ppm,s,2H; 4,55 ppm,s,2H; 4,95 ppm,m,2H; 7,5-8,0 ppm,m,4H.

### Stade B

Le fumarate de (4R,S) {[(amino-2 éthoxy)-2 éthoxy] méthyl}-2 (dichloro-2,3 phényl)-4 [(méthyl-2 propényl-2) oxycarbonyl]-3 méthoxycarbonyl-5 méthyl-6 dihydro-1,4 pyridine est obtenu à partir de l'ester obtenu au stade précédent et du dichloro-2,3 benzaldéhyde et selon le procédé décrit dans l'exemple 1 stades A et B.
Rendement : 21 %
Point de fusion : 116⁰ C
Analyse élémentaire :

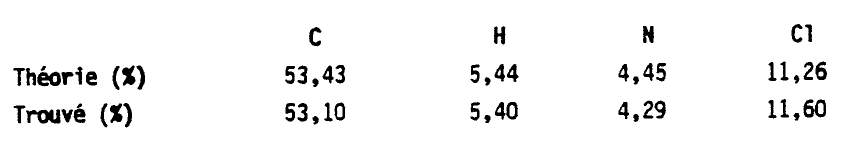

Les constantes physiques spectrales de la base correspondante sont indiquées dans le Tableau I.

### EXEMPLE 40 :

{[(amino-2 éthoxy)-2 éthoxy]-2 éthyl}-2 (dichloro-2,3 phényl)-4 éthoxycarbonyl-3 méthoxycarbonyl-5 méthyl-6 dihydro-1,4 pyridine

### STADE A :

Phényl-11 trioxa-3,6,10 undécanamine

Dans 295 ml de tétrahydrofuranne contenant 0,25 mole d'hydrure de sodium ajouter 0,25 mole d'aminoéthoxy éthanol et porter ensuite pendant 30 mn à reflux. Ajouter ensuite goutte à goutte du oxy-4 phényl-5 pentane sulfonate de sodium en solution dans le tétrahydrofuranne. Porter à reflux, pendant environ 2 heures, ensuite hydrolyser et ajouter 10 ml d'hydroxyde de sodium concentré, évaporer le solvant réactionnel et reprendre le résidu avec un mélange d'eau et d'éther éthylique. Epuiser avec l'acide chlorhydrique 1 N, basifier en refroidissant avec de l'hydroxyde de sodium concentré, extraire par l'éther, sécher et évaporer.
Rendement : 32%
Spectre de résonance magnétique nucléaire du proton (CDCI₃) : 1,9 ppm, m,2H; 2,7 ppm,t,2H; 3,3 à 3,8 ppm,m,10H; 4,5 ppm,s,2H; 7,3 ppm,s,5H.

### STADE B:

(phényl-11 trioxa-3,6,10 undécanyl-1) phtalimide

0,0395 mole de l'amine obtenue au stade précédent et 0,0375 mole d'anhydride phtalique sont portés sous agitation à 150° C pendant 3 heures en éliminant l'eau formée. Après purification sur colonne de silice en utilisant comme solvant d'élution un mélange de dichlorométhane et d'acétate d'éthyle (90:10) on obtient le produit attendu.
Rendement : 67%
Spectre de résonance magnétique nucléaire du proton (CDCI₃) : 1,7-2,1 ppm,m,2H; 3,4-4,0 ppm,m,12H; 4,5 ppm,s,2H; 7,3 ppm,s,5H; 7,5-8,0 ppm,m,4H.

### STADE C :

Dioxa-4,7 phtalimido-9 nonanol

Dans 90 ml d'acétonitrile contenant 0,0216 mole du composé décrit précédemment, et 0,054 mole d'iodure de sodium ajouter 40 ml d'acétonitrile contenant 0,054 mole d'éthérate de trifluorure de bore. Laisser une heure en contact à température ambiante, évaporer le solvant réactionnel, reprendre à l'eau, extraire à l'éther éthylique et laver au thiosulfate. Après purification sur colonne de silice en utilisant comme éluant d'abord du dichlorométhane et ensuite un mélange de dichlorométhane et de méthanol (80:20) on obtient le produit atendu.
Rendement : 28%
Spectre de résonance magnétique nucléaire du proton (CDCl₃) : 1,6-2,0 ppm,m,2H; 2,1-2,6 ppm,m,1 H échangeable; 3,5-4,1 ppm,m,12H; 7,6-8,0 ppm,m,4H.

### STADE D

Ester éthylique de l'acide phtalimido-11 oxo-3 dioxa-6,9 undécanoïque

L'alcool obtenu au stade précédent est transformé en l'acide correspondant par oxydation de Jones. L'acide, ensuite, est soumis à l'action de chlorure de thionyle, d'acide de Meldrum et d'éthanol selon le procédé décrit dans l'exemple 4 Stade A, pour obtenir le composé attendu.

### STADE E

L'{[(amino-2 éthoxy)-2 éthoxy]-2 éthyl}-2 (dichloro-2,3 phényl)-4 éthoxycarbonyl-3 méthoxycarbonyl-5 méthyl-6 dihydro-1,4 pyridine est obtenue à partir de l'ester décrit dans le Stade D et le dichloro-2,3 benzldéhyde selon le procédé décrit dans l'exemple 1, Stades B et C. Les constantes physiques spectrales de ce composé sont indiquées dans le Tableau 1.

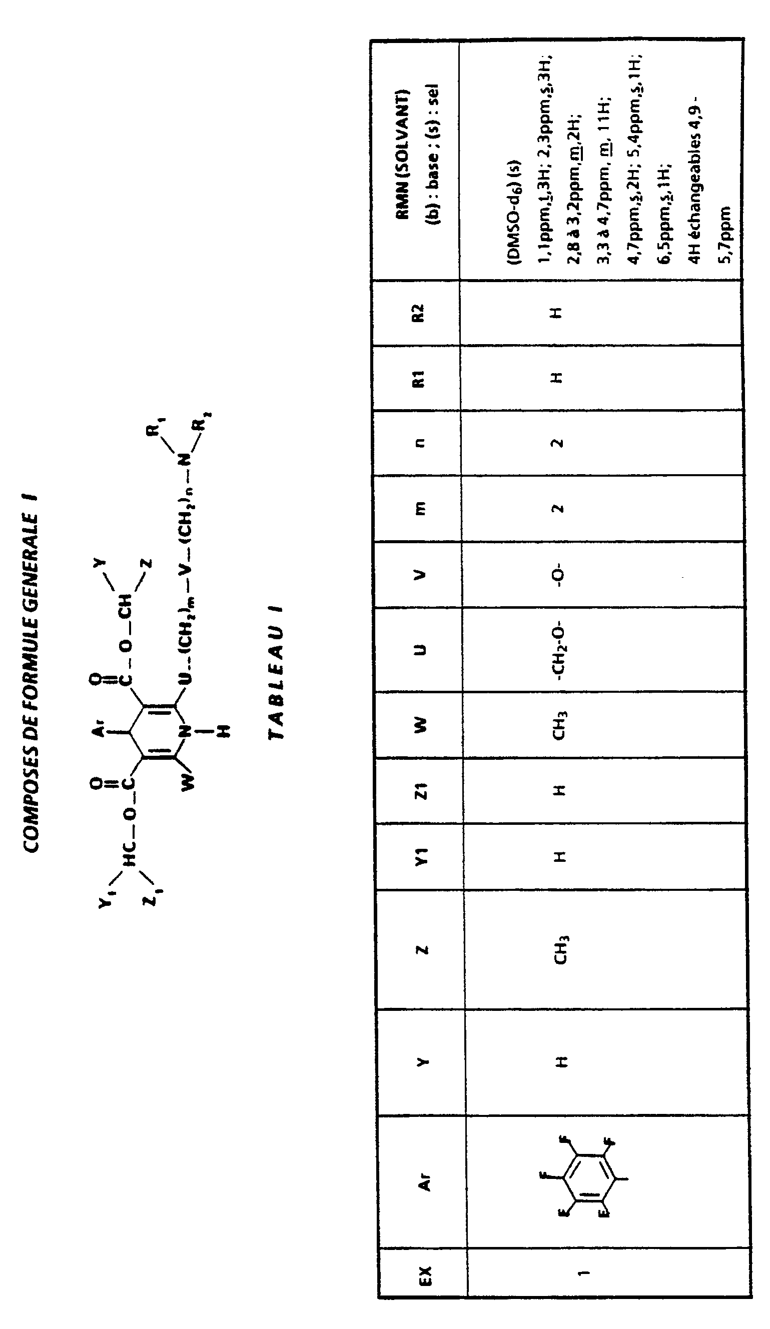

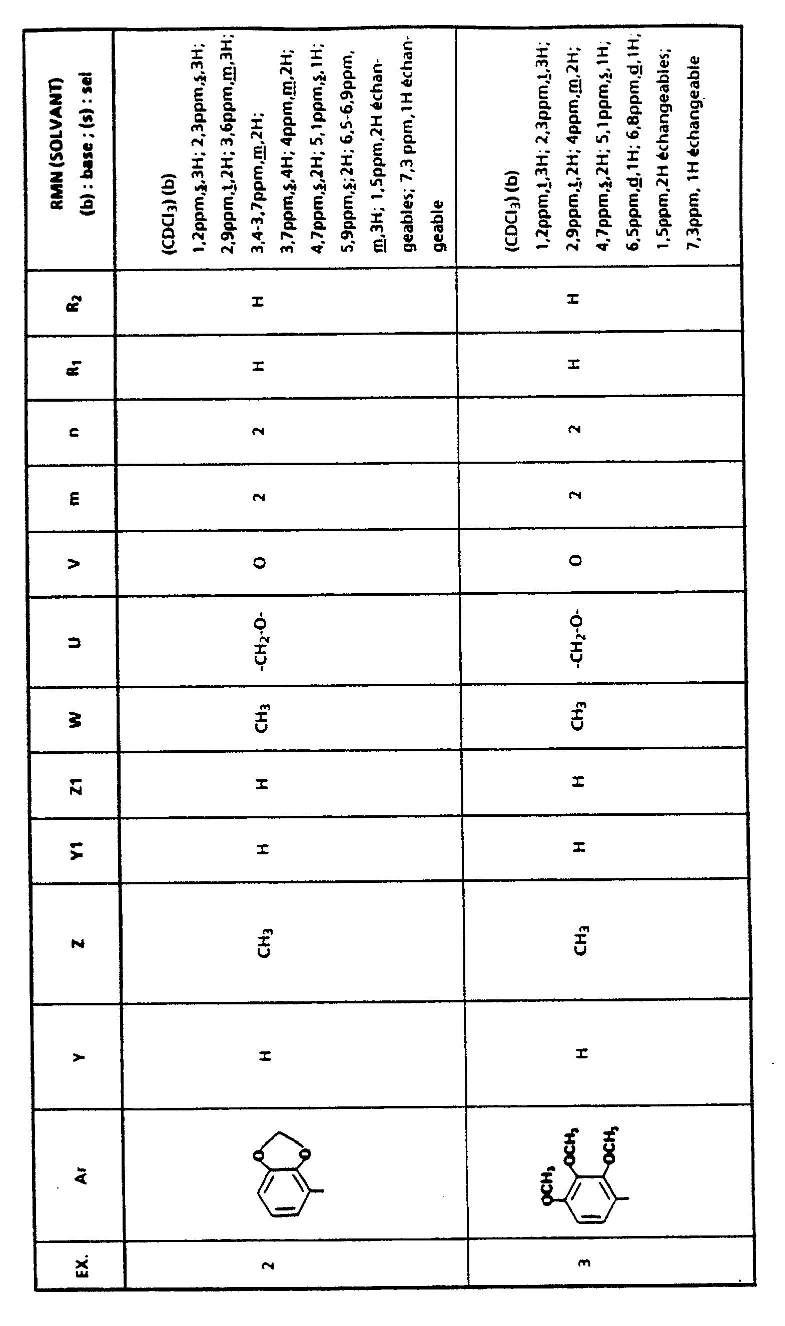

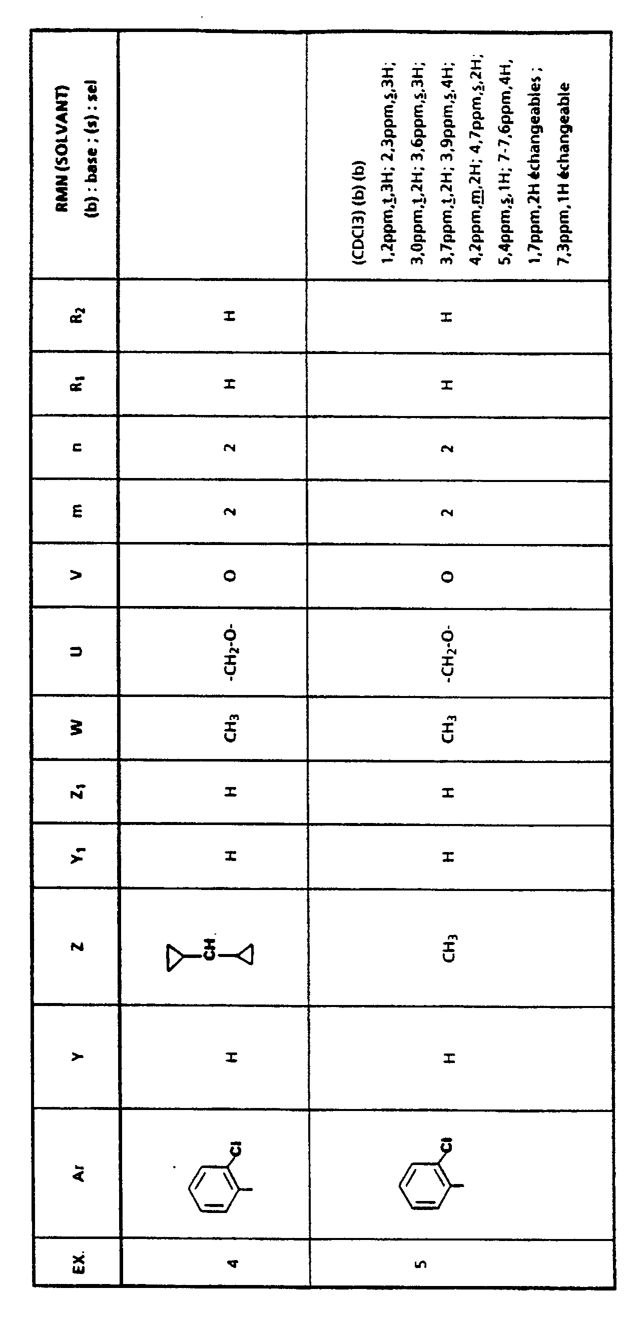

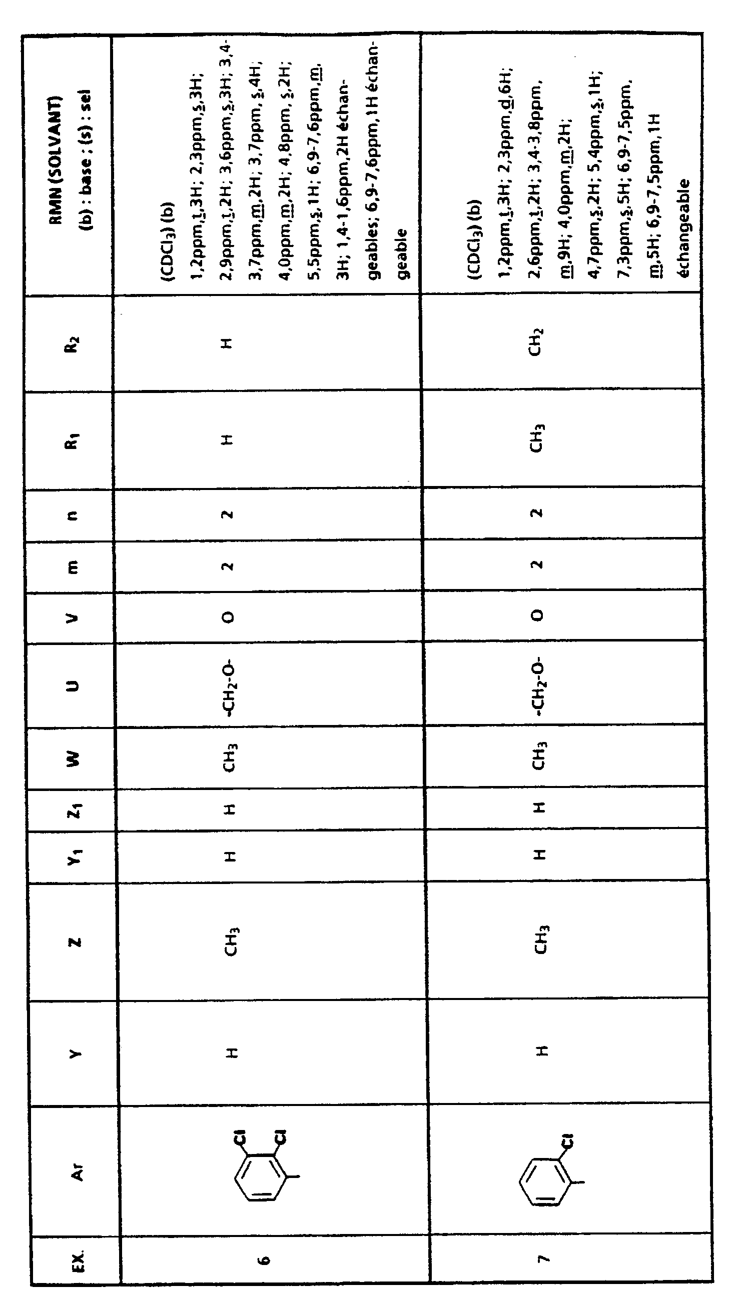

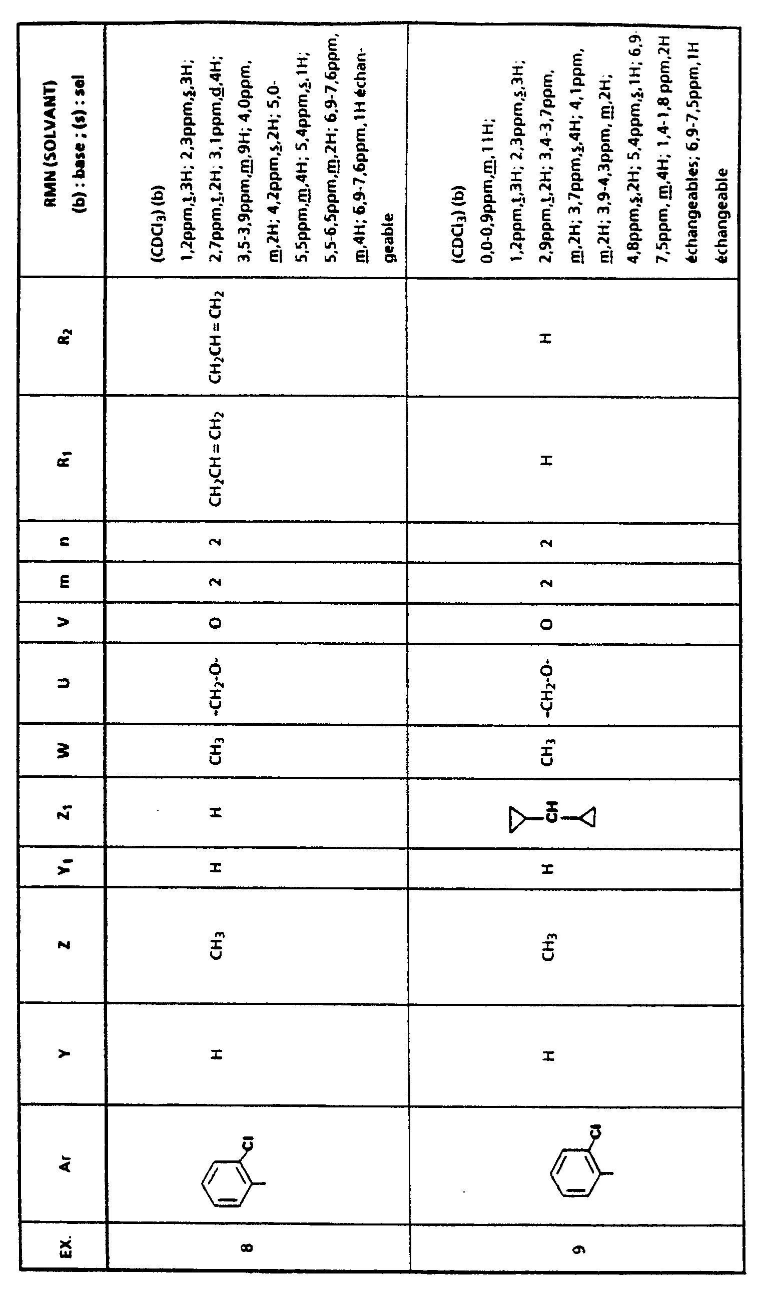

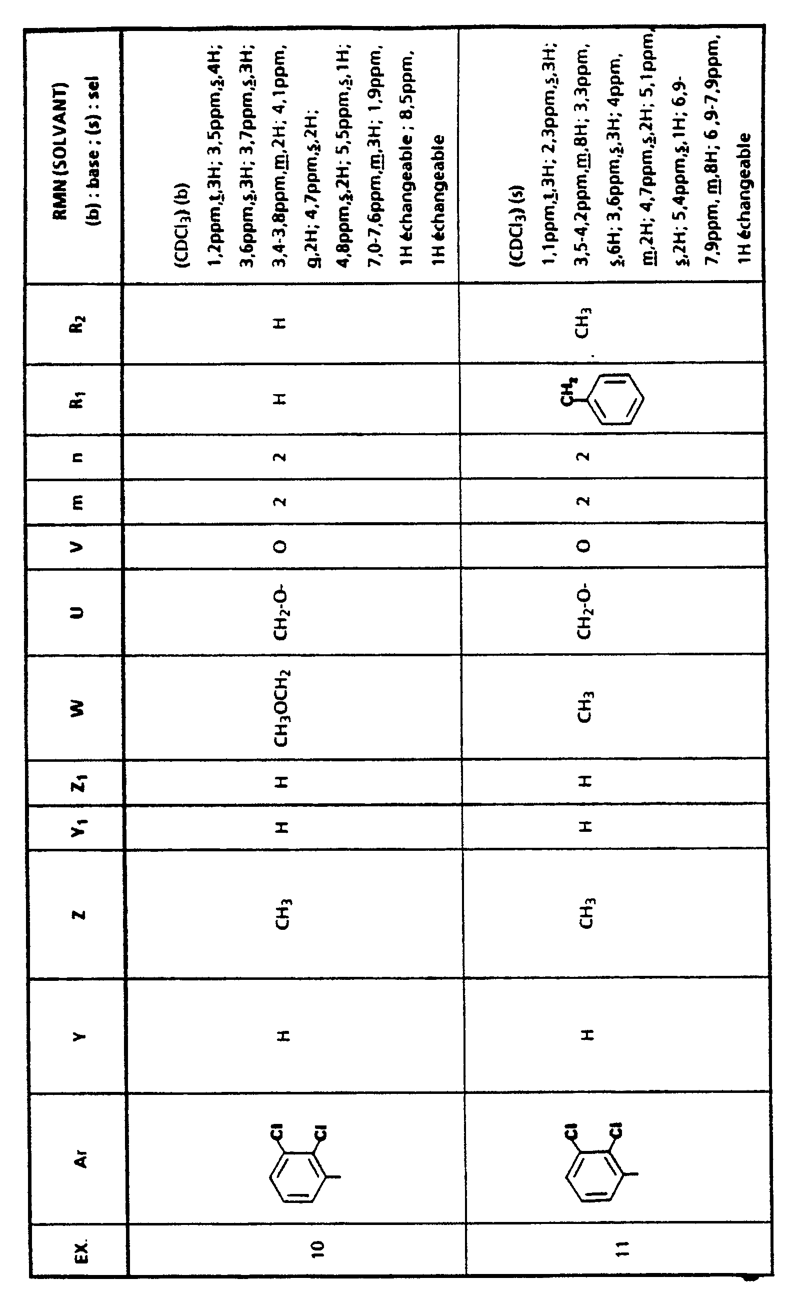

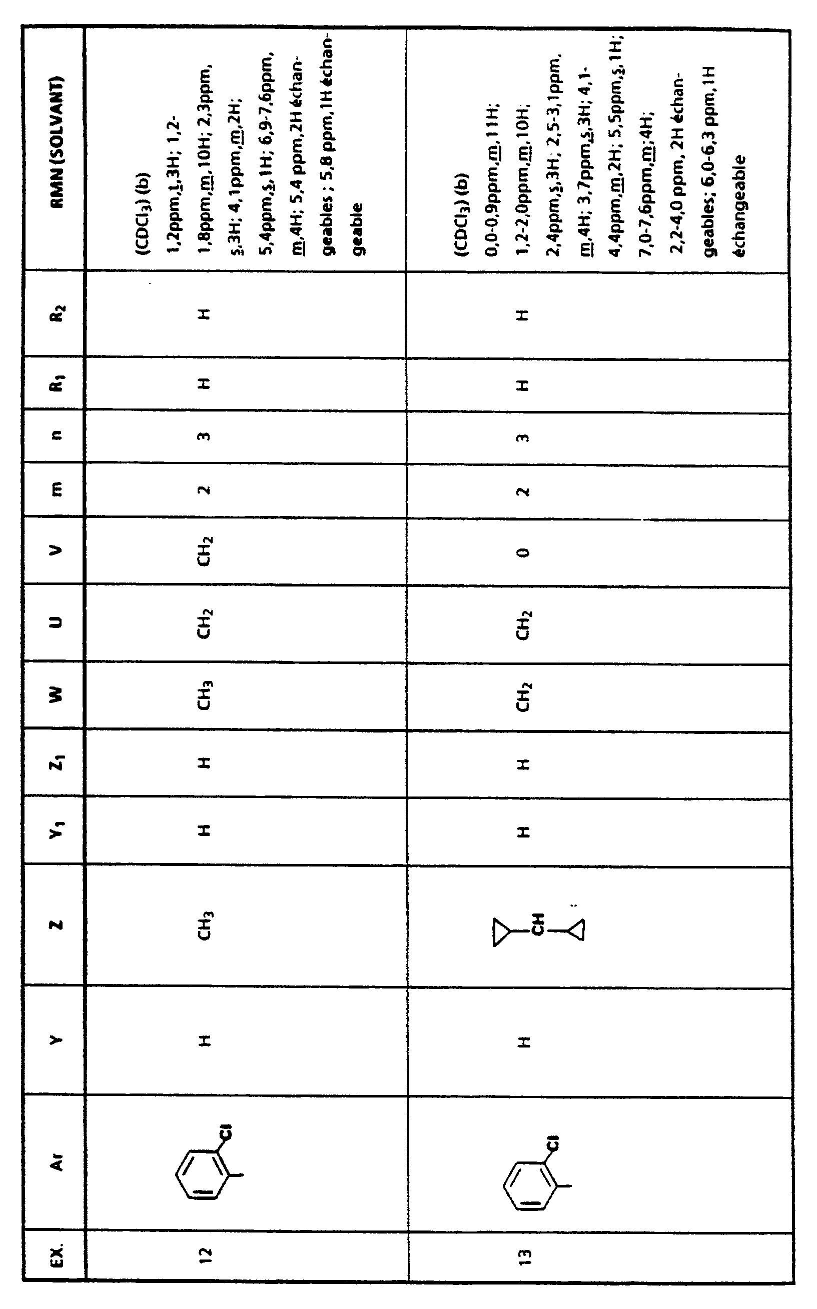

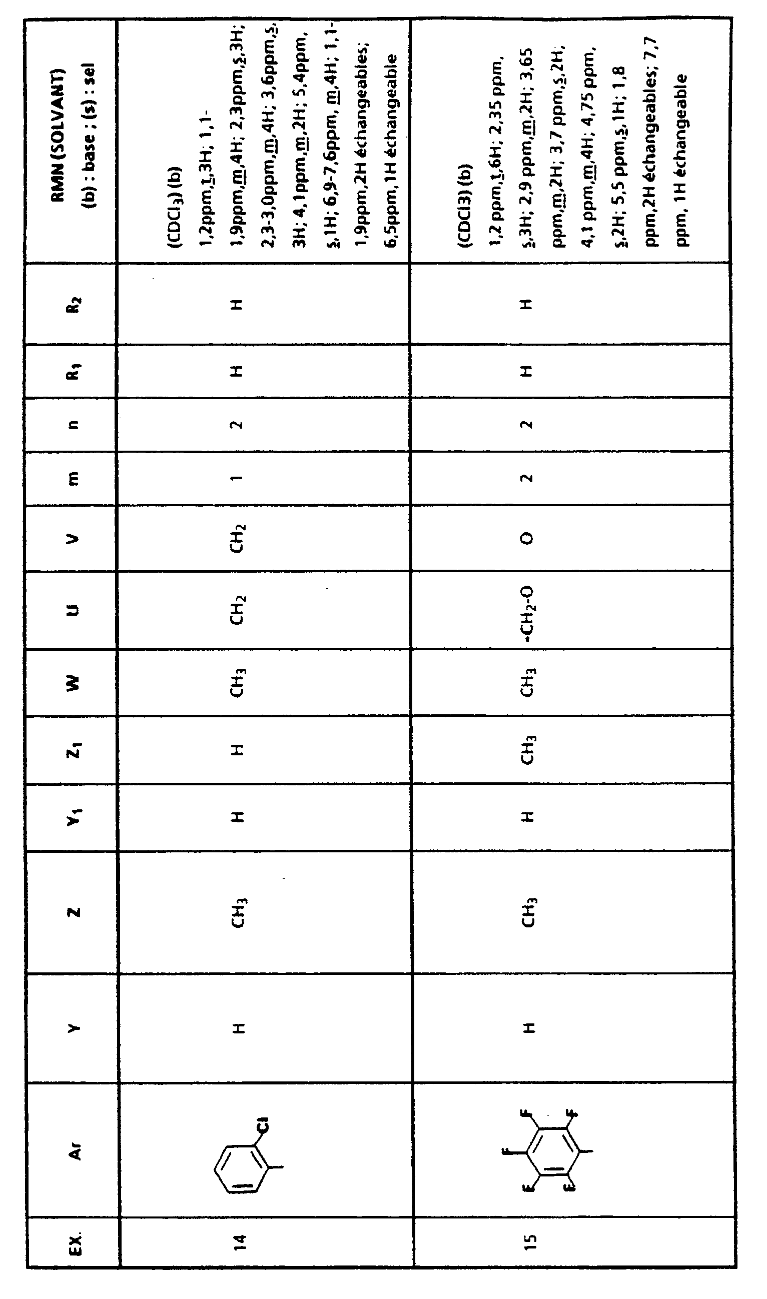

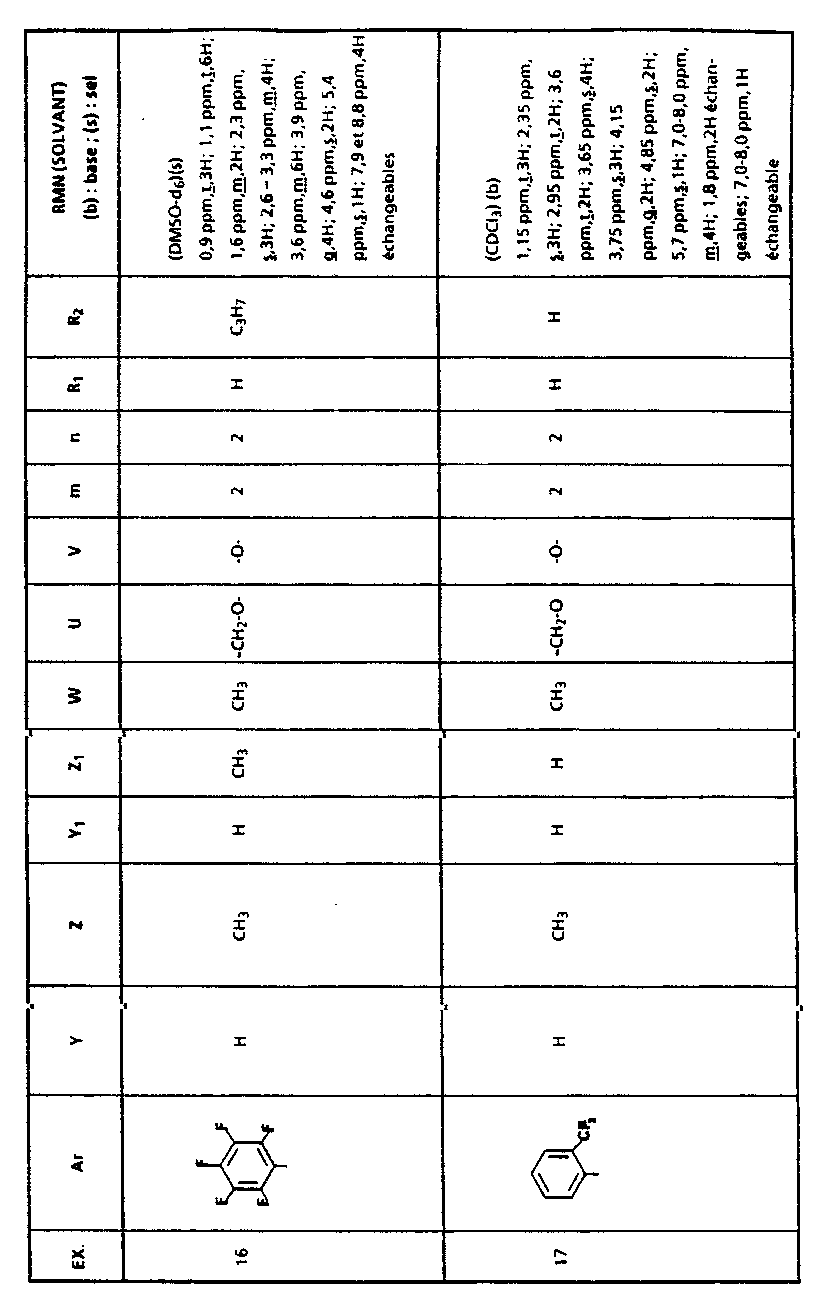

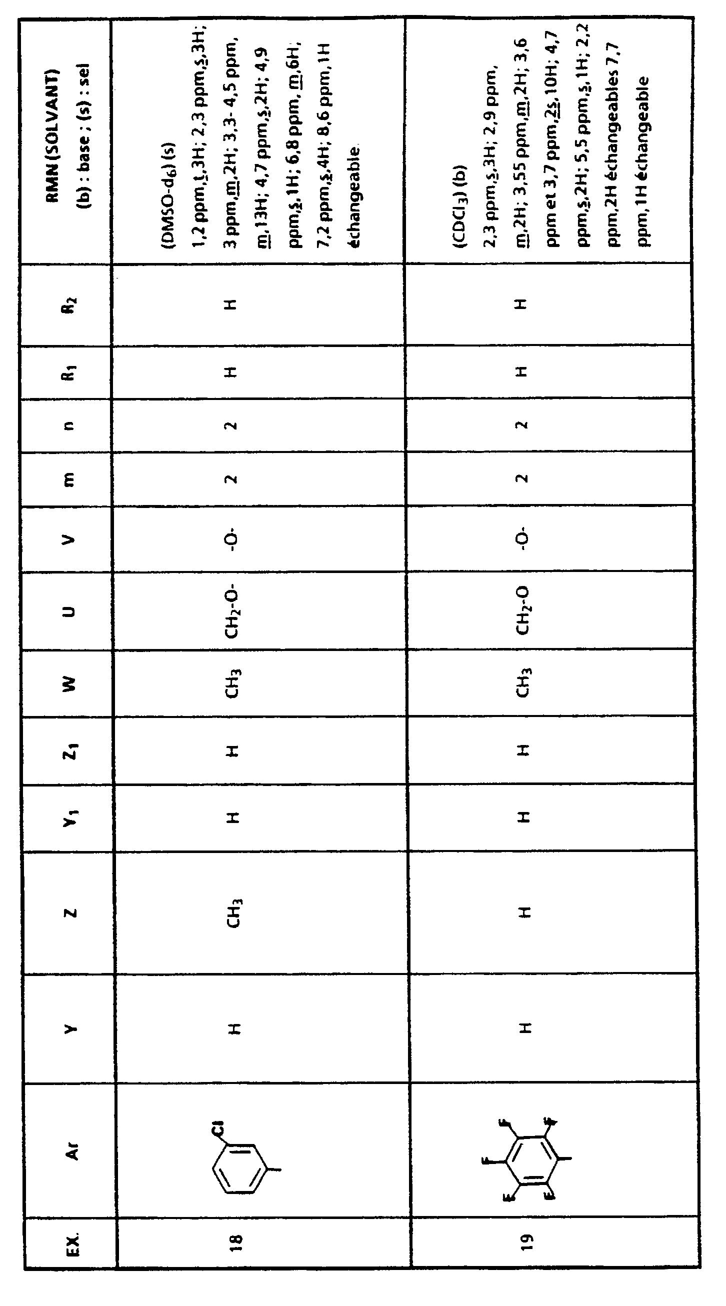

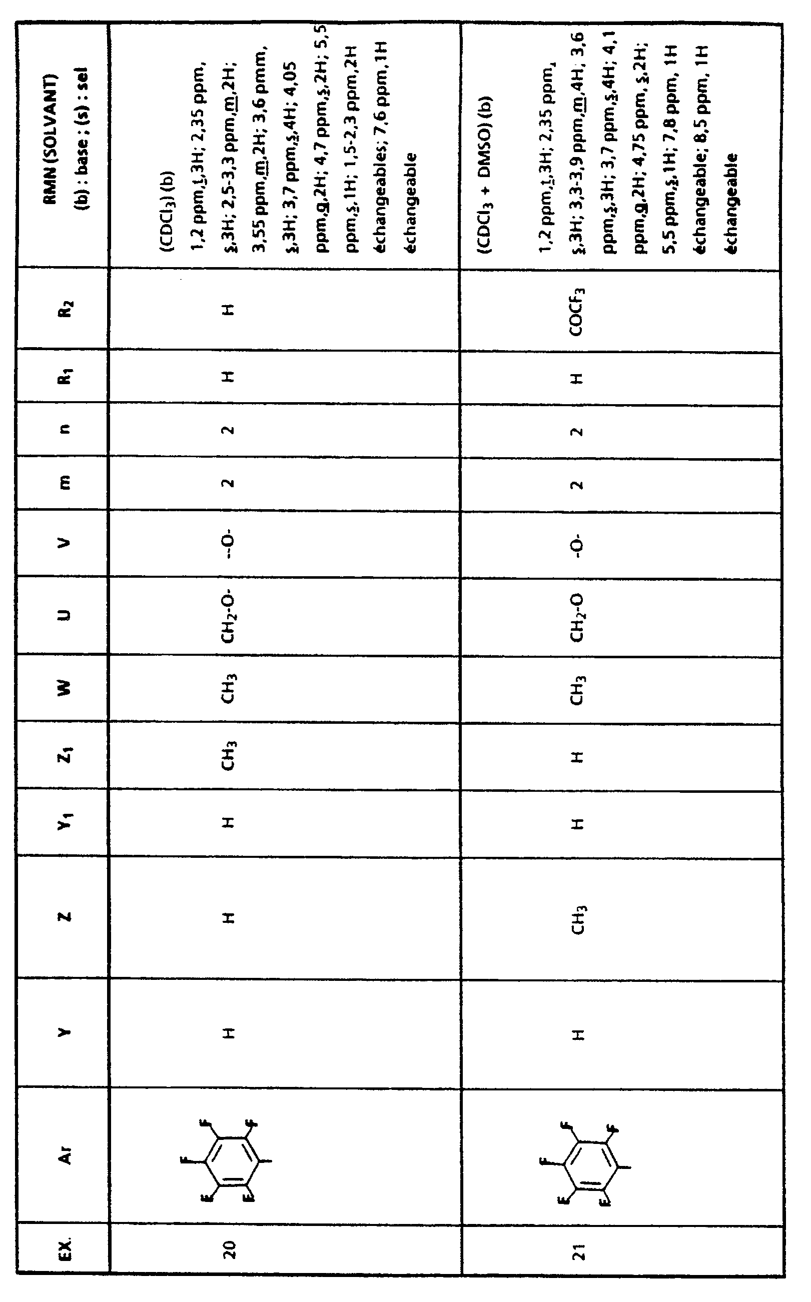

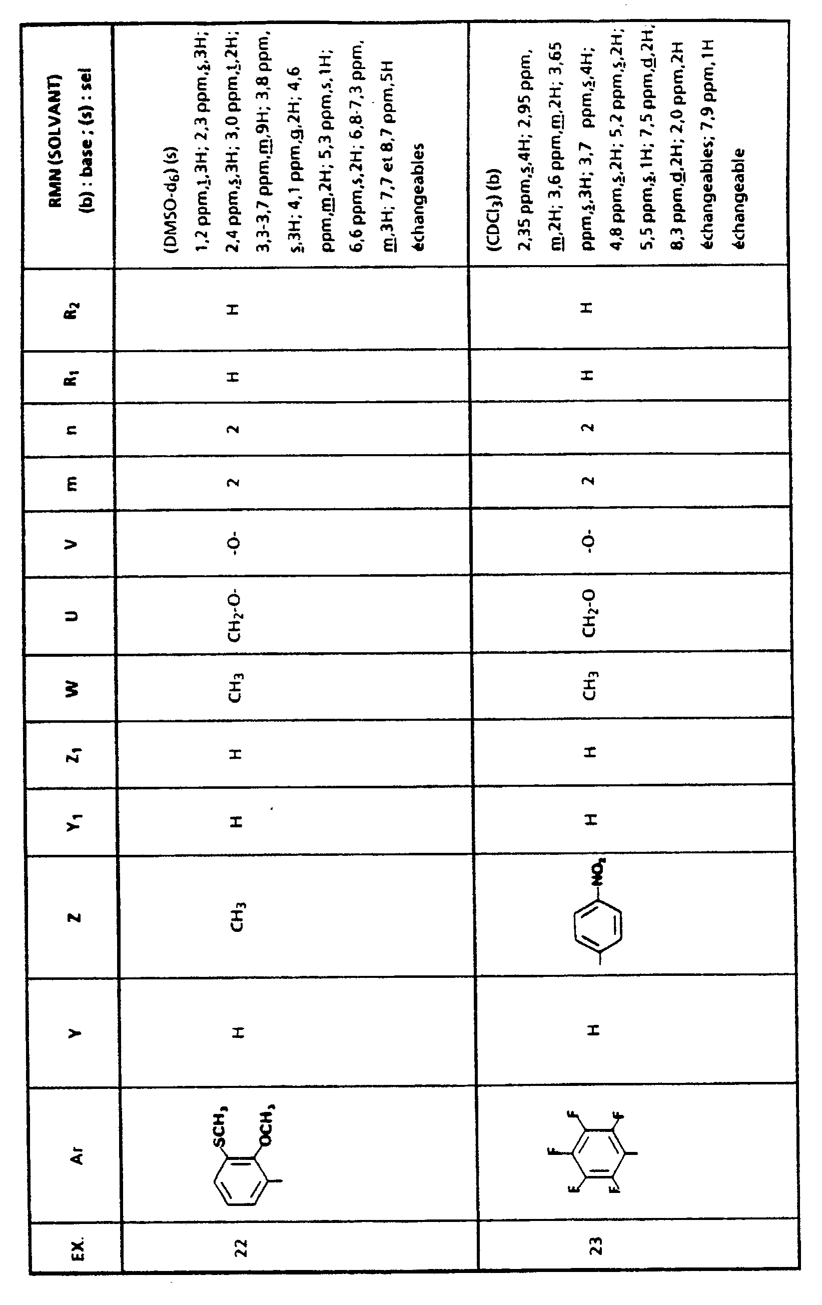

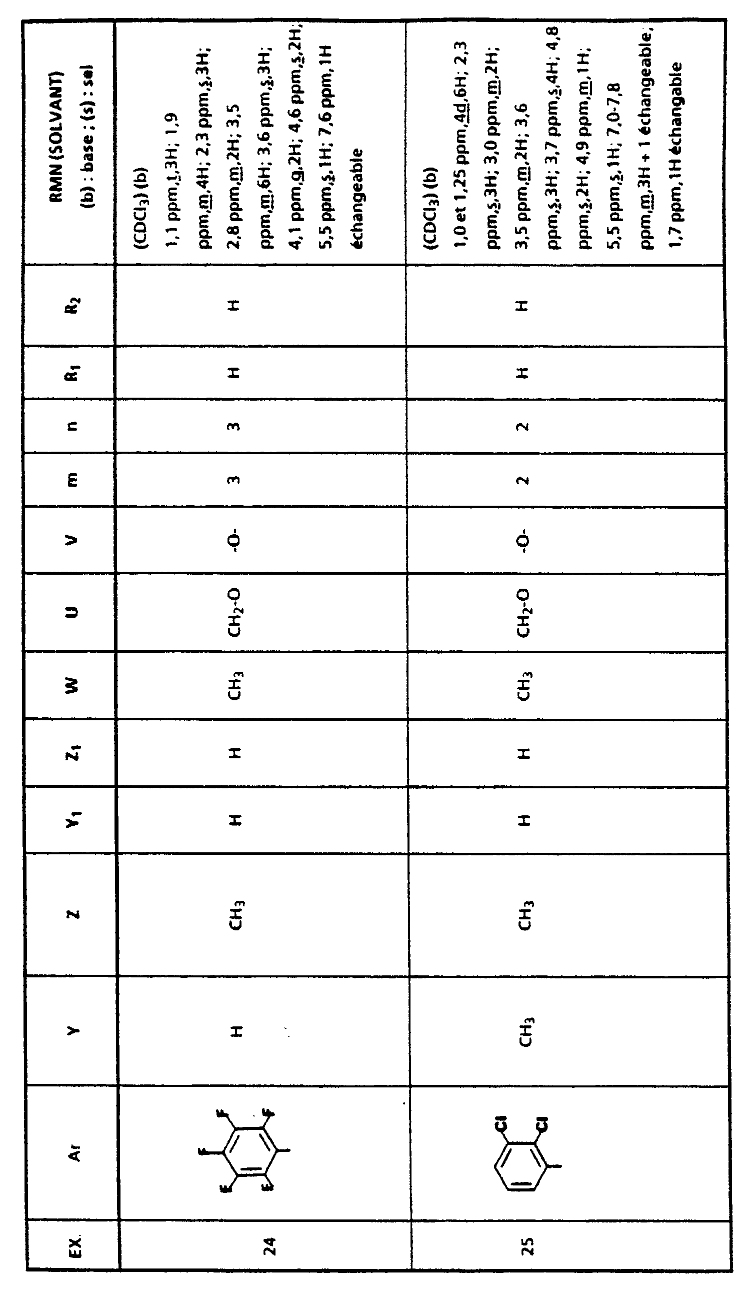

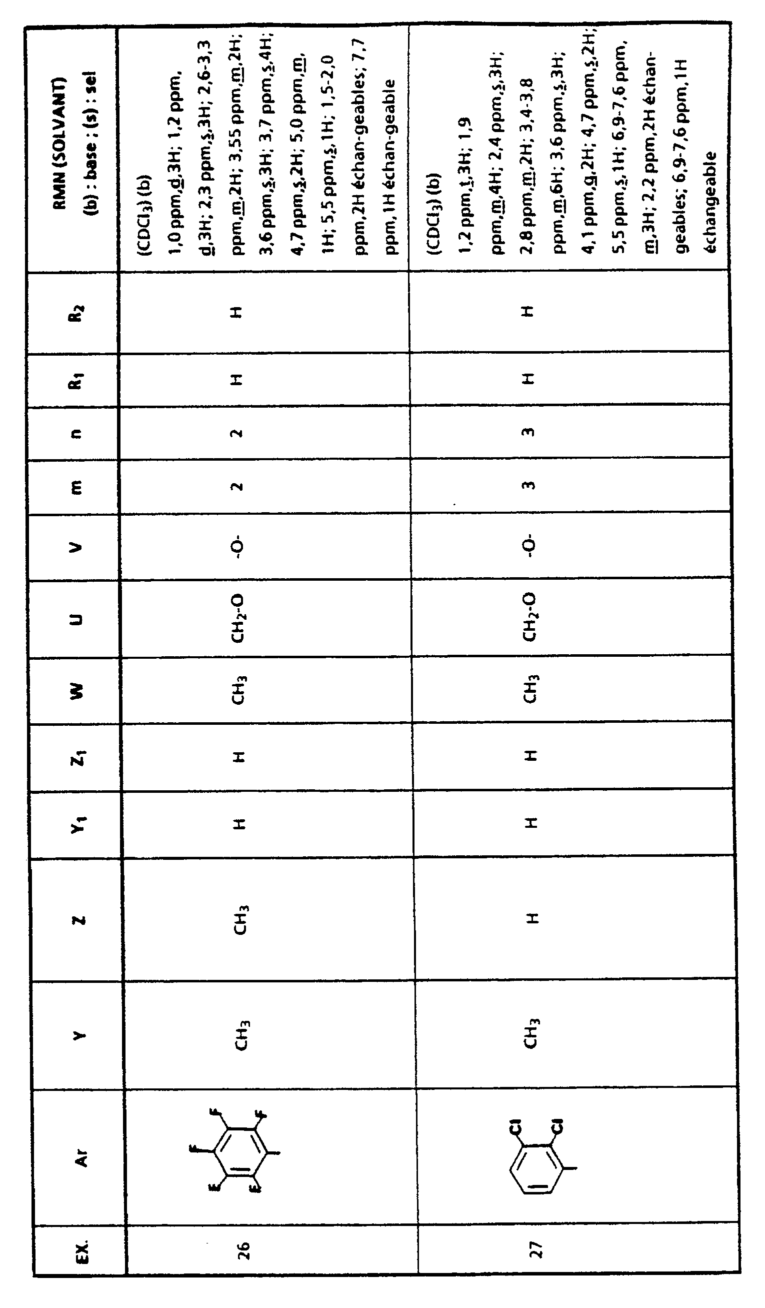

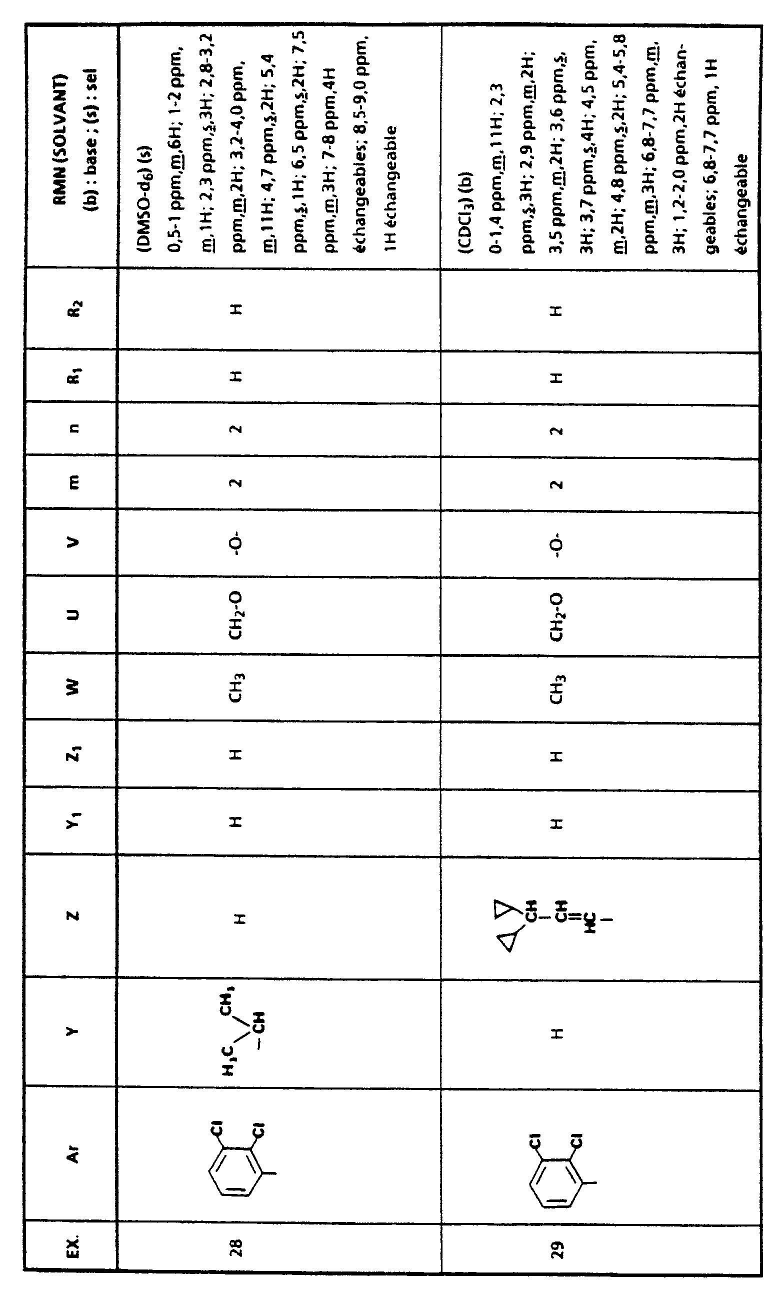

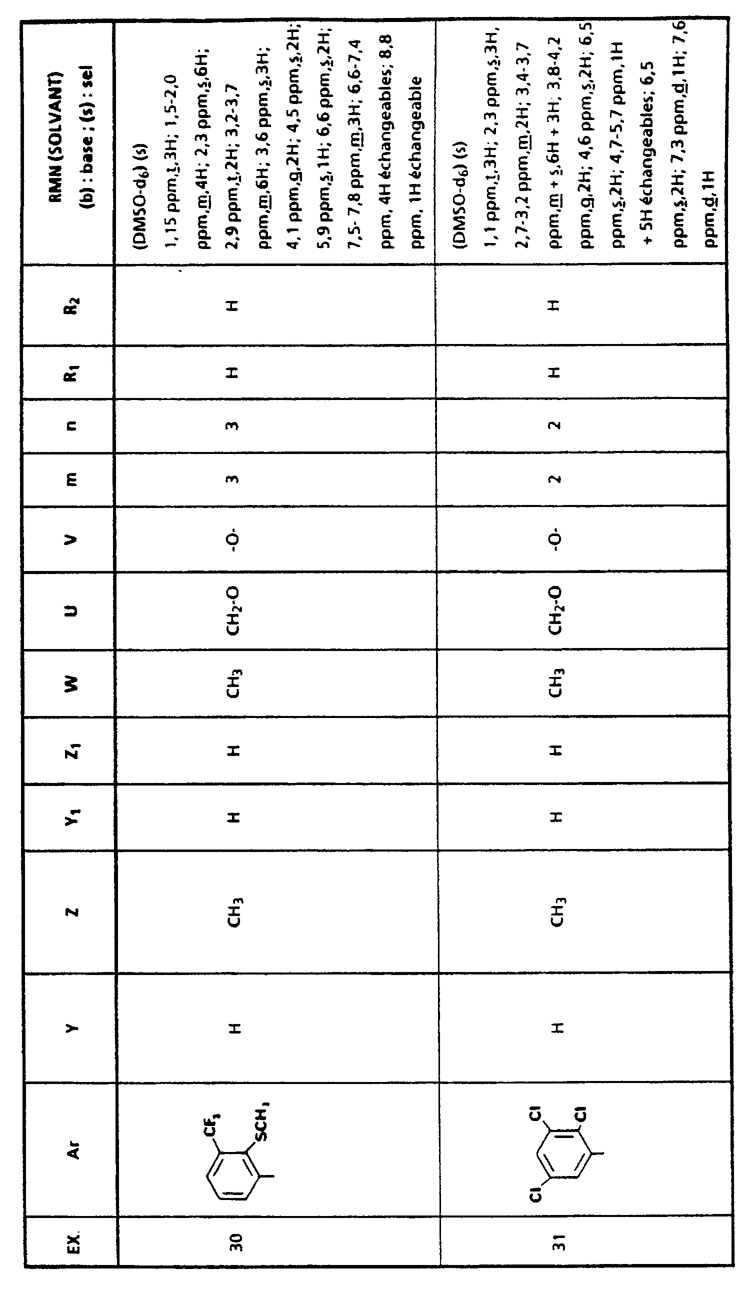

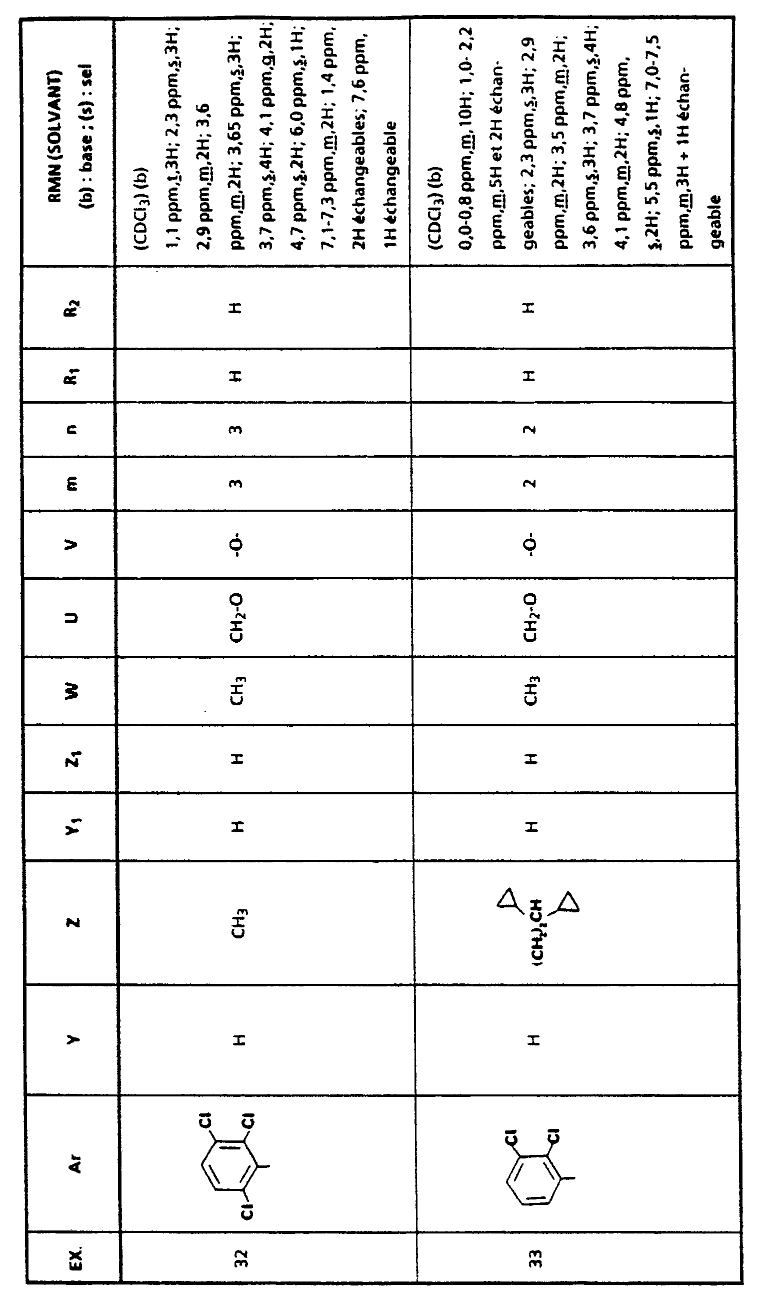

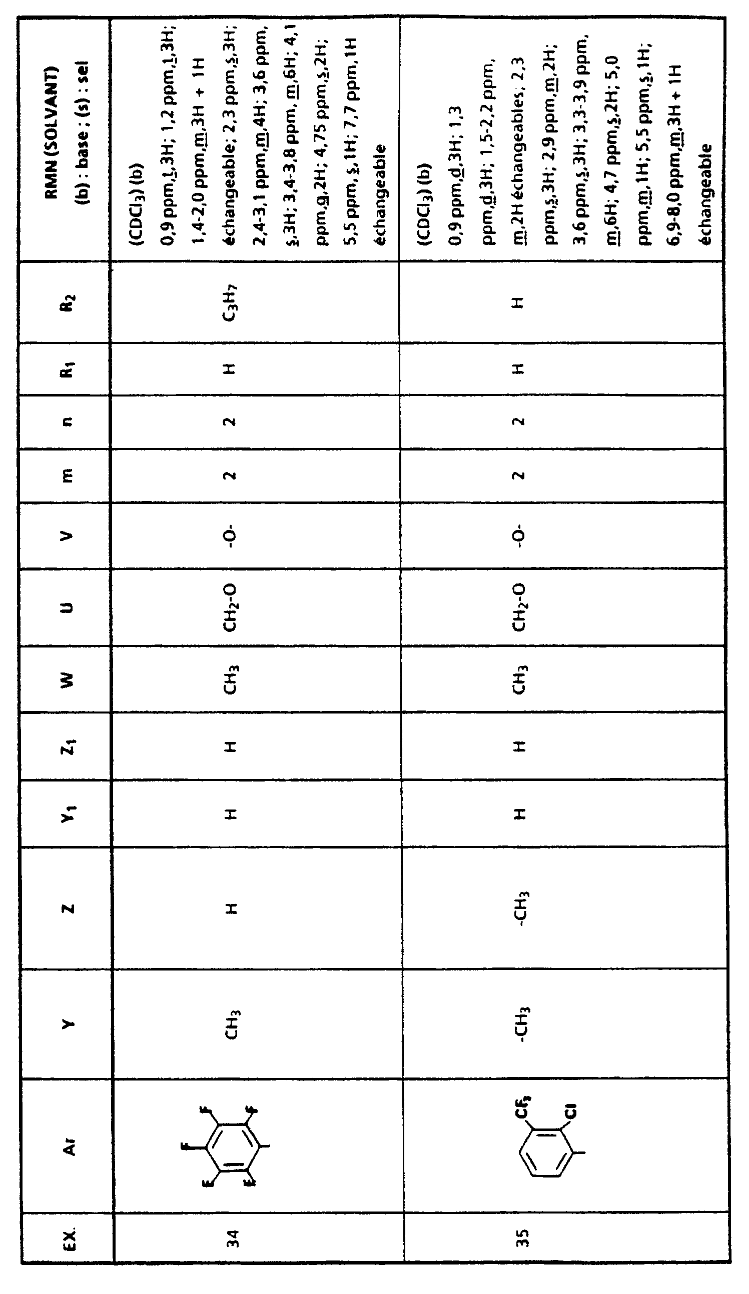

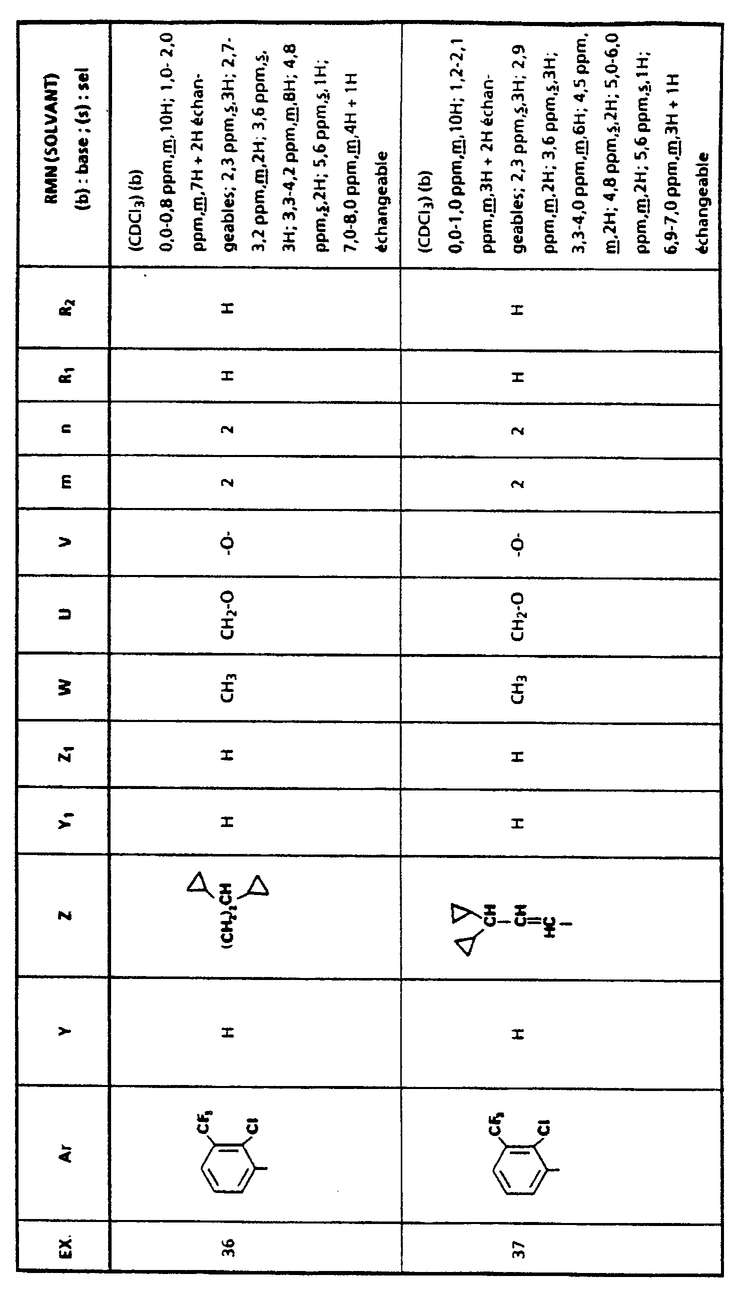

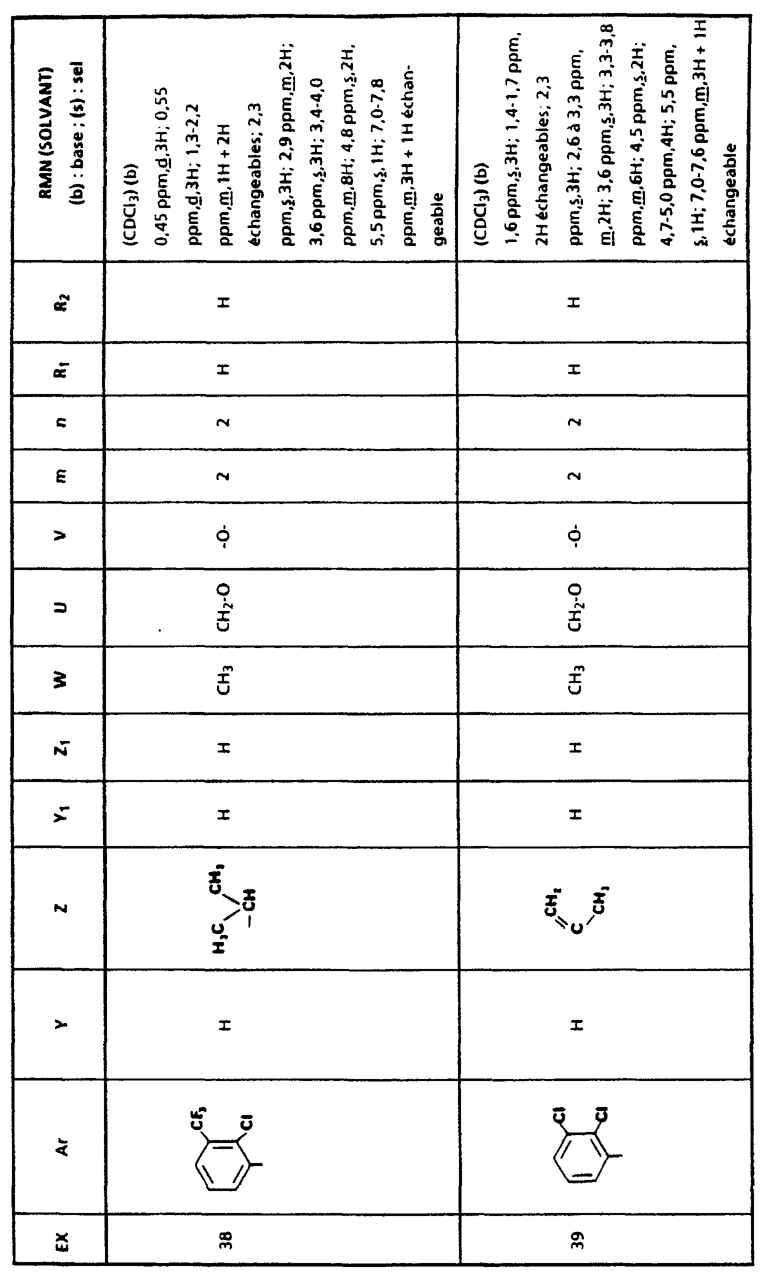

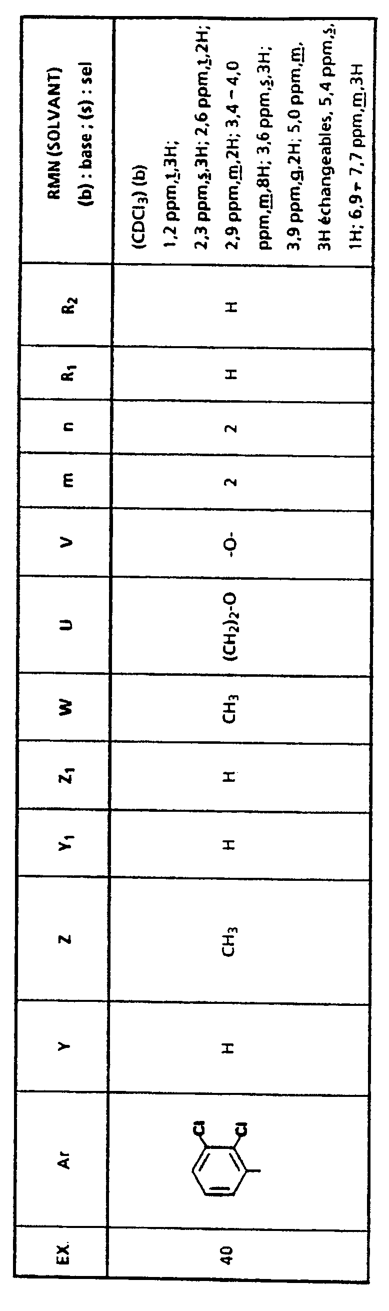

### ETUDE PHARMACOLOGIQUE

### EXEMPLE 41 :

### Evaluation de l'activité in vitro sur l'aorte de rat stimulée par les ions potassium.

Cet essai est effectué sur des organes isolés prélevés chez le rat Wistar mâle de 300 à 400 g placé sous diète hydrique 18 heures avant le sacrifice.

Après sacrifice rapide de l'animal, l'aorte (niveau crosse aortique) est prélevée et disséquée en anneau de 2 mm de longueur; l'endothélium est éliminé de façon mécanique. Après une période d'équilibrage de 1 heure dans la solution physiologique constituée de mM : NaCI 112, KCI 5, KH₂PO₄. 1, MgSO₄ 1,2, CaCl₂ 2,5, NaHCO₃ 25, glucose 11,5, les préparations sont stimulées par une solution riche en potassium. Cette dernière est constituée de mM : NaCI 37, KCI 80, KH₂PO₄ 1, MgS0₄ 1,2, CaCl₂ 2,5, NaHCO₃ 25, glucose 11,5. Cette solution à 37° C a un pH égal à 7,4.

Après 15 minutes de stabilisation, les substances à tester sont alors ajoutées (prise d'essai 0,2 ml) à doses cumulées toutes les 60 minutes. Les valeurs de relaxation obtenues permettent de construire une courbe action-dose conduisant au calcul d'un ICso (exprimé en M).

Les résultats de cette étude sont indiqués dans le tableau Il ci-après.

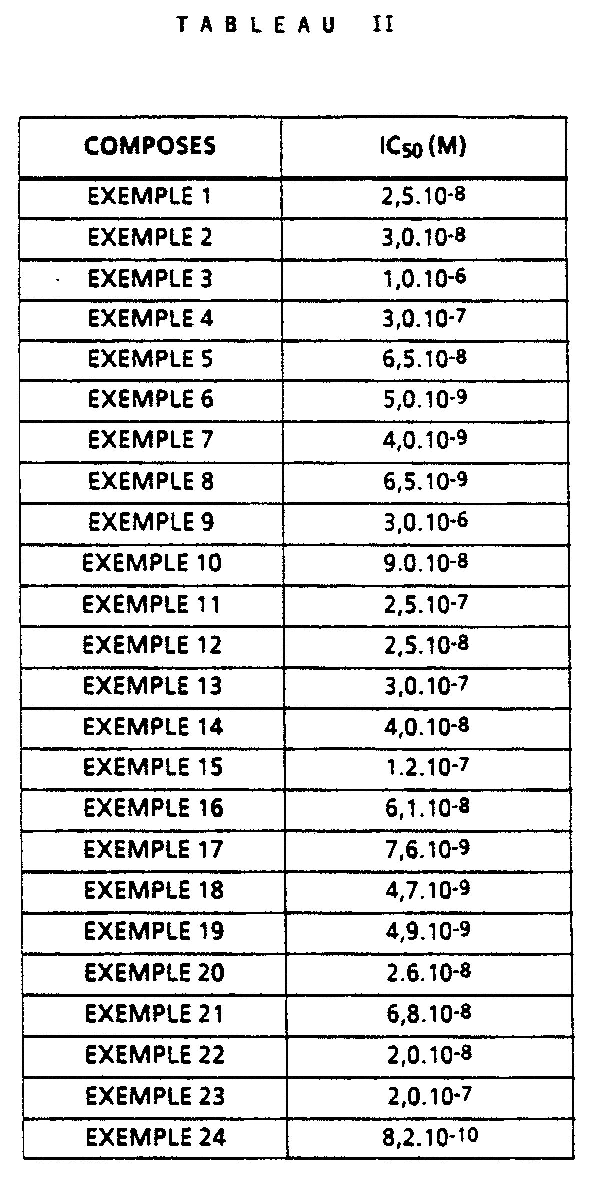

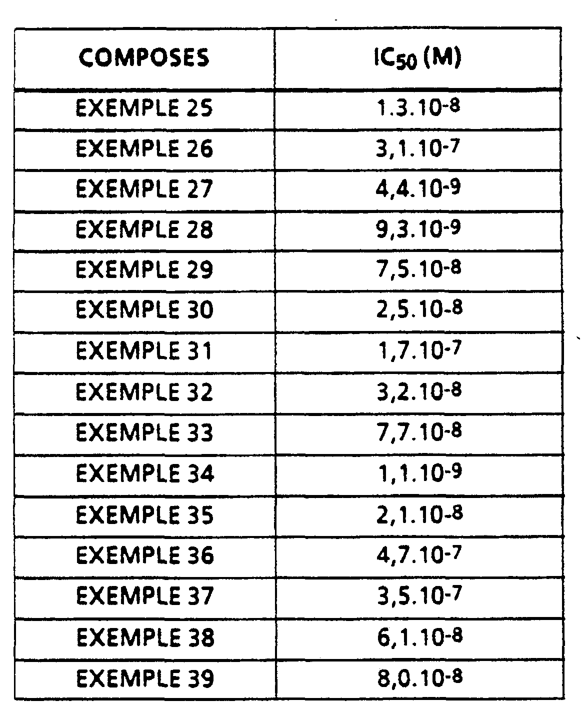

### EXEMPLE 42 :

### Etude chez le chien éveillé hypertendu rénal

Des chiens batards de 20 à 25 kg sont utilisés. Un cathéter en silastic ® mis en place sous anesthésie au niveau de l'aorte abdominale, laissé en chronique permet la mesure ultérieure de la pression artérielle chez l'animal vigile.

L'hypertension artérielle est induite par une seconde intervention sous anesthésie consistant en une constriction de l'artère rénale gauche, à l'aide d'un clip, réduisant son débit d'environ 70 %, le rein gauche est enveloppé aussi d'une capsule en latex pour limiter les compensations circulatoires; le rein controlatéral est laissé en place.

Les pressions artérielles systolique, diastolique et moyenne sont mesurées par l'intermédiaire d'un capteur de pression Statham @ P₂₃ connecté au cathéter silastic ® et relié à un "pressure-processor" Gould @. Les produits sont testés chez les animaux devenus hypertendus à l'état éveillé au moins une semaine après la seconde intervention. La pression artérielle est enregistrée en continu jusqu'à 7 heures après le traitement puis à 24 heures.

Les produits à tester sont administrés par la voie digestive après tubage gastrique, sous forme d'une solution aqueuse, hydroalcoolique etc... selon la solubilité des produits.

Les doses administrées sont exprimées en mg de base rapportée au poids (kg).

Les produits de l'invention ont été comparés à un composé de référence dérivé de la dihydro-1,4 pyridine, l'amlodipine. Les résultats de cette étude sont indiqués dans le tableau III.

Comme on peut observer sur ces deux tableaux les composés de la présente invention ont une activité comparable à l'amlodipine mais la durée de leur action est très supérieure.

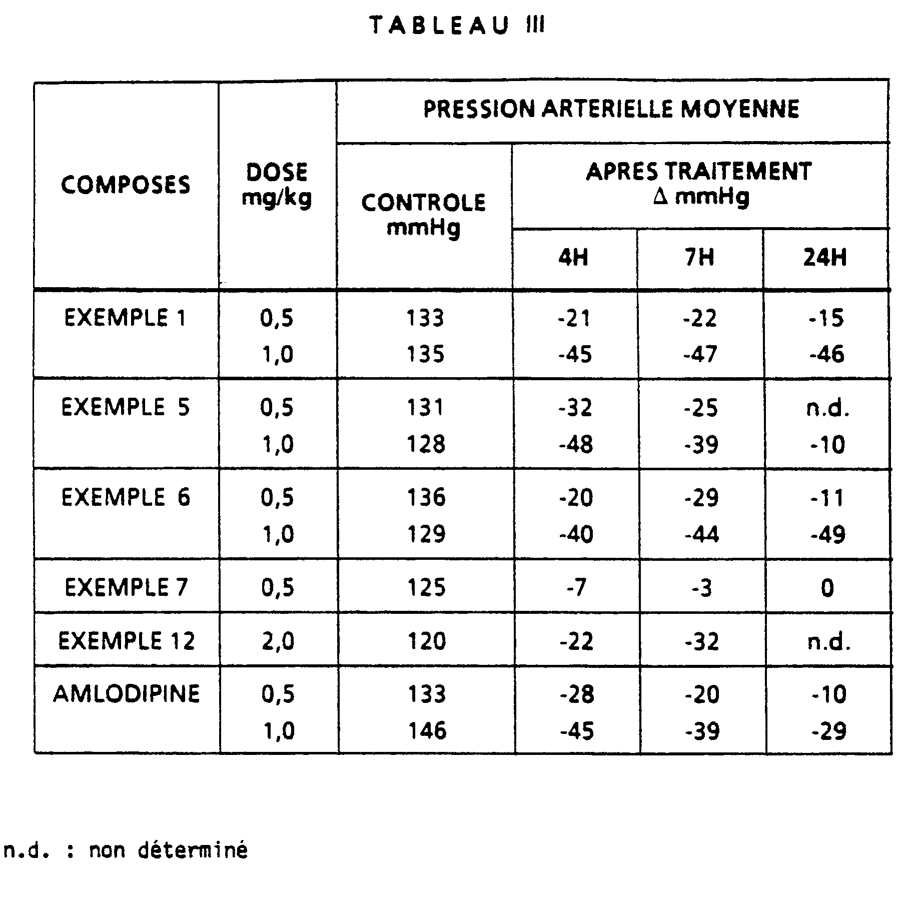

### EXEMPLE 43 :

### Etudes aigües chez le rat spontanément hypertendu (SHR) éveillé

Des rats mâles SHR de 270 à 320 g âgés de 16 à 24 semaines sont anesthésiés à l'éther. Un cathéter en polyéthylène est introduit dans l'artère fémorale et tunellisé au niveau de la queue. La pression artérielle fémorale est enregistrée à l'aide d'un capteur Statham ® P₂₃ sur un enregistreur Gould o 2400. Les animaux sont traités au moins une heure après intervention. Les produits sont administrés par voie orale sous forme dissoute. Les doses administrées sont exprimées en mg/kg de base. Les composés de l'invention ont été comparés à l'amlodipine et à un autre composé de référence, le dérivé de la dihydro-1,4 pyridine, la nifédipine.

Les résultats de cette étude sont rapportés au tableau IV. Comme dans l'exemple précédent, les composés de la présente invention se distinguent des composés de référence par la puissance et la durée de leur activité antihypertensive.

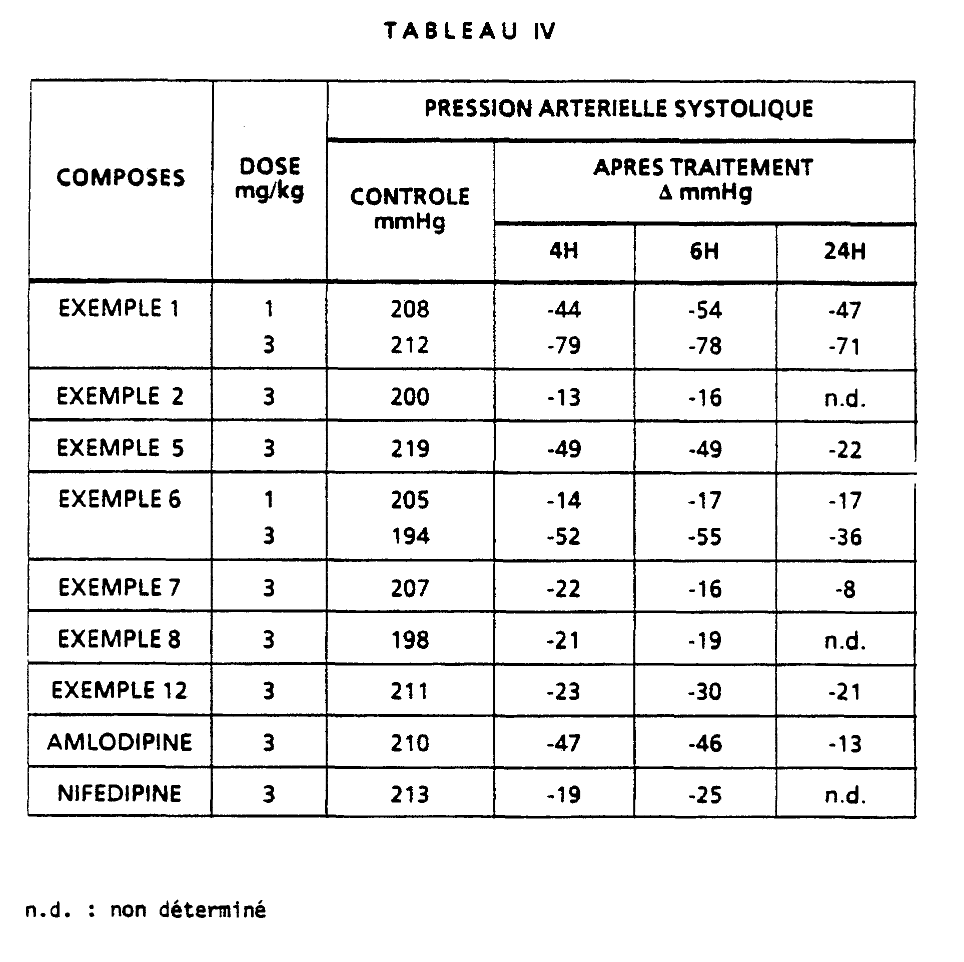

### EXEMPLE 44 :

### Etudes chroniques chez le rat spontanément hypertendu (SHR) éveillé

Lors de cette étude de rats mâles de 280 à 300 g âgés de 16 semaines sont utilisés. La pression artérielle systolique est mesurée grâce à un appareil Rhema multicanaux 8.000 @ selon la méthode indirecte à la queue de L'animal. Les produits à tester sont administrés en une prise unique quotidienne par voie orale.

Les mesures de pression artérielle sont effectuées chaque jour juste avant traitement. L'amlodipine a été aussi étudiée sous les mêmes conditions. Les résultats de cette étude sont rapportés au tableau V ci-après. Les résultats montrent que les produits de la présente invention à dose égale, ont une efficacité supérieure à l'amlodipine.

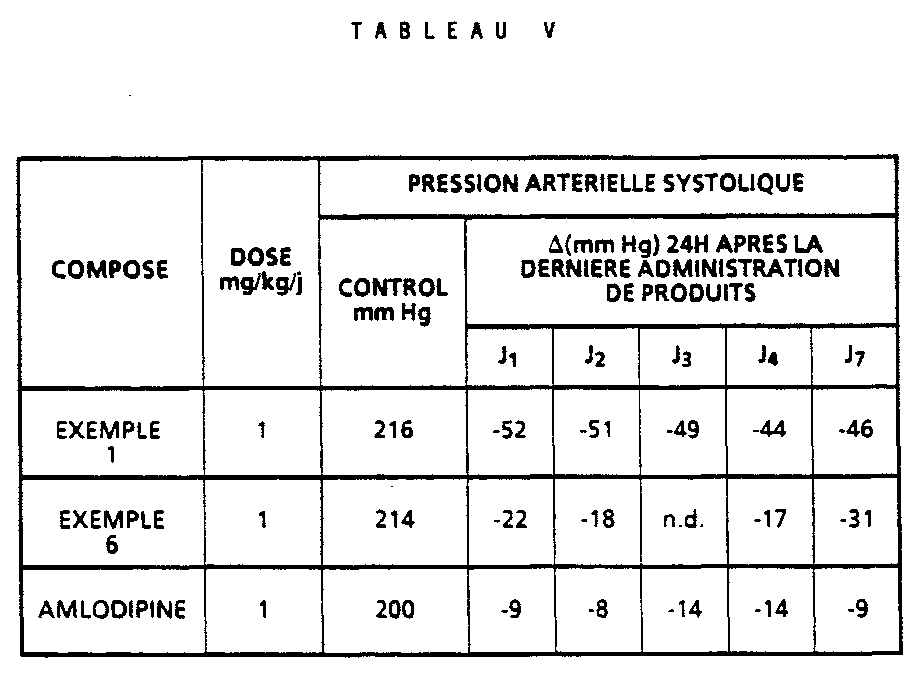

### PREPARATION PHARMACEUTIQUE

### EXEMPLE 45:

Gélules dosées à 2 mg d'hémifumarate de (4R,S) {[amino-2 éthoxy)-2 éthoxy] méthyl}-2 éthoxycarbonyl-3 méthoxycarbonyl-5 méthyl-6 pentafluorophényl-4 dihydro-1,4 pyridine (A.E.P.M.D.P.)

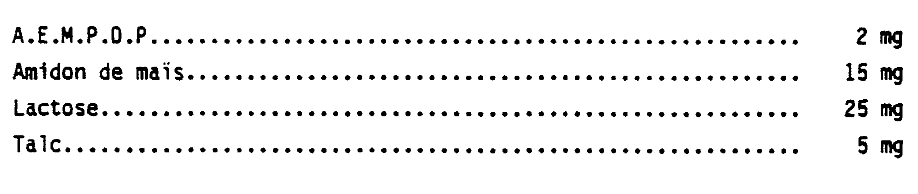

## Revendications

1. Composés de formule générale I : 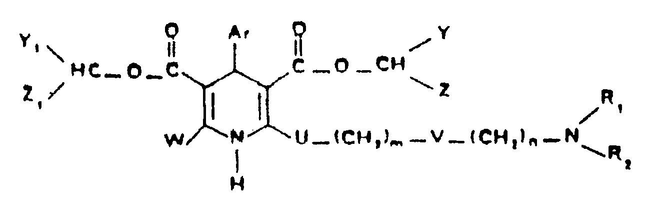 dans laquelle :
- Ar représente un radical phényle, comportant éventuellement un à cinq substituants identiques ou différents représentant chacun un atome d'halogène, un radical alkoxy renfermant de 1 à 4 atomes de carbone, un radical alkylthio renfermant de 1 à 4 atomes de carbone, un radical trihalogénométhyle, ou un radical méthylènedioxy,
- Y, Z, Y₁ et Z₁ identique ou différents représentent chacun un atome d'hydrogène, un radical alkyle inférieur linéaire ou ramifié renfermant de 1 à 4 atomes de carbone, un radical cyclopropyle, un radical dicyclopropylméthyle, un radical dicyclopropyl-2,2 éthyle, un radical dicyclopropyl-2,2 éthylène, un radical dicyclopropyl-3,3 propyle ou un radical dicyclopropyl-3,3 propylène-1,
- W représente un radical alkyle inférieur, linéaire ou ramifié, renfermant de 1 à 4 atomes de carbone ou un radical alcoxyméthyle inférieur renfermant de 2 à 5 atomes de carbone,
- V représente un radical méthylène ou un atome d'oxygène,
- U représente un radical méthylèneoxy ou un radical éthylèneoxy quand V représente un atome d'oxygène, ou un radical méthylène quand V représente aussi un radical méthylène,
- m et n identiques ou différents représentent un nombre entier pouvant prendre les valeurs de 1 à 4,
- Ri, et R₂ identiques ou différents représentent chacun un atome d'hydrogène, un radical alkyle inférieur linéaire ou ramifié renfermant de 1 à 4 atomes de carbone, un radical alcényle inférieur linéaire ou ramifié renfermant de 2 à 4 atomes de carbone, un radical trihalogénoacétyle, à condition toutefois que dans ce cas V ne représente jamais un radical méthylène, un radical phénalkyle de 7 à 10 atomes de carbone éventuellement substitué sur le cycle aromatique par un ou plusieurs radicaux alkyle ou alcoxy renfermant de 1 à 4 atomes de carbone ou par un ou plusieurs radicaux hydroxy, un radical hydroxy-1 phényl-2 éthyle éventuellement substitué sur le cycle aromatique par un ou plusieurs radicaux alkyle ou alcoxy renfermant de 1 à 4 atomes de carbone ou par un ou plusieurs radicaux hydroxy, ou forment ensemble avec l'atome d'azote auquel ils sont attachés un groupement phtalimido,
sous forme racémique ou d'isomères optiques, et leurs sels d'addition à un acide minéral ou organique pharmaceutiquement acceptable,
ainsi que leurs sels d'ammonium quaternaire formés avec un halogénure d'alkyle ou alkylène inférieur renfermant de 1 à 4 atomes de carbone quand ils comportent une amine tertiaire.

2. Composés de formule générale dans laquelle Ar représente un radical phényle polyhalogéné, Y₁ et Z₁ représentent chacun un atome d'hydrogène, W représente un méthyle, V représente un atome d'oxygène, U représente un radical méthylèneoxy, sous forme racémique ou d'isomères optiques et leurs sels d'addition à un acide minéral ou organique pharmaceutiquement acceptable.

3. La (4R,S) {[(amino-2 éthoxy)-2 éthoxy] méthyl}-2 éthoxycarbonyl-3 méthoxycarbonyl-5 méthyl-6 pentafluorophényl-4 dihydro-1,4 pyridine, ses isomères optiques et ses sels d'addition avec un acide minéral ou organique pharmaceutiquement acceptable.

4. La (4R,S) {[(amino-2 éthoxy)-2 éthoxy] méthyll-2 (chloro-2 phényl)-4 éthoxycarbonyl-3 méthoxycarbonyl-5 méthyl-6 dihydro-1,4 pyridine, ses isomères optiques et ses sels d'addition avec un acide minéral ou organique pharmaceutiquement acceptable.

5. La (4R,S) {[(amino-2 éthoxy)-2 éthoxy] méthyl)-2 (dichloro-2,3 phényl)-4 éthoxycarbonyl-3 méthoxycarbonyl-5 méthyl-6 dihydro-1,4 pyridine, ses isomères optiques et ses sels d'addition avec un acide minéral ou organique pharmaceutiquement acceptable.

6. La (4R,S) (chloro-2 phényl)-4 {[N,N diallylamino-2 éthoxy)-2 éthoxy] méthyl)-2 éthoxycarbonyl-3 méthoxycarbonyl-5 méthyl-6 dihydro-1,4 pyridine, ses isomères optiques et ses sels d'addition avec un acide minéral ou organique pharmaceutiquement acceptable.

7. La (4R,S) (amino-7 heptyl)-2 éthoxycarbonyl-3 (chloro-2 phényl)-4 méthoxycarbonyl-5 méthyl-6 dihydro-1,4 pyridine, ses isomères optiques et ses sels d'addition avec un acide minéral ou organique pharmaceutiquement acceptable.

8. La (4R,S) (amino-5 pentyl)-2 éthoxycarbonyl-3 (chloro-2 phényl)-4 méthoxycarbonyl-5 méthyl-6 dihydro-1,4 pyridine, ses isomères optiques et ses sels d'addition avec un acide minéral ou organique pharmaceutiquement acceptable.

9. La (4R,S) {[(amino-2 éthoxy)-2 éthoxyl-2 éthyl}-2 (dichloro-2,3 phényl)-4 éthoxycarbonyl-3 méthoxycarbonyl-5 méthyl-6 dihydro-1,4 pyridine.

10. Procédé de préparation de composés de formule générale I, caractérisé en ce que :
- soit
l'on condense un composé de formule générale Il : 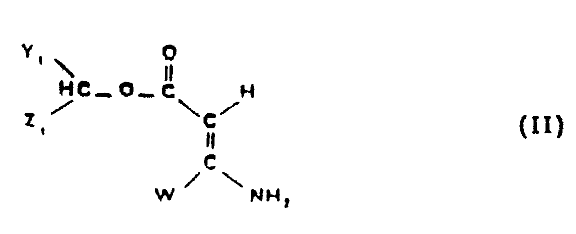 dans laquelle la définition des substituants Y₁, Z₁ et W demeure celle définie précédemment pour la formule générale I,
avec un cetoester de formule générale III, 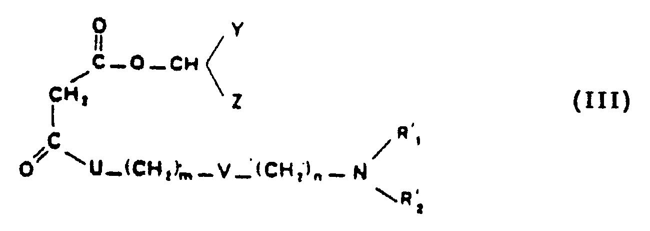 dans laquelle Y, Z, U, V, m et n ont la signification précédemment définie dans la formule I, et R'₁ et R'₂ représentent un radical méthyle et un radical benzyle, ou un atome d'hydrogène et un radical trihalogénoacétyle à condition toutefois que dans ce cas V ne représente jamais simultanément un radical méthylène, ou forment ensemble avec l'atome d'azote auquel ils sont attachés un radical phtalimido,
et avec un aldéhyde aromatique de formule générale IV : 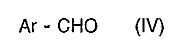 dans laquelle la définition de Ar demeure celle définie précédemment pour la formule générale I, dans un solvant organique polaire tel qu'un alcool primaire ou secondaire ou un acide organique, de petit poids moléculaire, et à une température comprise entre 40° C et 100° C, pour obtenir un composé de formule générale l', 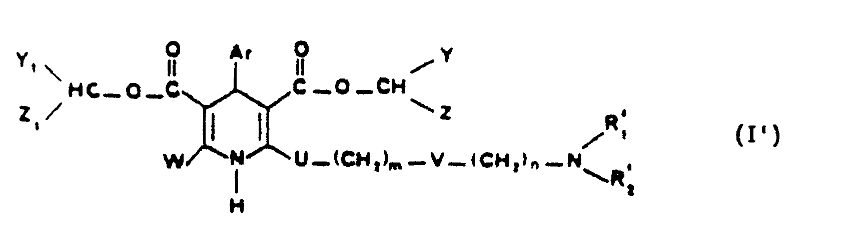 dans laquelle la définition de Ar, Y, Z, Y₁, Zᵢ, W, U, V, m et n demeure celle indiquée précédemment et la définition de R'₁ et R'₂ reste identique à celle donnée pour R'₁ et R'₂ de la formule générale III, soit
l'on condense un cetoester de formule générale V : 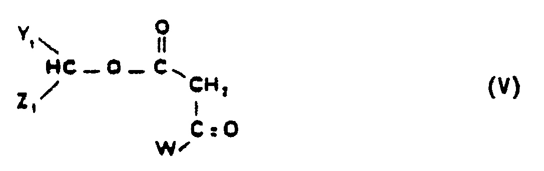 dans laquelle la définition de Y₁, Z₁ et W reste identique à celle donnée pour la formule I,
avec un composé de formule générale VI : 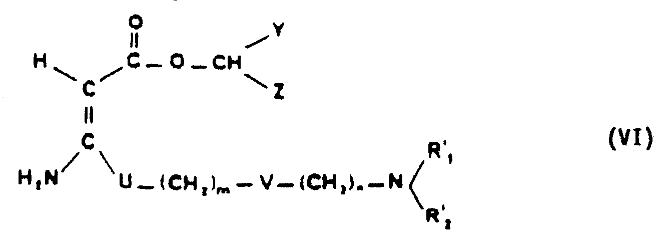 dans laquelle Y, Z, U, V, m et n ont la signification précédemment définie pour la formule I, et la définition de R'₁ et R'₂ reste identique à celle donnée pour la formule générale III,
et avec un aldéhyde aromatique de formule générale IV,
dans un solvant organique polaire tel qu'un alcool primaire ou secondaire ou un acide organique, de petit poids moléculaire, et à une température comprise entre 40° et 100 C,
pour obtenir un composé de formule générale l',
- soit
l'on condense un benzylidène de formule générale VII, 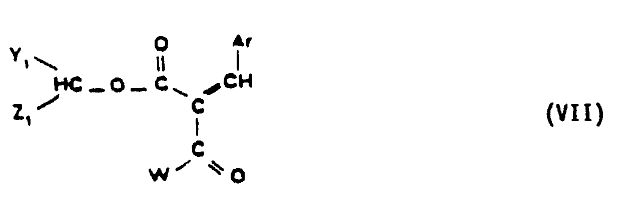 dans laquelle la définition de Y₁, Zᵢ, W et Ar reste identique à celle donnée pour la formule I,
avec un composé de formule générale VI, dans un solvant organique polaire tel qu'un alcool primaire ou secondaire ou un acide organique, de petit poids moléculaire et à une température comprise entre 40⁰ et 100° C,
pour obtenir les composés de formule générale l',
- et ensuite.
si on le désire, on soumet les composés de formule générale l'
dans laquelle la signification de Ar, Y, Z, Yi, Z₁, W, U, V, m et n demeure celle indiquée précédemment et R₁ et R₂ représentent un radical hydrogène et un radical trihalogénoacétyle, ou forment ensemble avec l'atome d'azote auquel ils sont attachés un radical phtalimido,
à l'action de l'hydrazine ou d'un sel minéral basique tel que le carbonate de potassium, en présence d'eau, dans un solvant alcoolique polaire de petit poids moléculaire miscible à l'eau, et à une température comprise entre 40⁰ et 100° C
pour obtenir les composés de la formule générale I,
dans laquelle Ar, Y, Z, Y₁, Z₁, W, U, V, m et n ont la signification précédemment définie et R₁ et R₂ représentent un atome d'hydrogène,
- et ensuite.
si on le désire
soit
l'on soumet à l'action d'un aryléthylèneoxyde de formule générale VIII : 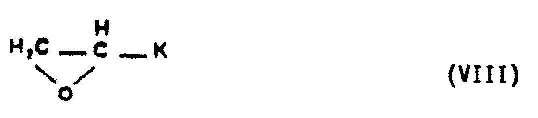 dans laquelle K représente un radical phényle, éventuellement substitué par un ou plusieurs radicaux alkyle ou alcoxy renfermant de 1 à 4 atomes de carbone ou un ou plusieurs radicaux hydroxy,
pour obtenir un composé de formule générale I"
dans laquelle la signification de Ar, Y, Z, Y₁, Zi, W, U, V, m et n demeure identique à celle indiquée précédemment, et la signification de K reste identique à celle donnée pour la formule générale VIII, 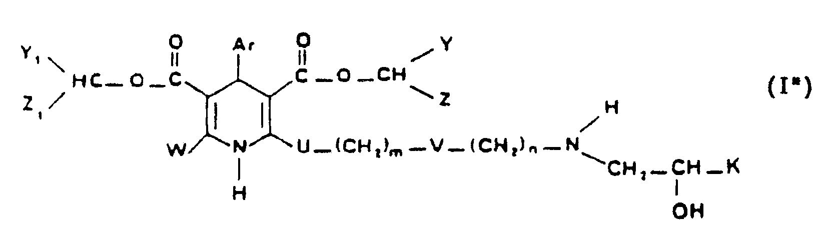
soit
l'on soumet à l'action d'un agent alcoylant de formule générale IX : 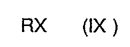 dans laquelle X représente un halogène et R représente un radical alkyle ou alkylène inférieur linéaire ou ramifié renfermant de 1 à 4 atomes de carbone,
dans un solvant organique polaire tel que l'acétonitrile en présence d'un sel minéral basique tel que le carbonate de potassium à une température comprise entre 40 et 100° C,
pour obtenir les composés de la formule générale I dans laquelle Ar, Y, Z, Y₁, Z₁, W, U, V, m et n ont la même signification que celle précédemment définie et R₁ et R₂ sont identiques et ont la même signification que R de la formule générale IX,
ou bien
soit d'abord à l'action du benzaldéhyde en présence d'un solvant aromatique inerte et apolaire, tel que le benzène et à une température entre 50-120° C, et ensuite, après élimination du solvant utilisé à l'action de borohydrure de sodium, en présence d'un alcool aliphatique polaire de petit poids moléculaire, pour obtenir un composé de formule générale 1
dans laquelle la signification de Ar, Y, Z, Y₁, Zi, W, U, Y, m et n demeure identique à celle indiquée précédemment, R₁ représente un atome d'hydrogène et R₂ un radical benzyle,
lequel ensuite,
si on le désire est soumis à l'action d'un agent alcoylant de formule générale IX,
pour obtenir un composé de la formule générale 1
dans laquelle Ar, Y, Z, Y₁, Z₁, W, U, Y, m et n ont la signification précédemment définie, la définition de R₁ est identique à celle de R de la formule générale IX et R₂ représente un radical benzyle, sous forme de sels d'ammonium quaternaire ou d'amines tertiaires,
lequel ensuite,
si on le désire est soumis à l'action du triéthylborohydrure de lithium ou à une hydrogénation catalytique pour obtenir respectivement par les sels d'ammonium quaternaire ou les amines tertiaires, les composés de la formule générale I dans laquelle Ar, Y, Z, Y₁, Zᵢ, W, U, Y, m et n ont la même signification que celle définie précédemment, R₁ ayant la même signification que R de la formule générale IX et R₂ représente respectivement un radical benzyle ou un atome d'halogène,
puis,
si on le désire, on le soumet à l'action d'un agent alkoylant de formule générale IX,
pour obtenir les composés de formule générale 1
dans laquelle R₁ et R₂ différents représentent chacun un radical alkyle inférieur, linéaire ou ramifié, renfermant de 1 à 4 atomes de carbone ou un radical alkylène linéaire ou ramifié renfermant de 1 à 4
atomes de carbone,
et ensuite,
si on le désire, on transforme les composés de la formule générale 1 en un sel d'addition avec un acide organique ou minéral pharmaceutiquement acceptable,
ou dans le cas où ils portent sur leur chaîne latérale en position 2 une amine tertiaire, en un sel d'ammonium quaternaire avec un alkyle ou alkylène halogéné de formule générale IX.

11. Composition pharmaceutique contenant comme principe actif un composé selon l'une quelconque des revendications 1 à 9, en association ou en mélange avec un excipient ou un véhicule inerte non toxique pharmaceutiquement acceptable.

12. Composition pharmaceutique selon la revendication 11 renfermant le principe actif à la dose de 0,05 à 50 mg.

13. Composition pharmaceutique selon les revendications 11 et 12 renfermant comme principe actif au moins un composé selon les revendications 1 à 9 utilisable dans le traitement des malades nécessitant des modérateurs de calcium.

## Claims

1. Compounds of the general formula I: 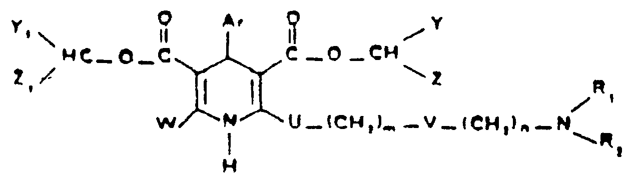 in which:
- Ar represents a phenyl radical optionally containing from one to five identical or different substituents each representing a halogen atom, an alkoxy radical containing from 1 to 4 carbon atoms, an alkylthio radical containing from 1 to 4 carbon atoms, a trihalomethyl radical or a methylenedioxy radical,
- each of Y, Z, Y₁ and Zi, which are identical or different, represents a hydrogen atom, a linear or branched lower alkyl radical containing from 1 to 4 carbon atoms, a cyclopropyl radical, a dicyclopropylmethyl radical, a 2,2-dicyclopropylethyl radical, a 2,2-dicyclopropylethenyl radical, a 3,3-dicyclopropylpropyl radical or a 3,3-dicyclopropylprop-1-enyl radical,
- W represents a linear or branched lower alkyl radical containing from 1 to 4 carbon atoms, or a lower alkoxymethyl radical containing from 2 to 5 carbon atoms,
- V represents a methylene radical or an oxygen atom,
- U represents a methyleneoxy radical or an ethyleneoxy radical when V represents an oxygen atom, or a methylene radical when V also represents a methylene radical,
- m and n, which are identical or different, represent an integer that may have a value of from 1 to 4,
- each of R₁ and R₂, which are identical or different, represents a hydrogen atom, a linear or branched lower alkyl radical containing from 1 to 4 carbon atoms, a linear or branched lower alkenyl radical containing from 2 to 4 carbon atoms, a trihaloacetyl radical, provided that, in that case, V does not represent a methylene radical, a phenalkyl radical that has from 7 to 10 carbon atoms and that is optionally substituted on the aromatic ring by one or more alkyl or alkoxy radicals containing from 1 to 4 carbon atoms or by one or more hydroxy radicals, or represents a 1-hydroxy-2-phenylethyl radical optionally substituted on the aromatic ring by one or more alkyl or alkoxy radicals containing from 1 to 4 carbon atoms or by one or more hydroxy radicals, or R₁ and R₂, together with the nitrogen atom to which they are bonded, form a phthalimido group,
in racemic form or in the form of optical isomers, and their addition salts with a pharmaceutically acceptable mineral or organic acid, and also, when they carry a tertiary amine, their quaternary ammonium salts formed with a lower alkyl or lower alkenyl halide containing from 1 to 4 carbon atoms.

2. Compounds of the general formula I in which Ar represents a polyhalogenated phenyl radical, each of Y₁ and Z₁ represents a hydrogen atom, W represents a methyl group, V represents an oxygen atom, and U represents a methyleneoxy radical, in racemic form or in the form of optical isomers, and their addition salts with a pharmaceutically acceptable mineral or organic acid.

3. (4R,S)-2-{[2-(2-aminoethoxy)ethoxy]methyl}-3-ethoxy-carbonyl-5-methoxycarbonyl-6-methyl-4-pentafluorophenyl-1,4-dihydropyridine, its optical isomers and its addition salts with a pharmaceutically acceptable mineral or organic acid.

4. (4R,S)-2-{[2-(2-aminoethoxy)ethoxy]methyl}-4-(2-chlorophenyl)-3-ethoxycarbonyl-5-methoxycarbonyl-6- methyi-1,4-dihydropyridine, its optical isomers and its addition salts with a pharmaceutically acceptable mineral or organic acid.

5. (4R,S)-2-{[2-(2-aminoethoxy)ethoxy]methyl}-4-(2,3-dichlorophenyl)-3-ethoxycarbonyl-5-methoxycarbonyl-6-methyl-1,4-dihydropyridine, its optical isomers and its addition salts with a pharmaceutically acceptable mineral or organic acid.

6. (4R,S)-4-(2-chlorophenyl)-2-{[2-(2-(N,N-diallylamino)-ethoxy)ethoxy]methyl}-3-ethoxycarbonyl-5-methoxycarbonyl-6-methyl-1,4-dihydropyridine, its optical isomers and its addition salts with a pharmaceutically acceptable mineral or organic acid.

7. (4R,S)-2-(7-aminoheptyl)-3-ethoxycarbonyl-4-(2-chlorophenyl)-5-methoxycarbonyl-6-methyl-1,4-dihydropyridine, its optical isomers and its addition salts with a pharmaceutically acceptable mineral or organic acid.

8. (4R,S)-2-(5-aminopentyl)-3-ethoxycarbonyl-4-(2-chlorophenyl) -5-methoxycarbonyl-6-methyl-1,4-dihydropyridine, its optical isomers and its addition salts with a pharmaceutically acceptable mineral or organic acid.

9. (4R,S)-2-{2-[2-(2-aminoethoxy)ethoxy]ethyl}-4-(2,3-dichlorophenyl)-3-ethoxycarbonyl-5-methoxycarbonyl-6-methyl-1,4-dihydropyridine.

10. Process for the preparation of compounds of the general formula I, characterised in that:
- either
a compound of the general formula II: 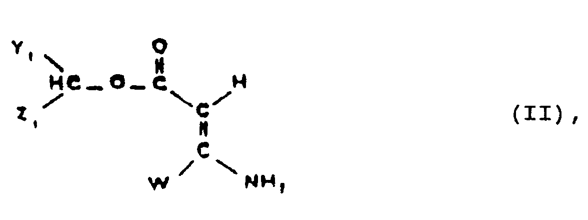 in which the definition of the substituents Yi, Z₁ and W is that defined above for the general formula I,
is condensed with a keto ester of the general formula III 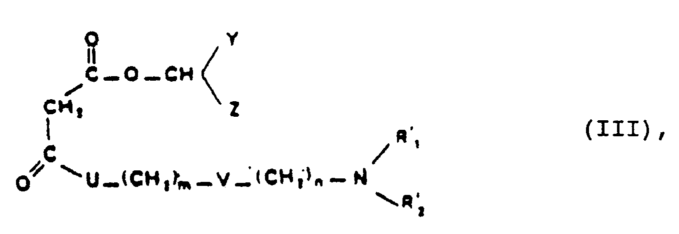 in which Y, Z, U, V, m and n have the meanings defined above in formula I, and R'₁ and R'₂ represent a methyl radical and a benzyl radical, or a hydrogen atom and a trihaloacetyl radical, provided that, in that case, V does not simultaneously represent a methylene radical, or R'₁ and R'₂, together with the nitrogen atom to which they are bonded, form a phthalimido radical,
and with an aromatic aldehyde of the general formula IV:
Ar - CHO (IV),
in which the definition of Ar is that defined above for the general formula I, in a low molecular weight polar organic solvent, such as a primary or secondary alcohol or an organic acid, at a temperature of from 40° C to 100° C, to obtain a compound of the general formula I' 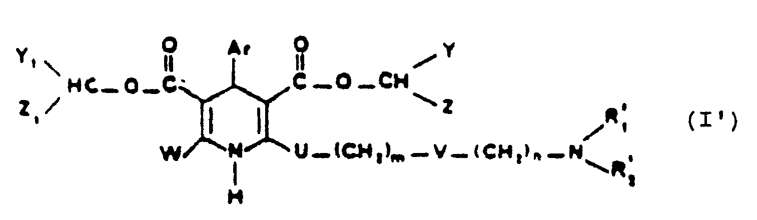 in which the definition of Ar, Y, Z, Yi, Zi, W, U, V, m and n is that given above and the definition of R'₁ and R'₂ is identical to that given for R'₁ and R'₂ in the general formula III,
- or
a keto ester of the general formula V: 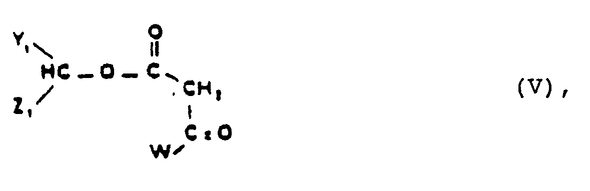 in which the definition of Yi, Z₁ and W is identical to that given for formula I,
is condensed with a compound of the general formula VI: 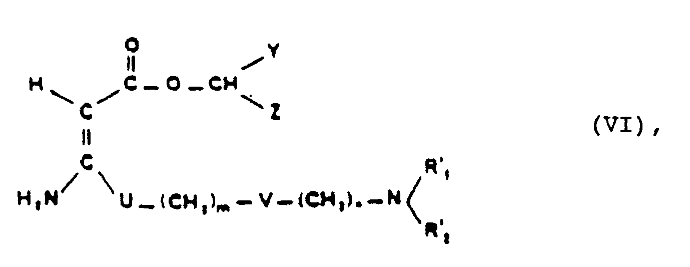 in which Y, Z, U, V, m and n have the meanings defined above for formula I and the definition of R'₁ and R'₂ is identical to that given for the general formula III,
and with an aromatic aldehyde of the general formula IV,
in a low molecular weight polar organic solvent, such as a primary or secondary alcohol or an organic acid, at a temperature of from 40° to 100° C,
to obtain a compound of the general formula I',
- or
a benzylidene of the general formula VII 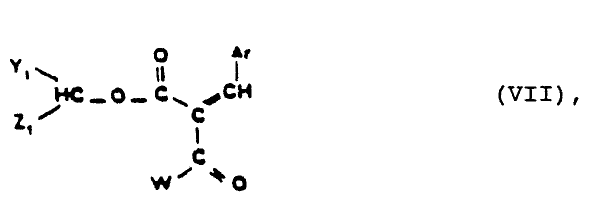 in which the definition of Yi, Zi, W and Ar is identical to that given for formula I,
is condensed with a compound of the general formula VI, in a low molecular weight polar organic solvent, such as a primary or secondary alcohol or an organic acid, at a temperature of from 40° to 100° C,
to obtain the compounds of the general formula I',
- and then,
if desired, the compounds of the general formula I',
in which the meaning of Ar, Y, Z, Y₁, Z₁, W, U, V, m and n is that given above and R₁ and R₂ represent a hydrogen radical and a trihaloacetyl radical or, together with the nitrogen atom to which they are bonded, form a phthalimido radical,
are treated with hydrazine or with a basic mineral salt, such as potassium carbonate, in the presence of water, in a low molecular weight, water-miscible, polar alcoholic solvent, at a temperature of from 40° to 100° C,
to obtain the compounds of the general formula I in which Ar, Y, Z, Yi, Zi, W, U, V, m and n have the meanings defined above and R₁ and R₂ represent a hydrogen atom,
- and then,
if desired,
those compounds of formula I
either
are treated with an arylethylene oxide of the general formula VIII: 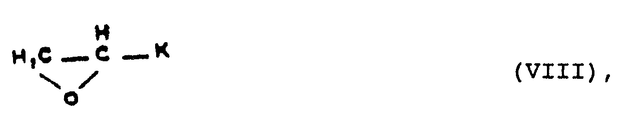 in which K represents a phenyl radical optionally substituted by one or more alkyl or alkoxy radicals containing from 1 to 4 carbon atoms or by one or more hydroxy radicals,
to obtain a compound of the general formula I" in which the meaning of Ar, Y, Z, Yi, Zᵢ, W, U, V, m and n is identical to that given above and the meaning of K is identical to that given for the general formula VIII, 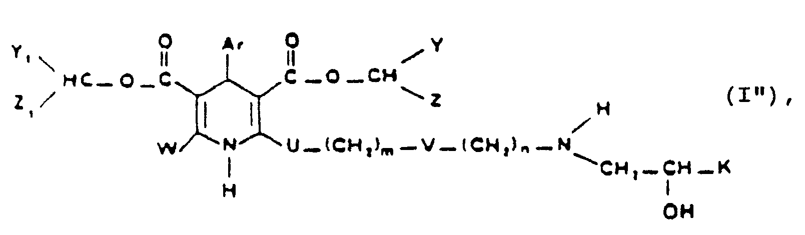 or
are treated with an alkylating agent of the general formula IX: 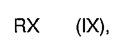 in which X represents halogen and R represents a linear or branched lower alkyl or alkenyl radical containing from 1 to 4 carbon atoms,
in a polar organic solvent, such as acetonitrile, in the presence of a basic mineral salt, such as potassium carbonate, at a temperature of from 40 to 100° C,
to obtain the compounds of the general formula I in which Ar, Y, Z, Yi, Zi, W, U, V, m and n have the same meaning as that defined above and R₁ and R₂ are identical and have the same meaning as R in the general formula IX,
or alternatively
are first treated with benzaldehyde in the presence of an inert apolar aromatic solvent, such as benzene, at a temperature of from 50 to 120 C, and then, after removing the solvent which has been used, are treated with sodium borohydride, in the presence of a low molecular weight polar aliphatic alcohol, to obtain a compound of the general formula I in which the meaning of Ar, Y, Z, Y₁, Z₁, W, U, V, m and n is identical to that given above, R₁ represents a hydrogen atom and R₂ represents a benzyl radical,
which then,
if desired, is treated with an alkylating agent of the general formula IX
to obtain a compound of the general formula I in which Ar, Y, Z, Y₁, Z₁, W, U, V, m and n have the meanings defined above, the definition of R₁ is identical to that of R in the general formula IX, and R₂ represents a benzyl radical, in the form of quaternary ammonium salts or tertiary amines,
which then,
if desired, is treated with lithium triethylborohydride or subjected to catalytic hydrogenation to obtain, from the quaternary ammonium salts or the tertiary amines, respectively, the compounds of the general formula I in which Ar, Y, Z, Y₁, Z₁, W, U, V, m and n have the same meaning as that defined above, R having the same meaning as R in the general formula IX, and R₂ represents a benzyl radical or a halogen atom, respectively,
then,
if desired, it is treated with an alkylating agent of the general formula IX
to obtain the compounds of the general formula I in which each of R₁ and R₂, which are different, represents a linear or branched lower alkyl radical containing from 1 to 4 carbon atoms, or a linear or branched alkenyl radical containing from 1 to 4 carbon atoms,
and then,
if desired, the compounds of the general formula are converted into an addition salt with a pharmaceutically acceptable organic or mineral acid,
or, in those cases where they carry a tertiary amine on their side-chain in the 2-position, into a quaternary ammonium salt with an alkyl or alkenyl halide of the general formula IX.

11. Pharmaceutical composition containing as active ingredient a compound according to any one of claims 1 to 9, in association or in admixture with a pharmaceutically acceptable, nontoxic, inert excipient or carrier.

12. Pharmaceutical composition according to claim 11 containing the active ingredient in an amount of from 0.05 to 50 mg.

13. Pharmaceutical composition according to claims 11 and 12 containing as active ingredient at least one compound according to claims 1 to 9 for use in the treatment of patients requiring calcium regulators.

## Patentansprüche

1. Verbindungen der allgemeinen Formel 1 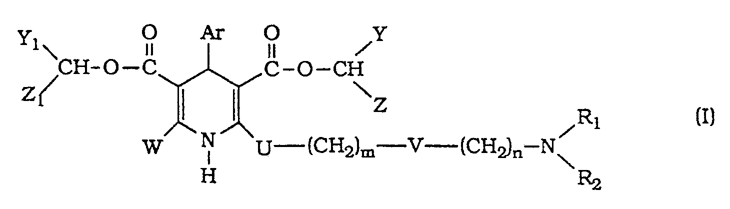 in der
- Ar eine Phenylgruppe darstellt, die gegebenenfalls einen bis fünf gleichartige oder verschiedene Substituenten aufweist, die jeweils Halogenatome, Alkoxygruppen mit 1 bis 4 Kohlenstoffatomen, Alkylthiogruppen mit 1 bis 4 Kohlenstoffatomen, Trihalogenmethylgruppen oder Methylendioxygruppen sind,
- Y, Z, Y₁ und Zi, die gleichartig oder verschieden sein können, jeweils Wasserstoffatome, niedrigmolekulare, geradkettige oder verzweigte Alkylgruppen mit 1 bis 4 Kohlenstoffatomen, Cyclopropylgruppen, Dicyclopropylmethylgruppen, 2,2-Dicyclopropyl-ethylgruppen, 2,2-Dicyclopropyl-ethylengruppen, 3,3-Dicyclopropyl-propylgruppen oder 3,3-Dicyclopropyl-propyl-1-en-gruppen bedeuten,
- W eine niedrigmolekulare, geradkettige oder verzweigte Alkylgruppe mit 1 bis 4 Kohlenstoffatomen oder eine niedrigmolekulare Alkoxymethylgruppe mit 2 bis 5 Kohlenstoffatomen darstellt,
- V eine Methylengruppe oder ein Sauerstoffatom bedeutet,
- U eine Methylenoxygruppe oder eine Ethylenoxygruppe, wenn V ein Sauerstoffatom darstellt, oder eine Methylengruppe bedeutet, wenn V ebenfalls eine Methylengruppe darstellt,
- m und n, die gleichartig oder verschieden sein können, jeweils ganze Zahlen mit Werten von 1 bis 4 bedeuten,
- R₁ und R₂, die gleichartig oder verschieden sein können, jeweils Wasserstoffatome, niedrigmolekulare, geradkettige oder verzweigte Alkylgruppen mit 1 bis 4 Kohlenstoffatomen, niedrigmolekulare, geradkettige oder verzweigte Alkenylgruppen mit 2 bis 4 Kohlenstoffatomen. Trihalogenoacetylgruppen, mit der Maßgabe, daß in diesem Fall V keine Methylengruppe darstellt, Phenylalkylgruppen mit 7 bis 10 Kohlenstoffatomen, die gegebenenfalls am aromatischen Kern durch eine oder mehrere Alkylgruppen oder Alkoxygruppen mit 1 bis 4 Kohlenstoffatomen oder durch eine oder mehrere Hydroxygruppen substituiert sind, 1-Hydroxy-2-phenyl-ethylgruppen, die gegebenenfalls am aromatischen Ring durch eine oder mehrere Alkyl- oder Alkoxygruppen mit 1 bis 4 Kohlenstoffatomen oder durch eine oder mehrere Hydroxygruppen substituiert sind oder gemeinsam mit dem Stickstoffatom, an das sie gebunden sind, eine Phthalimidogruppe bedeuten,
in Form des Racemats oder der optischen Isomeren und deren Additionssalze mit einer pharmazeutisch annehmbaren anorganischen oder organischen Säure, sowie deren mit einem niedrigmolekularen Alkyl- oder Alkenylhalogenid mit 1 bis 4 Kohlenstoffatomen gebildete quartäre Ammoniumsalze, wenn sie ein tertiäres Amin enthalten.

2. Verbindungen der allgemeinen Formel I, in der Ar eine polyhalogenierte Phenylgruppe, Y₁ und Z₁ jeweils ein Wasserstoffatom, W eine Methylgruppe, V ein Sauerstoffatom und U eine Methylenoxygruppe bedeuten, in Form des Racemats oder der optischen Isomeren und deren Additionssalze mit einer pharmazeutisch annehmbaren anorganischen oder organischen Säure.

3. (4R,S)-2-{[2-(2-Amino-ethoxy)-ethoxy]-methyl}-3-ethoxycarbonyl-5-methoxycarbonyl-6-methyl-4-pentafluorphenyl-1,4-dihydro-pyridin, dessen optische Isomeren und dessen Additionssalze mit einer pharmazeutisch annehmbaren anorganischen oder organischen Säure.

4. (4R,S)-2-{[2-(2-Amino-ethoxy)-ethoxy]-methyl}-4-(2-chlorphenyl)-3-ethoxycarbonyl-5-methoxycarbonyl-6-methyl-1,4-dihydro-pyridin,dessen optische Isomeren und dessen Additionssalze mit einer pharmazeutisch annehmbaren anorganischen oder organischen Säure.

5. (4R,S)-2-{[2-(2-Amino-ethoxy)-ethoxy]-mothyl}-4-(2,3-dichlor-phenyl)-3-ethoxycarbonyl-5-methoxycarbonyl-6-methyl-1,4-dihydro-pyridin, dessen optische Isomeren und dessen Additionssalze mit einer pharmazeutisch annehmbaren anorganischen oder organischen Säure.

6. (4R,S)-4-(2-Chlor-phenyl)-2-{[2-(2-N,N-diallylamino-ethoxy)-ethoxy]-methyl}-3-ethoxycarbonyl-5-methoxycarbonyl-6-methyl-1,4-dihydro-pyridin, dessen optische Isomeren und dessen Additionssalze mit einer pharmazeutisch annehmbaren anorganischen oder organischen Säure.

7. (4R,S)-2-(7-Amino-heptyl)-3-ethoxycarbonyl-4-(2-chlor-phenyl)-5-methoxycarbonyl-6-methyl-1,4-dihydro- pyridin, dessen optische Isomeren und dessen Addltionssalze mit einer pharmazeutisch annehmbaren anorganischen oder organischen Säure.

8. (4R,S)-2-(5-Amino-pentyl)-3-ethoxycarbonyl-4-(2-chlor-phenyl)-5-methoxycarbonyl-6-methyl-1,4-dihydro- pyridin, dessen optische Isomeren und dessen Additionssalze mit einer pharmazeutisch annehmbaren anorganischen oder organischen Säure.

9. (4R,S)-2-{2-[2-(2-Amino-ethoxy)-ethoxy]-ethyl}-4-(2,3-dichlor-phenyl)-3-ethoxycarbonyl-5-methoxycarbonyl-6-methyl-1,4-dihydro-pyridin.

10. Verfahren zur Herstellung der Verbindungen der allgemeinen Formel I, dadurch gekennzeichnet, daß man
- entweder
e̅i̅n̅e̅ V̅e̅rbindung der allgemeinen Formel II 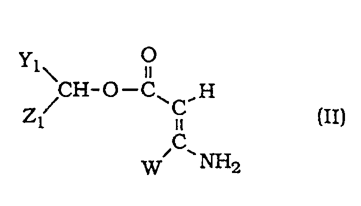 in der die Definition der Substituenten Y₁, Z₁ und W der für die allgemeine Formel angegebenen entspricht,
mit einem Ketoester der allgemeinen Formel III 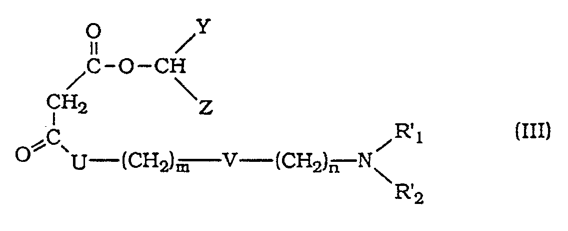 in der Y, Z, U, V, m und n die bezüglich der allgemeinen Formel I angegebenen Bedeutungen besitzen und R'₁ und R'₂ eine Methylgruppe und eine Benzylgruppe oder ein Wasserstoffatom und eine Trihalogenoacetylgruppe mit der Maßgabe, daß V nicht gleichzeitig Methylgruppen bedeutet, oder gemeinsam mit dem Stickstoffatom, an das sie gebunden sind, eine Phthalimidogruppe bedeuten, und mit einem aromatischen Aldehyd der allgemeinen Formel IV 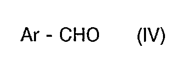 in der Ar die bezüglich der allgemeinen Formel I angegebenen Bedeutungen besitzt, in einem polaren organischen Lösungsmittel, wie einem primären oder sekundären Alkohol oder einer organischen Säure mit niedrigem Molekulargewicht und bei einer Temperatur zwischen 40 und 100°C kondensiert zur Bildung einer Verbindung der allgemeinen Formel I' 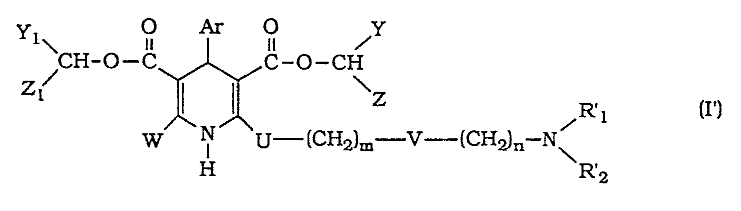 in der Ar, Y, Z, Yi, Zi, W, U, V, m und n die oben angegebenen Bedeutungen besitzen und R'₁ und R'₂ die bezüglich der allgemeinen Formel III angegebenen Bedeutungen von R'₁ und R'₂ aufweisen, oder
einen Ketoester der allgemeinen Formel V 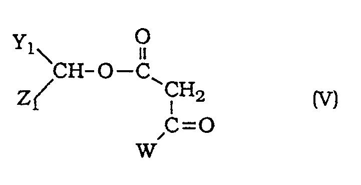 in der Y₁, Z₁ und W die bezüglich der Formel I angegebenen Bedeutungen besitzen,
mit einer Verbindung der allgemeinen Formel VI 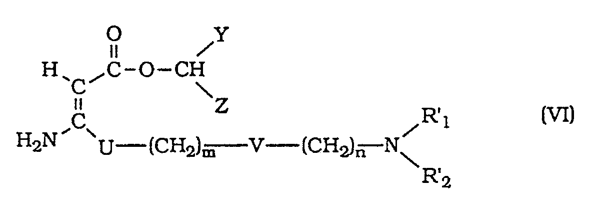 in der Y, Z, U, V, m und n die bezüglich der Formel I angegebenen Bedeutungen besitzen und die Definition von R'₁ und R'₂ gleich ist jener der allgemeinen Formel III gegebenen, und mit einem aromatischen Aldehyd der allgemeinen Formel IV
in einem polaren organischen Lösungsmittel, wie einem primären oder sekundären Alkohol oder einer organischen Säure mit niedrigem Molekulargewicht bei einer Temperatur zwischen 40 und 100 C kondensiert zur Bildung einer Verbindung der allgemeinen Formel I',
- oder
ein Benzyliden der allgemeinen Formel VII 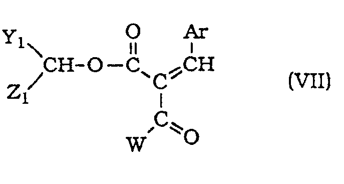 in der Yi, Z₁, W und Ar die bezüglich der Formel I angegebenen Bedeutungen besitzen,
mit einer Verbindung der allgemeinen Formel VI in einem polaren organischen Lösungsmittel, wie einem primären oder sekundären Alkohol oder einer organischen Säure mit niedrigem Molekulargewicht bei einer Temperatur zwischen 40 und 100° C kondensiert zur Bildung der Verbindungen der allgemeinen Formel
- und anschließend
ge̅w̅ü̅ns̅c̅h̅t̅e̅n̅f̅a̅l̅l̅s̅ d̅ie Verbindungen der allgemeinen Formel I', in der Ar, Y, Z, Yi, Z₁, W, U, V, m und n die oben angegebenen Bedeutungen besitzen und R₁ und R₂ ein Wasserstoffatom und eine Trihalogenoacetylgruppe oder gemeinsam mit dem Stickstoffatom, an das sie gebunden sind, eine Phthalimidogruppe bedeuten,
der Einwirkung von Hydrazin oder eines basischen anorganischen Salzes, wie Kaliumcarbonat, in Gegenwart von Wasser in einem mit Wassermischbaren polaren alkoholischen Lösungsmittel mit niedrigem Molekulargewicht bei einer Temperatur zwischen 40 und 100° C unterwirft zur Bildung der Verbindungen der allgemeinen Formel I, in der Ar, Y, Z, Y₁, Z₁, W, U, V, m und n die oben angegebenen Bedeutungen besitzen und R₁ und R₂ ein Wasserstoffatom bedeuten,
- die man anschließend
gewünschtenfalls
entweder
unter der Einwirkung eines Arylethylenoxids der allgemeinen Formel VIII 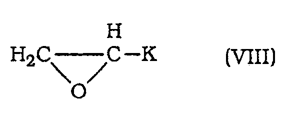 in der K eine gegebenenfalls durch eine oder mehrere Alkylgruppen oder Alkoxygruppen mit 1 bis 4 Kohlenstoffatomen oder eine oder mehrere Hydroxygruppen substituierte Phenylgruppe darstellt, unterwirft zur Bildung einer Verbindung der allgemeinen Formell" 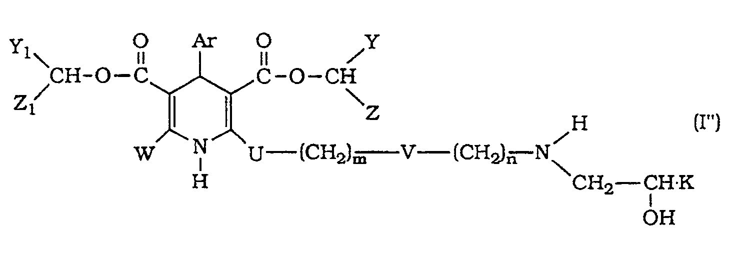 in der die Bedeutung von Ar, Y, Z, Y₁, Z₁, W, U, V, m und n identisch der zuvor angegebenen bleibt, und die Bedeutung von K identisch ist mit der für die allgemeine Formel VIII gegebenen, oder
der Einwirkung eines Alkylierungsmittels der allgemeinen Formel IX 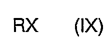 in der X ein Halogen und R eine niedrigmolekulare, geradkettige oder verzweigte Alkyl- oder Alkylengruppe mit 1 bis 4 Kohlenstoffatomen bedeuten,
in einem polaren organischen Lösungsmittel, wie Acetonitril, in Gegenwart eines basischen Mineralsalzes, wie Kaliumcarbonat, bei einer Temperatur zwischen 40 und 100°C unterwirft zur Bildung der Verbindungen der allgemeinen Formel I, in der Ar, Y, Z, Yi, Z₁, W, U, V, m und n die zuvor angegebenen Bedeutungen besitzen und R₁ und R₂ gleichartig sind und die gleichen Bedeutungen besitzen wie R der allgemeinen Formel IX; oder
entweder zunächst der Einwirkung von Benzaldehyd in Gegenwart eines inerten und apolaren aromatischen Lösungsmittels, wie Benzol, bei einer Temperatur zwischen 50 und 120° C unterwirft und anschließend nach der Entfernung des verwendeten Lösungsmittels mit Natriumborhydrid in Gegenwart eines polaren aliphatischen Alkohols mit niedrigem Molekulargewicht behandelt zur Bildung einer Verbindung der allgemeinen Formel 1, in der Ar, Y, Z, Y₁, Z1, W, U, V, m und n die oben angegebenen Bedeutungen besitzen, R₁ ein Wasserstoffatom und R₂ eine Benzylgruppe bedeuten,
welche man anschließend
gewünschtenfalls der Einwirkung eines Alkylierungsmittels der allgemeinen Formel IX unterwirft zur Bildung einer Verbindung der allgemeinen Formel I, in der Ar, Y, Z, Y₁, Z1, W, U, V, m und n die zuvor angegebenen Bedeutungen besitzen, die Definition von R₁ identisch ist mit jener von R der allgemeinen Formel IX und R₂ eine Benzylgruppe bedeutet, in Form der quaternären Ammoniumsalze oder der tertiärem Amine,
welches man anschließend
gewünschtenfalls der Einwirkung von Lithiumtriethylborhydrid oder einer katalytischen Hydrierung unterwirft zur Bildung der entsprechenden quartären Ammoniumsalze oder tertiären Amine der Verbindungen der allgemeinen Formel I, in der Ar, Y, Z, Y₁, Z₁, W, U, V, m und n die oben angegebenen Bedeutungen besitzen, R₁ die gleiche Bedeutung besitzt wie R der allgemeinen Formel IX und R₂ eine Benzylgruppe bzw. ein Halogenatom bedeutet,
worauf
man gewünschtenfalls diese Verbindungen der Einwirkung eines Alkylierungsmittels der allgemeinen Formel IX unterwirft zur Bildung der Verbindungen der allgemeinen Formel I, in der R₁ und R₂ verschieden sind und jeweils niedrigmolekulare, geradkettige oder verzweigte Alkylgruppen mit 1 bis 4 Kohlenstoffatomen oder geradkettige oder verzweigte Alkylengruppen mit 1 bis 4 Kohlenstoffatomen bedeuten,
und anschließend
gewünschtenfalls die Verbindungen der allgemeinen Formel I mit einer pharmazeutisch annehmbaren anorganischen oder organischen Säure in ein Additionssalz überfuhrt,
oder dann, wenn sie an ihrer Seitenkette in Position 2 ein tertiäres Amin aufweisen, mit einer halogenierten Alkylverbindung oder Alkylenverbindung der allgemeinen Formel IX in ein quartäres Ammoniumsalz umwandelt.

11. Pharmazeutische Zubereitung enthaltend als Wirkstoff eine Verbindung nach einem der Ansprüche 1 bis 9 in Kombination oder in Mischung mit einem inerten, nichttoxischen, pharmazeutisch annehmbaren Bindemittel oder Trägermaterial.

12. Pharmazeutische Zubereitung nach Anspruch 11, enthaltend den Wirkstoff in einer Dosis von 0,05 bis 50 mg.

13. Pharmazeutische Zubereitung nach den Ansprüchen 11 und 12, enthaltend als Wirkstoff mindestens eine Verbindung nach den Ansprüchen 1 bis 9 in einer Form zur Behandlung von Kranken, welche Calciummoderatoren benötigen.
